# EUROPEAN PATENT APPLICATION

(11) **EP 2 574 665 A1**
(43) Date of publication of application: **03.04.2013**
(21) Application number: 12172058.5
(22) Date of filing: 03.03.2008
(51) Int. Cl.: C12N 5/07, G01N 33/50, A61Q 19/00

(54) **Procurement, isolation and cryopreservation of endometrial/menstrual cells**

(30) Priority: 01.03.2007 US 904836 P; 20.03.2007 US 918961 P; 17.09.2007 US 994166 P; 09.10.2007 US 998255 P; 22.01.2008 US 11881 P; 29.02.2008 US 67849 P
(62) Divisional of application: 08726385.1
(71) Applicant: Cryo-Cell International, Inc., Oldsmar FL 34677 (US); Walton, Mercedes A., Mendham NJ 07945 (US); Allickson, Julie G., Odessa, FL 33556 (US)
(72) Inventor: Walton, Mercedes A., Mendham, NJ 07945 (US); Allickson, Julie G., Odessa, FL 33556 (US)
(74) Representative: Atkinson, Jennifer

(57) **Abstract**

Compositions comprising menstrual stem cells (MSCs) and methods, processes, and system therefore are provided by the invention. MSCs are processed from menstrual flow collected during menses. MSCs may be cryopreserved, processed through various culturing and selection steps in preparation for cryopreservation, or processed for therapeutic or cosmeceutical use. Cryopreserved MSCs may be thawed in preparation for therapeutic and cosmeceutical use. MSCs express CD9, CD10, CD13, CD29, CD44, CD49e, CD49f, CD59, CD81, CD105, CD166, and HLA class I, and have low or no expression of CD3 and HLA class II.

## Description

### CROSS-REFERENCE TO RELATED APPLICATIONS

This application claims the priorities of U.S. Provisional Patent Application Serial Nos. 60/904,836, filed March 1, 2007, 60/918,961, filed March 20, 2007, 60/994,166, filed September 17, 2007, 60/998,255, filed October 9, 2007, 61/011,881, filed January 22, 2008, and filed February 29, 2008, each entitled "Procurement, Isolation and Cryopreservation of Endometrial/Menstrual Cells", the entireties of which are incorporated herein by reference.

### BACKGROUND OF THE INVENTION

### Field of the Invention

The invention relates generally to methods, processes, and systems for obtaining menstrual stem cells (MSCs). The invention relates specifically to methods, processes, and systems for procuring and processing blood, fluids, and tissues obtained during menstruation to isolate and collect a population of menstrual stem cells that may be cryopreserved or further processed through various culturing and selection steps to obtain MSCs in preparation for cryopreservation or for therapeutic or cosmeceutical use. The invention also relates to compositions of matter comprising at least one menstrual stem cell.

### Description of the Prior Art

Stem cells inherently possess the capability to undergo cellular division and cellular differentiation, *in vivo* by way of control of cell-to-cell contact and intrinsic signals. Stem cells have been shown to be capable of dividing and differentiating *in vitro* into a variety of cells by controlling cell contact and intrinsic signals by stimulation with local environmental factors. It is recognized that stem cells may be obtained from several sources including a variety of adult tissues, such as bone marrow and also embryonic tissues.

Certain stem cells express the antigenic factor CD117 that is also known as a c-kit receptor, Steel factor receptor, and stem cell factor receptor. The gene for c-kit encodes a tyrosine kinase growth factor receptor for Stem Cell Factor (SCF), which is also known as mast cell growth factor and is essential for hematopoiesis, melanogenesis, and fertility. It is recognized that CD117 is expressed in hematopoietic stem cells, mast cells, germ cells, melanocytes, certain basal epithelial cells, luminal epithelium of the breast, and the interstitial cells of Cajal of the gastrointestinal tract. CD117 imparts a critical role in germ cell establishment, maintenance, and function. Research indicates that in the embryonic gonad, CD117 and its corresponding ligand SCF, are essential for the primordial germ cell survival and proliferation. Additionally, research indicates that CD 117 and its corresponding ligand SCF are essential for the gamete production in response to gonadotropic hormones. In other words, CD117 in combination with the ligand SCF are necessary for the survival and proliferation of germ cells of the testis, the spermatogonia, and for the growth and maturation of oocytes. Research also indicates that CD117 is a potent growth factor for primitive hematopoietic cell proliferation *in vitro.*

Therapeutic applications for stem cells began with the early experimentation of bone marrow. Nearly a century ago, physicians administered bone marrow by mouth to patients with anemia and leukemia. Although such therapy was unsuccessful, laboratory researchers eventually demonstrated that mice with defective marrow could be restored to health with infusions into the blood stream of marrow taken from other mice. This caused physicians to speculate whether it was feasible to transplant bone marrow from one human to another (allogeneic transplantation). Stem cells from bone marrow and umbilical have been extensively used primarily in hematopoietic transplants to treat a wide variety of diseases. From the first stem cell transplants that utilized bone marrow, other sources for stem cells have been identified to include amniotic fluid, placenta, umbilical cord, cord lining, Wharton's Jelly, fat, and epithelial cells.

Effects of the human immune system, the body's inherent mechanism to defend itself from infection and foreign substances, became a critical consideration in early transplants as researchers' encountered illness and/or fatalities resulting from the body's rejection of cells later characterized as Graft versus Host Disease (GVHD). Performing marrow transplants in humans was not attempted on a larger scale until after the discovery of the relationship between human histocompatibility antigens and the human immune system. These proteins, found on the surface of most cells in the body, are called human leukocyte antigens, or HLA antigens. The body's immune system uses these HLA antigens to identify which cells belong in the body and which do not. Whenever the immune system does not recognize the antigens on a cell, it creates antibodies and other substances to destroy the cell. Objects that the body looks for and destroys are infection-causing bacteria, viruses, tumor cells, and foreign objects such as splinters. In this way, the immune system defends the body against things that can enter the body and cause harm. Depending upon the disease state targeted for treatment, autologous transplantation utilizing an individuals own stem cells may have certain potential advantages resulting from the benefits of an exact HLA match. The closer the donor's and recipient's HLA antigens match, the less likely it is that the T cells (immune system cells) of the donated marrow will react against the patient's body.

The transplantation of adult stem cells derived from bone marrow has been successfully used in treatment of human disease such as Fanconi's Anemia, Aplastic Anemia, Acute and Chronic Leukemias, Myeloproliferative Disorders, Myelodysplastic Syndromes, Lymphoproliferative Disorders, and other malignancies. Alternative sources of bone marrow adult stem cells include peripheral blood progenitor cells, umbilical cord blood and mesenchymal stem cells harvested from these sources. However, there are several shortcomings associated with therapeutic use of adult stem cells. Adult stem cells have been shown to have limited efficacy such as slow growth and loss of pluripotency after several passages in culture.

Embryonic stem cells have demonstrated proliferative potential making them suitable for cellular therapy. However, human embryonic stem cells have been shown to produce teratomas. Additionally, the harvesting of stem cells from embryos poses ethical concerns due in part to the destruction of the growing embryo in the harvesting process.

Other sources of stem cells have been identified that overcome at least some of the issues associated with adult and embryonic stem cells.

One source is umbilical cord blood that contains human adult stem cells. Umbilical cord blood has proven to be a viable source of stem cells for several reasons. Umbilical cord blood is relatively easy to procure during delivery of a child and to process for cryopreservation. Umbilical cord blood provides a suitable source of stem cells capable of cellular division and differentiation into a variety of cell types. Stem cells derived from cord blood have been used in clinical settings to treat several known human disorders and diseases as an alternative to bone marrow. In particular, stem cells derived from cord blood have been successful in hematopoietic engraftment, hematopoietic reconstitution, and treating spinal cord injuries. Additionally, stem cells derived from cord blood have been shown in animal models to reverse the effects of stroke and myocardial infarction.

It has been determined that endometrial/menstrual stem cells (MSCs) demonstrate a proliferative capacity in culture similar to embryonic stem cells. MSCs also display certain mesenchymal stem cell markers and certain embryonic stem cell markers demonstrating the potential for pluripotent capability *in vitro* and *in vivo.* The use of MSCs as a source of stem cells is beneficial for several reasons. First, MSCs demonstrate the capability to differentiate into specific cell lineages. Second, any ethical concerns or considerations are alleviated by collecting MSCs that are otherwise considered waste. Finally, the process of procuring MSCs during a menstrual cycle imparts relatively no risk to a donor.

It is believed that there is no known methodology directed to collecting MSCs, nor procuring a suitable sample amount of MSCs, and related processing and cell collection methodologies useful for obtaining a viable, maximum yield of MSCs expressing certain mesenchymal stem cell markers and/or certain embryonic-like antigenic factors among other cell surface factors expressed by MSCs.

Accordingly, there is a present need for methods to collect a suitable amount of MSCs present in fluid, blood, and tissue shed during menstruation in preparation for ready shipment to a laboratory that processes the menstrual fluid, blood and tissue to isolate and cryopreserve or process MSCs. There is also a present need for methods to process menstrual fluid, blood, and tissue to obtain a cell preparation of MSCs derived from the menstrual blood, fluid, and tissue. There is also a further need to isolate, select, and/or culture MSCs from menstrual flow in preparation for cryopreservation, further processing or therapeutic use, such as, for example, cells that may express at least one of the cell surface markers or intracellular markers comprising CD9, CD10, CD13, CD29, CD44, CD49e, CD49f, CD59, CD81, CD105, CD166, and HLA class I, while expressing low or no levels of at least one of cell markers CD3 and HLA class II. Other cell marker characteristics are provided with the results of the practice of the embodiments of the invention. There is yet a further need to cryopreserve the population of MSCs or specific MSCs obtained and collected from the menstrual blood, fluid, and tissue.

### SUMMARY OF THE INVENTION

There is a present need for a source of stem cells that are easy to collect, process, and cryopreserve and that exhibit the potential for therapeutic, cosmeceutical, or other uses. This need is met by the methods, processes, and compositions of the present invention.

The present invention provides a method for obtaining menstrual stem cells. In an embodiment, the method comprises a step of isolating menstrual stem cells from menstrual flow collected during menstruation. The step of isolating menstrual stem cells from menstrual flow comprises processing the menstrual flow with at least one step of centrifugation, density gradient centrifugation, filtration, or sedimentation. The step of isolating menstrual stem cells may also optionally comprise decontaminating the menstrual stem cells with a solution comprising at least one antibiotic. In the embodiment, the method comprises an additional step of processing the menstrual stem cells isolated from the menstrual flow. The step of processing menstrual stem cell isolated from a menstrual flow sample may comprise at least one step of selecting menstrual stem cells for certain cell surface markers, or culturing menstrual stem cells from a post-processing sample, a selected sample, or a thawed sample of menstrual stem cells.

In an embodiment, the step of processing menstrual stem cells isolated from menstrual flow comprises the step of selecting menstrual stem cells for at least one cell marker comprising CD9, CD10, CD13, CD29, CD44, CD49e, CD49f, CD59, CD81, CD105, CD166, and HLA class I. The selected cells may be further processed by culturing the menstrual stem cells. The cultured menstrual stem cells may then be subject to a further step of selecting menstrual stem cells for at least one cell marker comprising CD9, CD10, CD13, CD29, CD44, CD49e, CD49f, CD59, CD81, CD105, CD166, and HLA class I. The selected menstrual stem cells may be subject to a further step of culturing the menstrual stem cells. At any point after a step of selecting or culturing menstrual stem cells, the menstrual stem cells may be subject to a step of preparing the menstrual stem cells for cryopreservation or therapeutic or cosmeceutical use. Menstrual stem cells that have been prepared for cryopreservation may then be cryopreserved and later thawed for use.

In another embodiment, the step of processing menstrual stem cells isolated from the menstrual flow comprises culturing menstrual stem cells. The cultured menstrual stem cells may then be subject to a step of selecting menstrual stem cells for certain cell surface markers. The selected menstrual stem cells may then be subject to a further step of culturing the menstrual stem cells. The further cultured menstrual stem cells may then be subject to an additional step of selecting menstrual stem cells for a cell marker. At any point after a step of selecting or culturing menstrual stem cells, the menstrual stem cells may be subject to a step of preparing the menstrual stem cells for cryopreservation or therapeutic or cosmeceutical use. Menstrual stem cells that have been prepared for cryopreservation may then be cryopreserved and later thawed for use.

In another further embodiment, the step of processing menstrual stem cells isolated from the menstrual flow comprises a step of cryopreserving menstrual stem cells.

In yet another embodiment, the step of processing menstrual stem cells comprises the further step of preparing a composition comprising menstrual stem cells and a cell media to maintain viability of the menstrual stem cells.

In an even further embodiment, the step of processing menstrual stem cells comprises the further step ofpreparing a composition comprising menstrual stem cells and a cosmeceutical preparation.

In a further embodiment, cryopreserved menstrual stem cells may be thawed in preparation for further processing comprising at least one step of either culturing or selecting menstrual stem cells. Alternatively, cryopreserved menstrual stem cells may be thawed in preparation for therapeutic or cosmeceutical use.

The present invention also provides a process for collecting menstrual stem cells from menstrual flow. In an embodiment, the process comprises procuring menstrual flow during a menstrual cycle; transporting the menstrual flow to a processing facility, isolating menstrual stem cells from menstrual flow into a cell preparation, and processing the cell preparation of menstrual stem cells.

The present invention also provides a system for collecting menstrual stem cells from menstrual flow. In an embodiment, the system comprises a menstrual stem cell isolator, wherein menstrual stem cell isolator separates menstrual stem cells from menstrual flow, a menstrual stem cell collector, wherein the menstrual stem cell collector collects the menstrual stem cells, and a menstrual stem cell processor, wherein the menstrual stem cell processor prepares menstrual stem cells for therapeutic use or cryopreservation.

The present invention provides a method for cryopreserving menstrual stem cells. In an embodiment, the method comprises the steps of isolating menstrual stem cells from menstrual flow collected during menstruation, collecting menstrual stem cells isolated from menstrual flow, and cryopreserving menstrual stem cells isolated from the menstrual flow.

The method of cryopreserving menstrual stem cells including the step of isolating menstrual stem cells from the menstrual flow comprises processing the menstrual flow by at least one step of centrifugation, density gradient centrifugation, filtration, or sedimentation. The step of isolating menstrual stem cells may also optionally comprise decontaminating the menstrual stem cells with a solution comprising at least one antibiotic. The method may comprise at least one step of selecting menstrual stem cells for a cell marker prior to at least at step of cryopreservation or further cell culture. The method may comprise at least one further step of culturing selected menstrual stem cells.

The method of cryopreserving menstrual stem cells including the step of collecting menstrual stem cells isolated from menstrual flow comprises at least one step of culturing menstrual stem cells. The method may comprise at least one step of selecting menstrual stem cells from a cell culture.

The present invention provides a population of menstrual stem cells obtained by the process comprising the steps of isolating menstrual stem cells from menstrual flow collected during menstruation and collecting menstrual stem cells isolated from menstrual flow.

The present invention provides a process for enriching a population of menstrual stem cells collected from menstrual flow. In an embodiment, the process comprises the steps of isolating menstrual stem cells from menstrual flow collected during menstruation and collecting menstrual stem cells isolated from menstrual flow. The process comprises at least one step of selecting menstrual stem cells for at least one cell marker of the group comprising CD9, CD10, CD13, CD29, CD44, CD49e, CD49f, CD59, CD81, CD105, CD166, and HLA class I. The process may comprise at least one step of culturing menstrual stem cells. The process may comprise at least one step of selecting menstrual stem cells and at least one step of culturing selected menstrual stem cells. The step of isolating menstrual stem cells may also optionally comprise decontaminating the menstrual stem cells with a solution comprising at least one antibiotic. In the alternative, the process may comprise at least one step of culturing menstrual stem cells and at least one step of selecting menstrual stem cells from a cell culture.

The present invention provides a process for enriching a population of menstrual stem cells. In an embodiment, the process comprises the steps of isolating menstrual stem cells from menstrual flow, collecting menstrual stem cells isolated from menstrual flow and selecting menstrual stem cells expressing cell markers associated with MSCs.

The present invention provides a population of menstrual stem cells comprising at least one of the cell markers comprising CD9, CD10, CD13, CD29, CD44, CD49e, CD49f, CD59, CD81, CD105, CD166, and HLA class I.

The present invention provides an enriched population of cells comprising menstrual stem cells expressing at least one of the cell markers comprising CD9, CD10, CD13, CD29, CD44, CD49e, CD49f, CD59, CD81, CD105, CD166, and HLA class I; and expressing low or no cell markers comprising at least one of the cell markers CD3 and HLA class II.

The present invention provides a composition comprising at least one menstrual stem cell and a cryopreservation agent.

The present invention provides a composition comprising at least one menstrual stem cell and a cell media.

The present invention provides a composition comprising at least one menstrual stem cell and a cosmeceutical agent.

The present invention provides a composition comprising at least one menstrual stem cell and a preservation agent to maintain cell viability.

The present invention provides a composition comprising at least one menstrual stem cell and cell media in a container in preparation for therapeutic use of the at least one menstrual stem cell.

The cells collected in accordance with the present invention may be used in therapeutic, cosmeceutical, or regenerative medicine or other suitable applications.

The present invention provides methods and processes for collecting at least one sample of menstrual flow comprising menstrual blood, fluid, cells, and tissue, for preparing the menstrual flow for transport by combining a menstrual flow sample with collection media, and for transporting the menstrual flow sample to a processing facility where the menstrual flow sample is processed to isolate, collect, and process MSCs for cryopreservation or therapeutic or cosmeceutical use.

The present invention also provides methods and processes for isolating, collecting, and concentrating MSCs in manner to produce a high yield of viable MSCs.

In an embodiment, the step of selecting menstrual stem cells may involve positively selecting cells expressing at least one of the cell markers present with menstrual stem cells. In an alternative, the step of selecting menstrual stem cell may comprise negatively selecting cells by removing unwanted cells expressing cell markers that are not present with menstrual stem cells. The negative selection may comprise selecting cells expressing CD45, for example, or other cell surface marker not present on a menstrual stem cell.

In a further embodiment, the step of selecting menstrual stem cells may comprise using a commercially available depletion or concentration kit, such as, for example, RosetteSep, EasySep, RoboSep, StemSep, or SpinSep from Stem Cell Technologies, Inc.

In yet a further embodiment, the step of selecting menstrual stem cells may comprise selecting menstrual stem cells using ALDEFLUOR® (Aldagen, Inc.) to select MSCs and to eliminate dead and/or dying cells without intact cellular membranes. The ALDEFLUOR® kit may be used to select ALDH^{br} cells positive for surface markers present with menstrual stem cells, Lineage-antigen negative (Lin⁻) cells, colony-forming cells, long-term culture initiating cells, and NOD/SCID repopulating cells.

In yet an even further embodiment, the step of selecting menstrual stem cells may comprise subjecting the menstrual stem cells by serum deprivation for about 2 weeks to about 4 weeks to concentrate MSCs expressing pertinent MSC markers. Once the cells are harvested and cultured, hematopoietic stem cells should be eliminated.

### CLAUSES

The application provides amongst other things, the subject matter of the following clauses:
1. A method for obtaining menstrual stem cells, the method comprising the steps of: a) isolating menstrual stem cells from menstrual flow; and b) processing the menstrual stem cells isolated from the menstrual flow.
2. The method of clause 1, wherein the step of isolating menstrual stem cell from the menstrual flow comprises processing the menstrual flow with at least one of centrifugation, density gradient centrifugation, filtration or sedimentation.
3. The method of clause 1, wherein the step of processing the menstrual stem cells isolated from the menstrual flow comprises selecting menstrual stem cells for at least one cell marker comprising CD9, CD10, CD13, CD29, CD44, CD49e, CD49f, CD59, CD81, CD105, CD 166, and HLA class I.
4. The method of clause 3, wherein the step of processing the menstrual stem cells comprises the further step of culturing menstrual stem cells.
5. The method of clause 4, wherein the step of processing menstrual stem cells comprises the further step of selecting menstrual stem cells for at least one cell marker comprising CD9, CD10, CD13, CD29, CD44, CD49e, CD49f, CD59, CD81, CD105, CD 166, and HLA class I.
6. The method of clause 5, wherein the step of processing menstrual stem cells comprises the further step of culturing menstrual stem cells.
7. The method of clause 1, wherein the step of processing menstrual stem cells isolated from the menstrual flow comprises culturing menstrual stem cells.
8. The method of clause 7, wherein the step of processing the menstrual stem cells comprising the further step of selecting menstrual stem cells.
9. The method of clause 8, wherein the step of processing menstrual stem cells comprises the further step of culturing menstrual stem cells.
10. The method of clause 9, wherein the step of processing menstrual stem cells comprises the further step of selecting menstrual stem cells for a cell marker.
11. The method of clause 1, wherein the method comprises the further step of cryopreserving menstrual stem cells.
12. The method of clause 11, wherein the method comprises the further steps of thawing cryopreserved menstrual stem cells and processing thawed menstrual stem cells.
13. The method of clause 1, wherein the method comprises the further step ofpreparing a therapeutic composition comprising menstrual stem cells and a cell media to maintain viability of the menstrual stem cells.
14. The method of clause 1, wherein the method comprises the further step of preparing a cosmeceutical composition comprising menstrual stem cells and a cosmeceutical preparation.
15. A process for collecting menstrual stem cells from menstrual flow, the process comprising: a) procuring menstrual flow during a menstrual cycle; b) transporting the menstrual flow to a processing facility; c) isolating menstrual stem cells from menstrual flow into a cell preparation; and d) processing the cell preparation of menstrual stem cells.
16. A system for collecting menstrual stem cells from menstrual flow, the system comprising: a) a menstrual stem cell isolator, wherein menstrual stem cell isolator separates menstrual stem cells from menstrual flow; b) a menstrual stem cell collector, wherein the menstrual stem cell collector collects the menstrual stem cells; and c) a menstrual stem cell processor, wherein the menstrual stem cell processor prepares menstrual stem cells for therapeutic use or cryopreservation.
17. A method for cryopreserving menstrual stem cells, the method comprising the steps of: a) isolating menstrual stem cells from menstrual flow collected during menstruation; b) collecting menstrual stem cells isolated from menstrual flow; and c) cryopreserving menstrual stem cells isolated from the menstrual flow.
18. The method of clause 17, wherein the step of isolating menstrual stem cells from the menstrual flow comprises processing the menstrual flow by at least one of centrifugation, density gradient centrifugation, filtration or sedimentation.
19. The method of clause 17, wherein the step of collecting menstrual stem cells isolated from menstrual flow comprises at least one step of selecting menstrual stem cells for a cell marker.
20. The method of clause 19, wherein the step of collecting menstrual stem cells isolated from menstrual flow comprises at least one step of culturing selected menstrual stem cells.
21. The method of clause 17, wherein the step of collecting menstrual stem cells isolated from menstrual flow comprises at least one step of culturing menstrual stem cells.
22. The method of clause 21, wherein the step of collecting menstrual stem cells isolated from menstrual flow comprises at least one step of selecting menstrual stem cells from a cell culture.
23. A population of menstrual stem cells obtained by the process comprising: a) isolating menstrual stem cells from menstrual flow collected during menstruation; and b) collecting menstrual stem cells isolated from menstrual flow.
24. A process for enriching a population of menstrual stem cells collected from menstrual flow, the process comprising the steps of: a) isolating menstrual stem cells from menstrual flow collected during menstruation; and b) collecting menstrual stem cells isolated from menstrual flow.
25. The process of clause 24, wherein the process comprises at least one step of selecting menstrual stem cells for at least one of a cell marker CD9, CD10, CD 13, CD29, CD44, CD49e, CD49f, CD59, CD81, CD105, CD166, and HLA class I.
26. The process of clause 24, wherein the process comprises at least one step of culturing menstrual stem cells.
27. The process of clause 24, wherein the process comprises at least one step of selecting menstrual stem cells and at least one step of culturing selected menstrual stem cells.
28. The process of clause 24, wherein the process comprises at least one step of culturing menstrual stem cells and at least one step of selecting menstrual stem cells from a cell culture.
29. A process for enriching a population of menstrual stem cells expressing CD117, the process comprising the steps of: a) isolating menstrual stem cells from menstrual flow, b) collecting menstrual stem cells isolated from menstrual flow; and c) selecting menstrual stem cells expressing CD117.
30. A population of menstrual stem cells comprising at least one of the cell markers comprising CD9, CD10, CD13, CD29, CD44, CD49e, CD49f, CD59, CD81, CD105, CD166, and HLA class I.
31. An enriched population of cells comprising menstrual stem cells a) expressing at least one of the cell markers comprising CD9, CD10, CD13, CD29, CD44, CD49e, CD49f, CD59, CD81, CD105, CD166, and HLA class I; and b) expressing low or no of at least one of the cell markers comprising CD3 and HLA class II.
32. A composition comprising at least one menstrual stem cell and a cryopreservation agent.
33. A composition comprising at least one menstrual stem cell and a cell media.
34. A composition comprising at least one menstrual stem cell and a cosmeceutical agent.
35. A composition comprising at least one menstrual stem cell and a preservation agent to maintain cell viability.
36. A composition comprising at least one menstrual stem cell and cell media in a container in preparation for therapeutic use of the at least one menstrual stem cell.

### BRIEF DESCRIPTION OF THE FIGURES

FIG. 1 is a flow chart illustrating generally overall methods and processes of the present invention;

FIG. 2 is a flow chart showing an embodiment of the invention for collecting and cryopreserving menstrual stem cells;

FIG. 3 is a flow chart showing another embodiment of the invention for collecting and cryopreserving menstrual stem cells;

FIG. 4 is a flow chart showing yet another embodiment of the invention for collecting and cryopreserving menstrual stem cells;

FIG. 5 is a flow chart showing a further embodiment of the invention for collecting and cryopreserving menstrual stem cells;

FIG. 6 is a flow chart showing yet a further embodiment of the invention for collecting and cryopreserving menstrual stem cells;

FIG. 7 is a flow chart showing an even further embodiment of the invention for collecting and cryopreserving menstrual stem cells;

FIG. 8 is a flow chart showing yet an even further embodiment of the invention for collecting and cryopreserving menstrual stem cells;

FIG. 9 is a flow chart showing an additional embodiment of the invention for collecting and cryopreserving menstrual stem cells;

FIG. 10 is a flow chart showing an further additional embodiment of the invention for collecting and cryopreserving menstrual stem cells;

FIG. 11 is a flow chart showing an embodiment of the invention for collecting menstrual stem cells and preparing the menstrual stem cells for therapeutic use;

FIG. 12 is a flow chart showing a further embodiment of the invention for collecting menstrual stem cells and preparing the menstrual stem cells for therapeutic use;

FIG. 13 is a flow chart showing a yet a further embodiment of the invention for collecting menstrual stem cells and preparing the menstrual stem cells for therapeutic use;

FIG. 14 is a flow chart showing an even further embodiment of the invention for collecting menstrual stem cells and preparing the menstrual stem cells for therapeutic use;

FIG. 15 is a flow chart showing yet an even further embodiment of the invention for collecting menstrual stem cells and preparing the menstrual stem cells for therapeutic use;

FIG. 16 is a flow chart showing an additional embodiment of the invention for collecting menstrual stem cells and preparing the menstrual stem cells for therapeutic use;

FIG. 17 is a flow chart showing an additional further embodiment of the invention for collecting menstrual stem cells and preparing the menstrual stem cells for therapeutic use;

FIG. 18 is a flow chart showing a further embodiment of the invention for collecting menstrual stem cells and preparing the menstrual stem cells for therapeutic use;

FIG. 19 is a flow chart showing another embodiment of the invention for collecting menstrual stem cells and preparing the menstrual stem cells for therapeutic use;

FIG. 20 is a flow chart showing an embodiment of the invention for thawing cryopreserved menstrual stem cells in preparation for therapeutic use;

FIG. 21 is a flow chart showing another embodiment of the invention for preparing cryopreserved menstrual stem cells for therapeutic use;

FIG. 22 is a flow chart showing yet another embodiment of the invention for preparing cryopreserved menstrual stem cells for therapeutic use;

FIG. 23 is a flow chart showing yet a further embodiment of the invention for preparing cryopreserved menstrual stem cells for therapeutic use;

FIG. 24 is a flow chart showing an even further embodiment of the invention for preparing cryopreserved menstrual stem cells for therapeutic use;

FIG. 25 is a flow chart showing a further embodiment of the invention for preparing cryopreserved menstrual stem cells for therapeutic use;

FIG. 26 is a flow chart showing yet an even further embodiment of the invention for preparing cryopreserved menstrual stem cells for therapeutic use;

FIG. 27 is a flow chart showing another embodiment of the invention for preparing cryopreserved menstrual stem cells for therapeutic use;

FIG. 28 is a flow chart showing another further embodiment of the invention for preparing cryopreserved menstrual stem cells for therapeutic use;

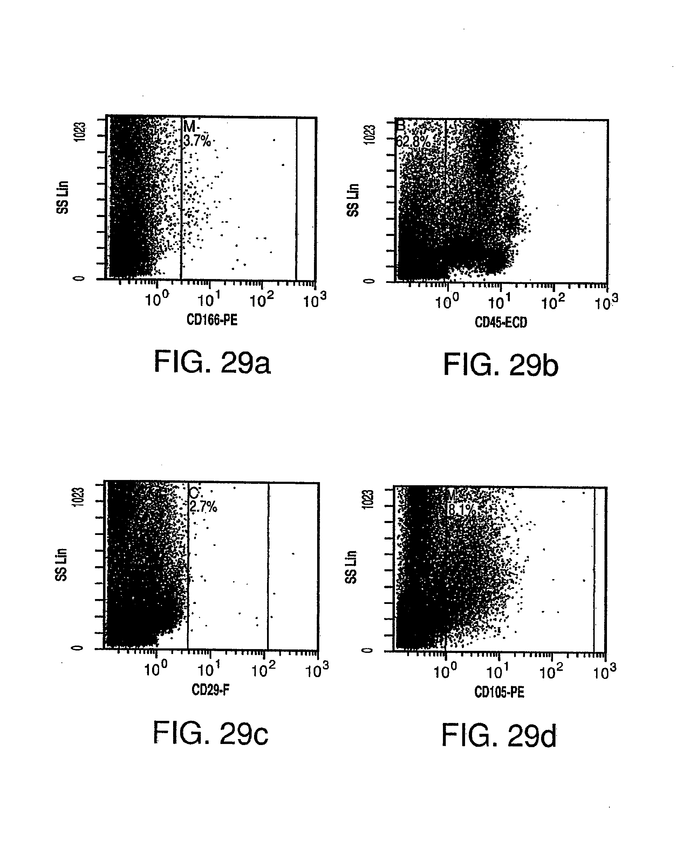

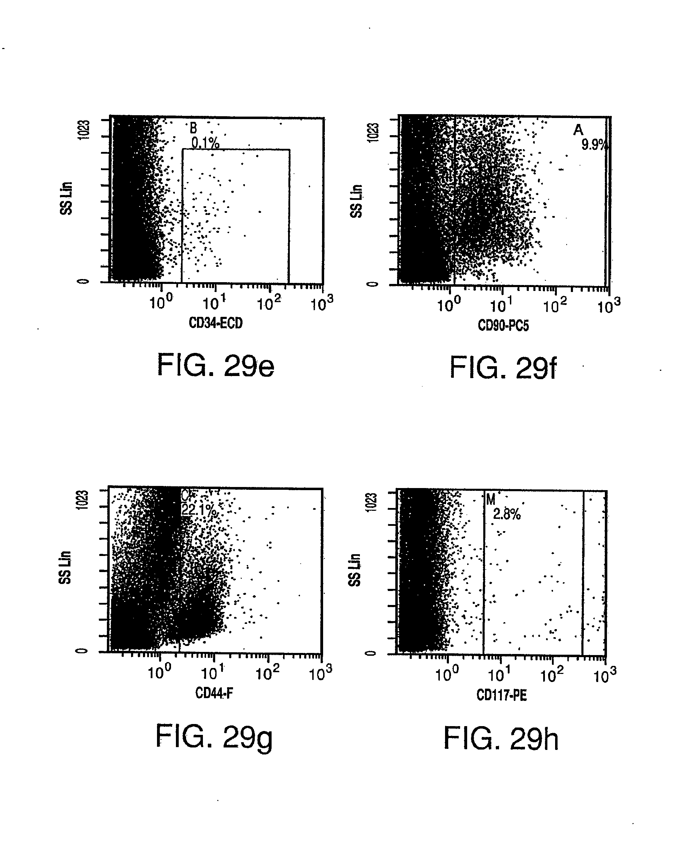

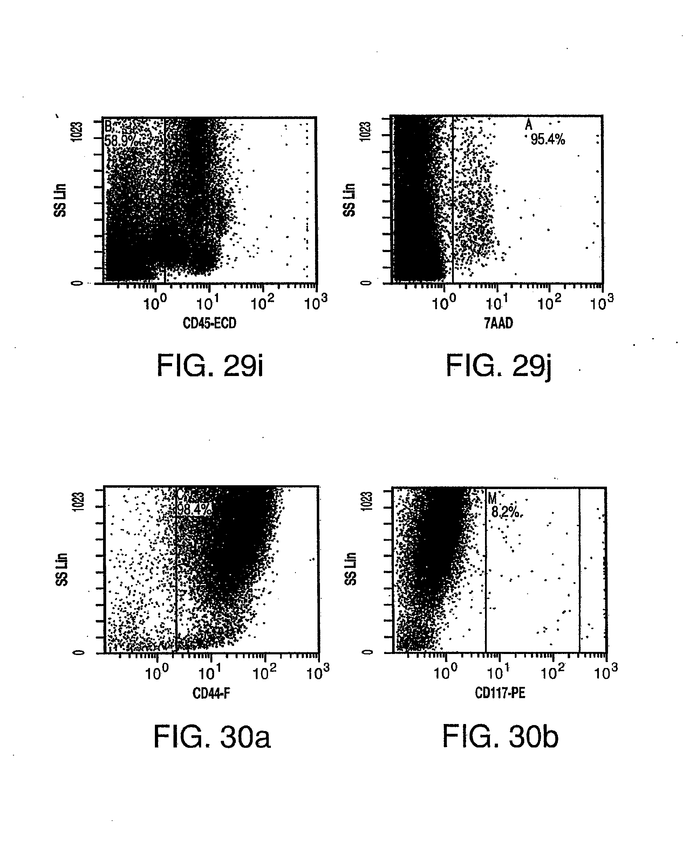

FIGS. 29a through 29j show the results of flow cytometry analysis for the post-processing sample and for the isotype in reference to M2-01 as set forth below. The post-processing sample was collected from M2-01 as about one ml of cellular suspension and placed in an analysis tube for flow cytometry analysis. The results indicate a concentration of CD117+ cells at 0.4% of the TNC population, a concentration of CD44+ cells at 17.7% of the TNC population, a concentration of CD166+ cells at 3.6% of the TNC population, a concentration of CD105+ cells at 8.0% of the TNC population, a concentration of CD90+ cells at 6.8% of the TNC population, a concentration of CD29+ cells at 0.1 % of the TNC population, a concentration of CD34+ cells at 0.1 % of the TNC population, and a 95.4% cell viability as determined by 7AAD;

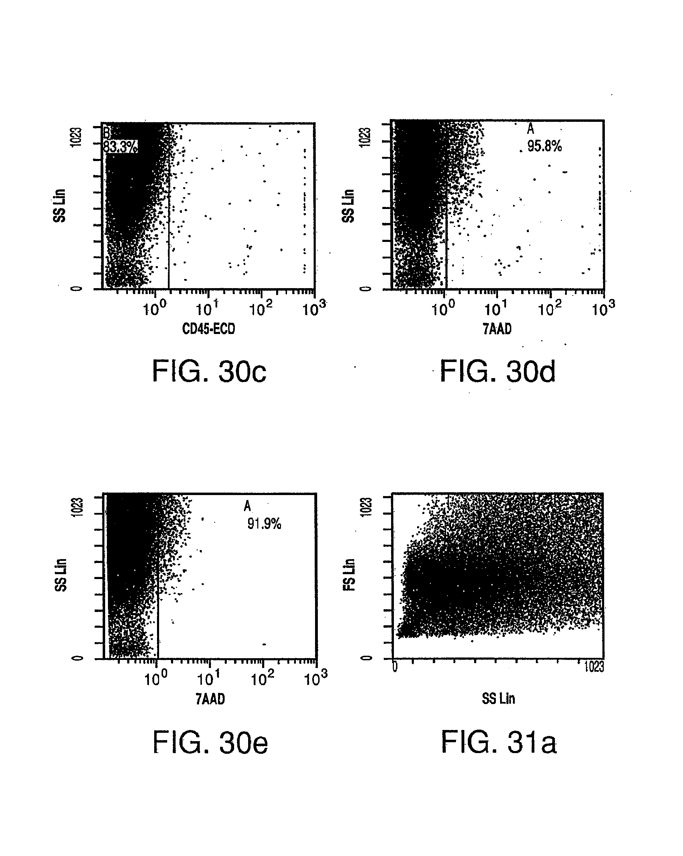

FIGS. 30a through 30e show the results of flow cytometry analysis in reference to cells in a positive fraction obtained by way of the CD 117 selection methods of the invention. After CD117 selection, about one ml of cellular suspension was collected and placed in an analysis tube for flow cytometry analysis. The results indicate a concentration of CD117+ cells at 7.2% of the TNC population, a concentration of CD44+ cells at 93.6% of the TNC population and 95.8% cell viability as determined by 7AAD. The results were calculated by running the processing sample in accordance with the flow cytometry analysis of the invention and subtracting background calculations obtained with a post-processing sample run with an isotype of an IgG antibody;

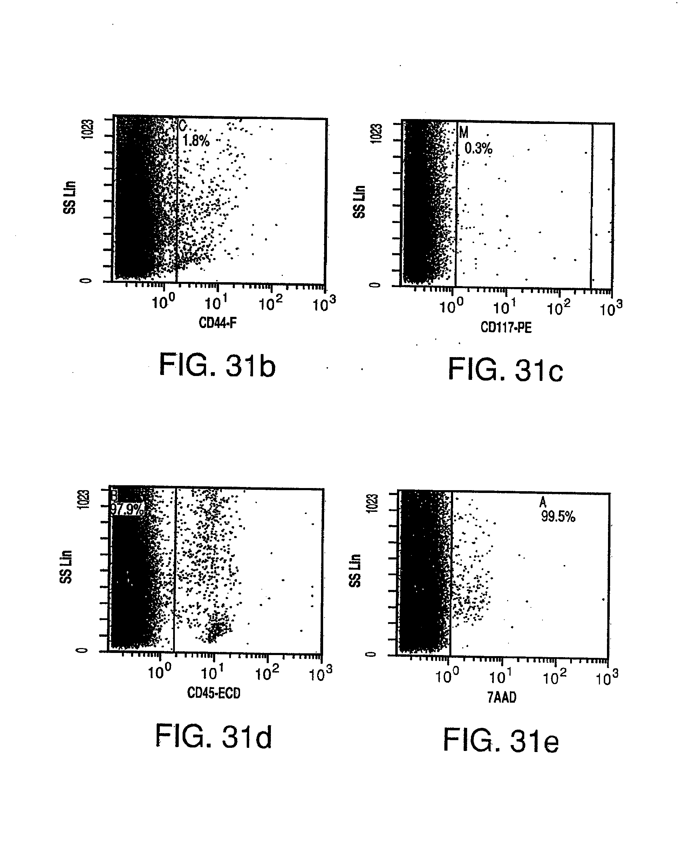

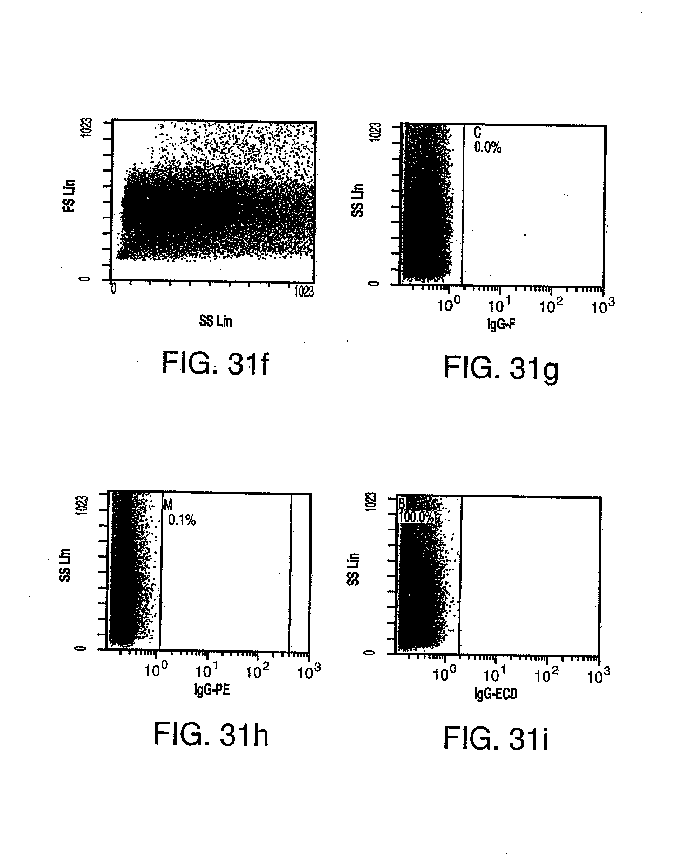

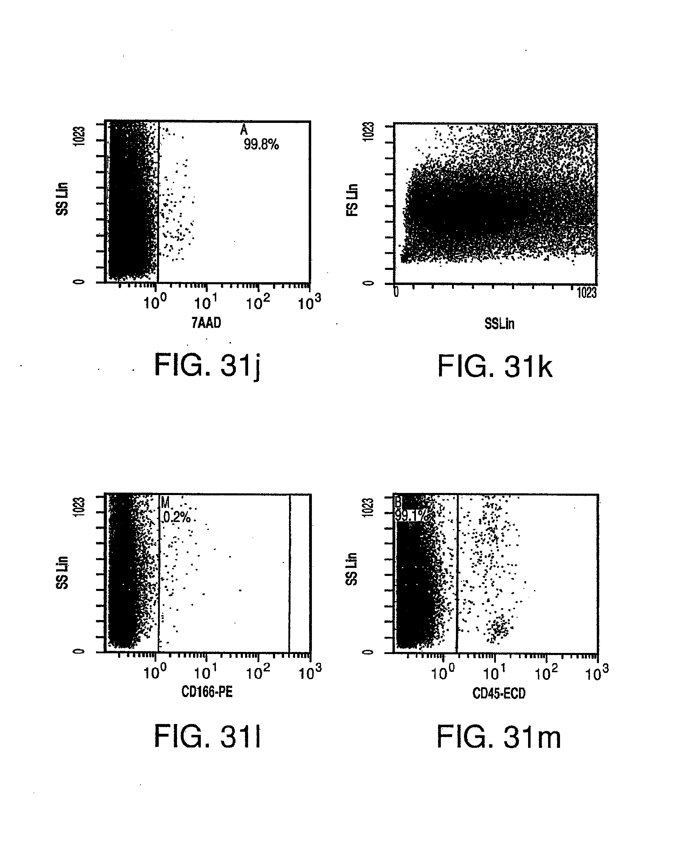

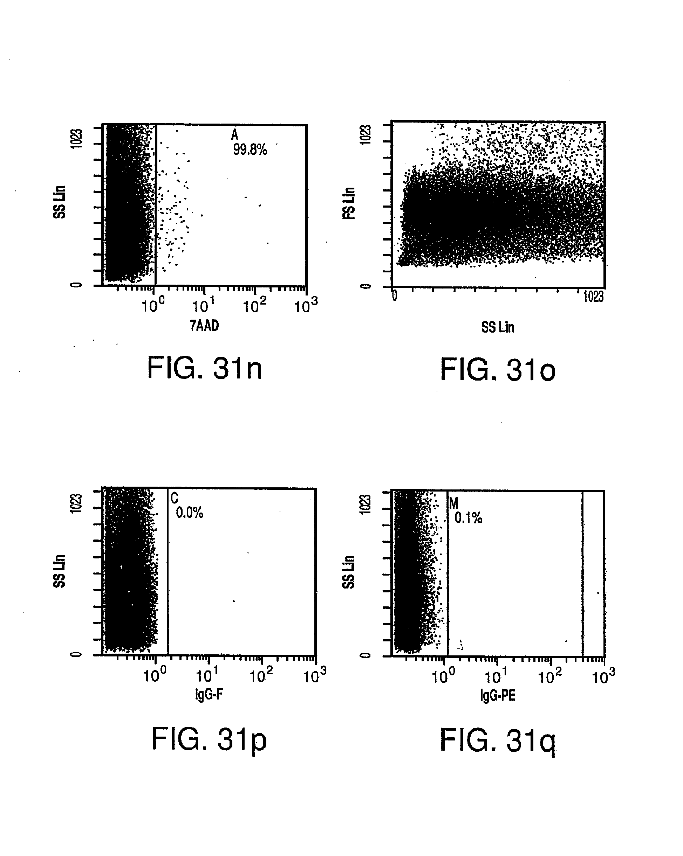

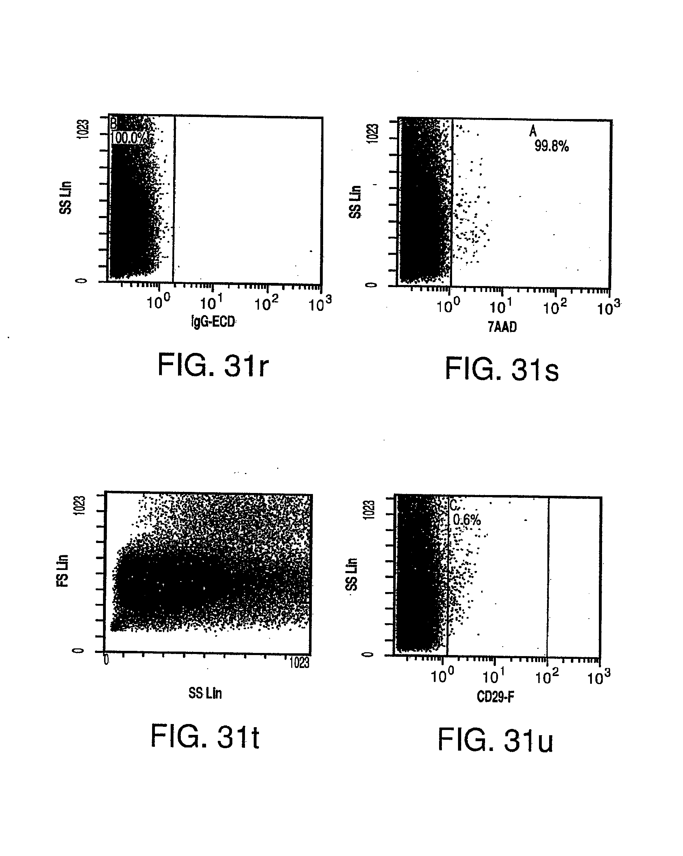

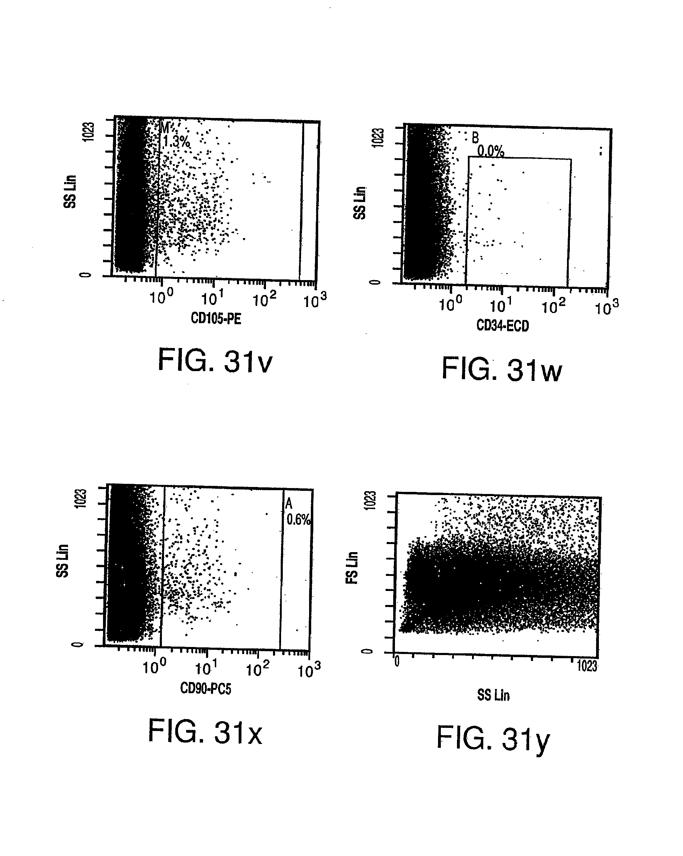

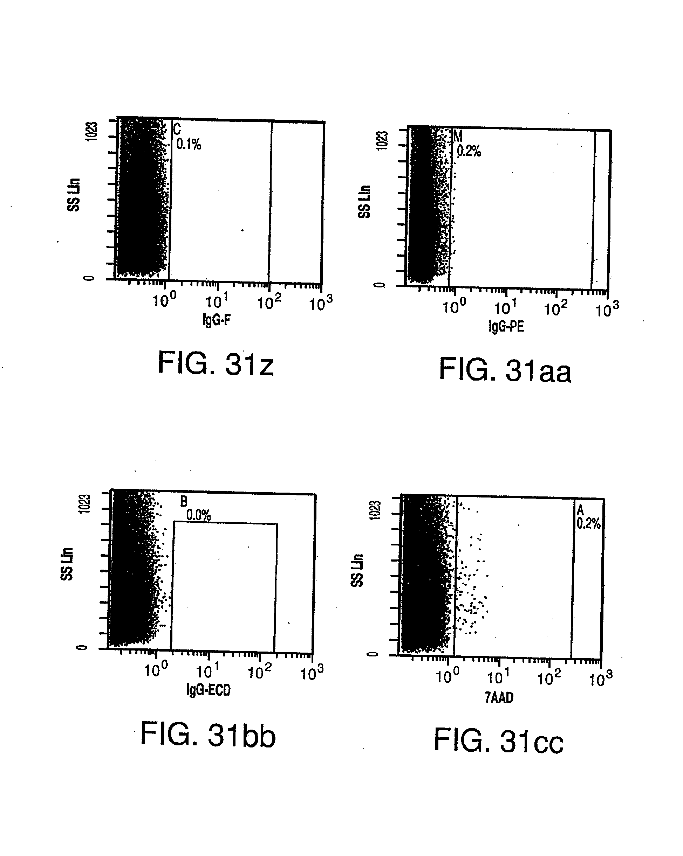

FIGS. 31a through 31cc illustrate the results of flow cytometry analysis of the post-processing sample and for the isotype in reference to M2-02C as set forth below. The post-processing sample was collected as about one ml of cellular suspension and placed in an analysis tube for flow cytometry analysis. The results indicate a concentration of CD117+ cells at 0.2% of the TNC population, a concentration of CD44+ cells at 1.8% of the TNC population, a concentration of CD166+ cells at 0.1% of the TNC population, a concentration of CD105+ cells at 1.1% of the TNC population, a concentration of CD90+ cells at 0.4% of the TNC population, a concentration of CD29+ cells at 0.5% of the TNC population, a concentration of CD34+ cells at 0.0% of the TNC population, and a 99.5% cell viability as determined by 7AAD;

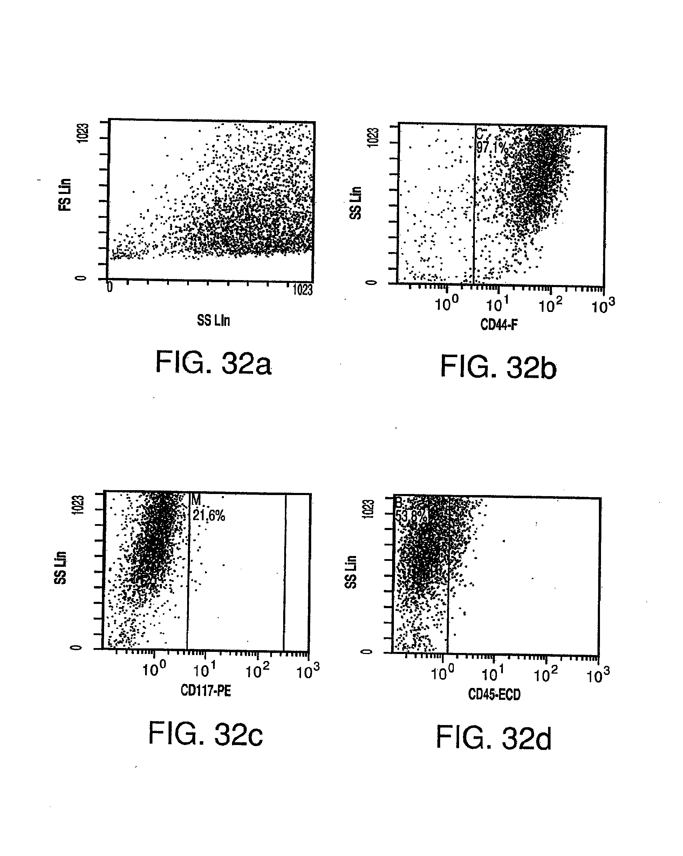

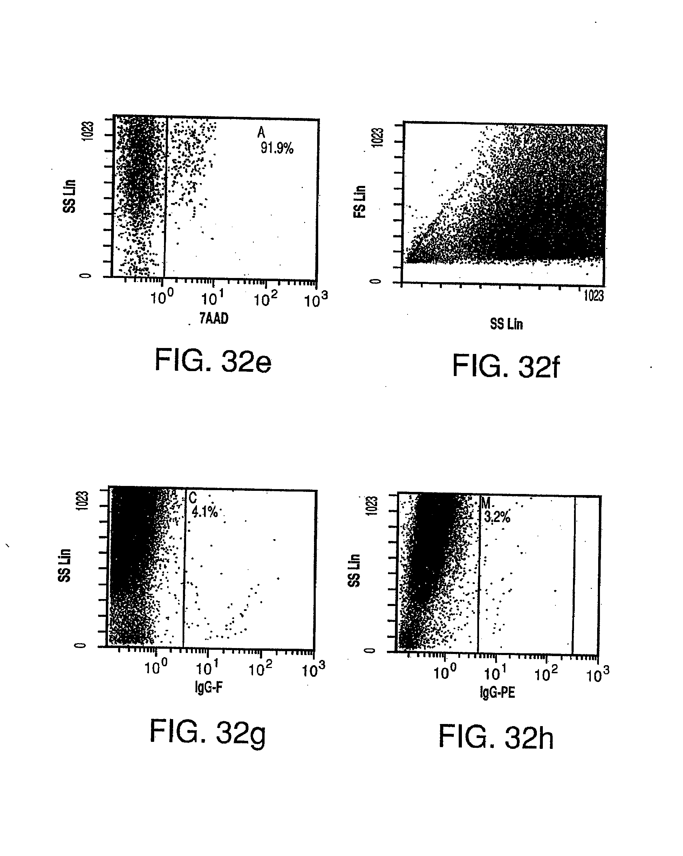

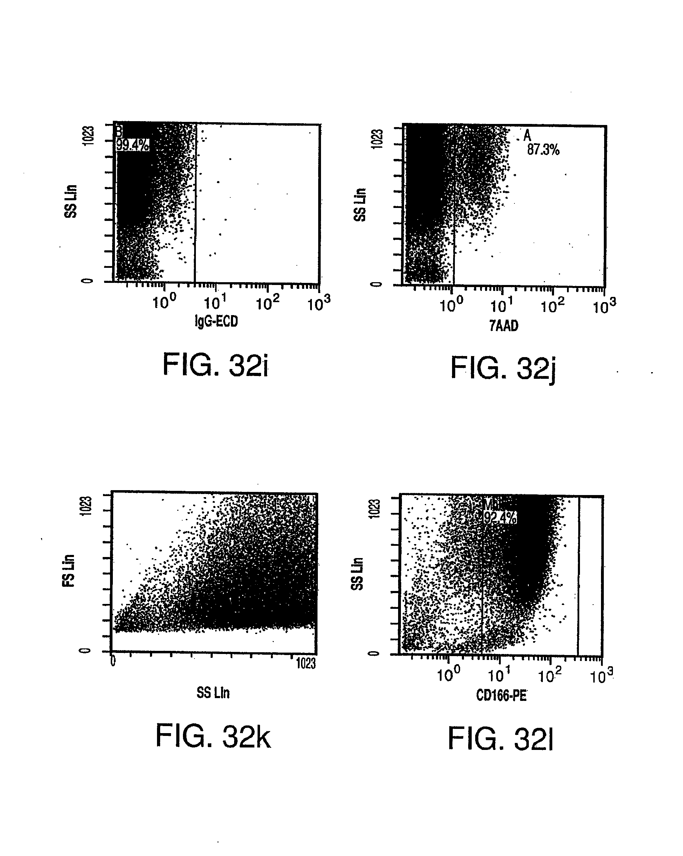

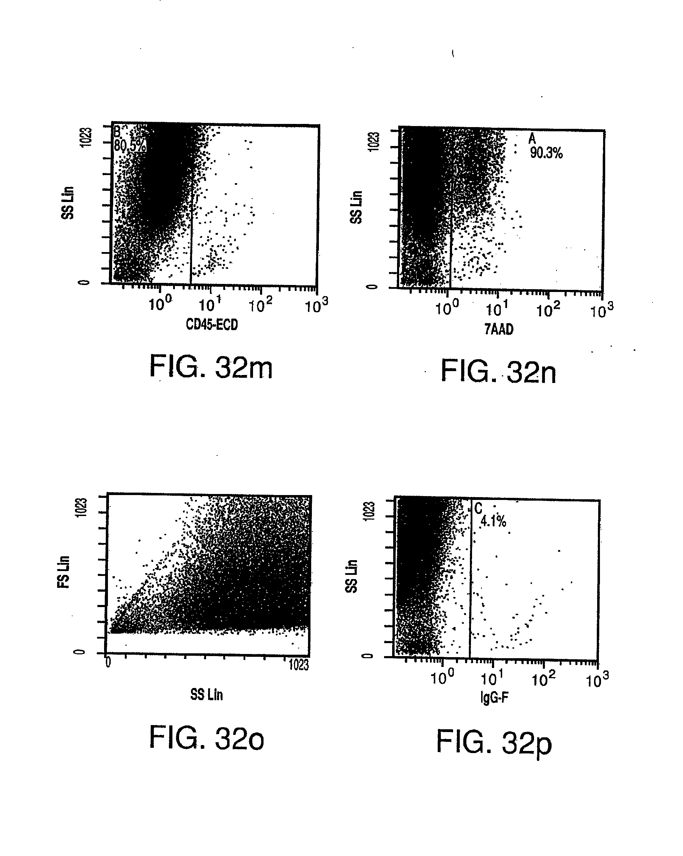

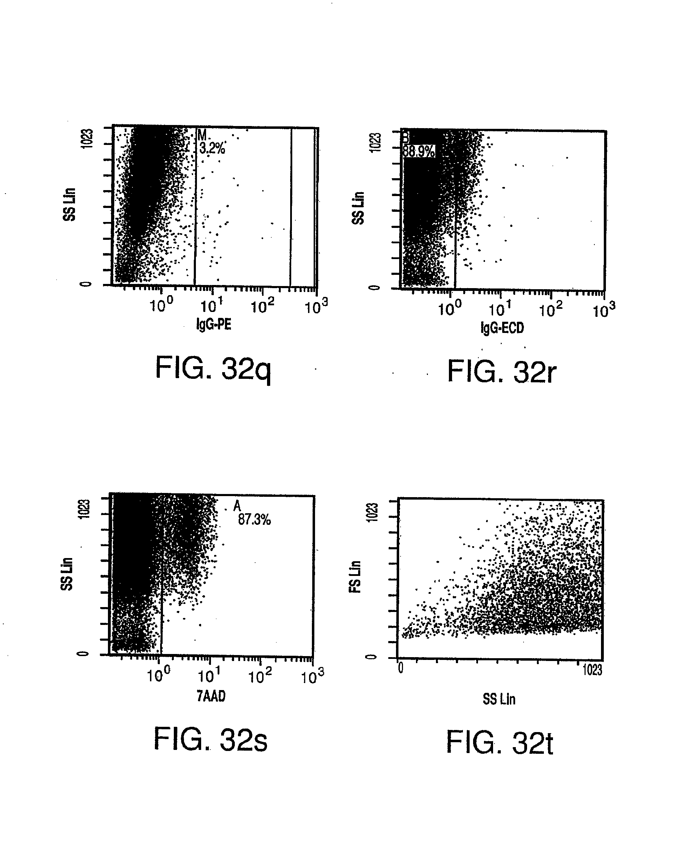

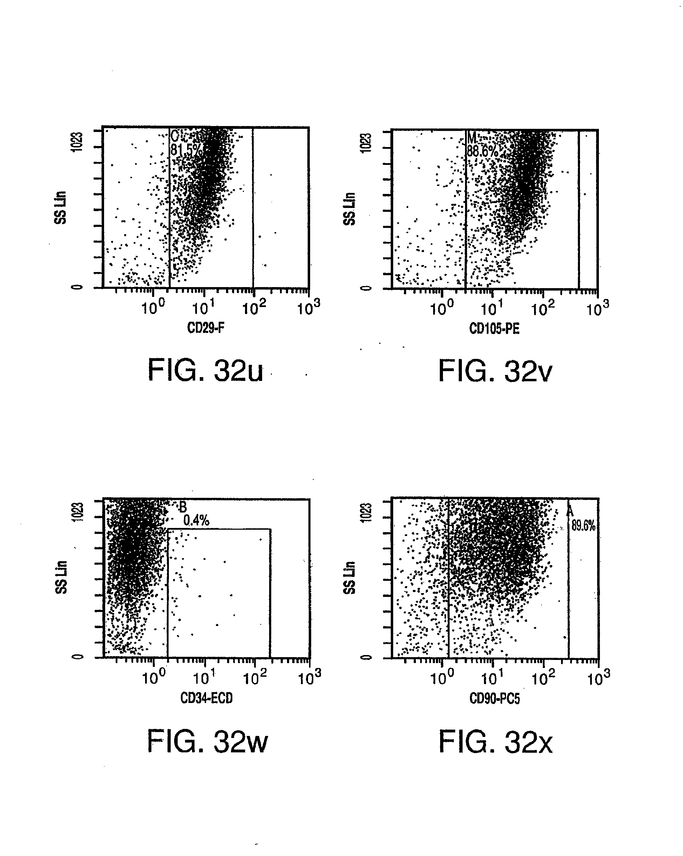

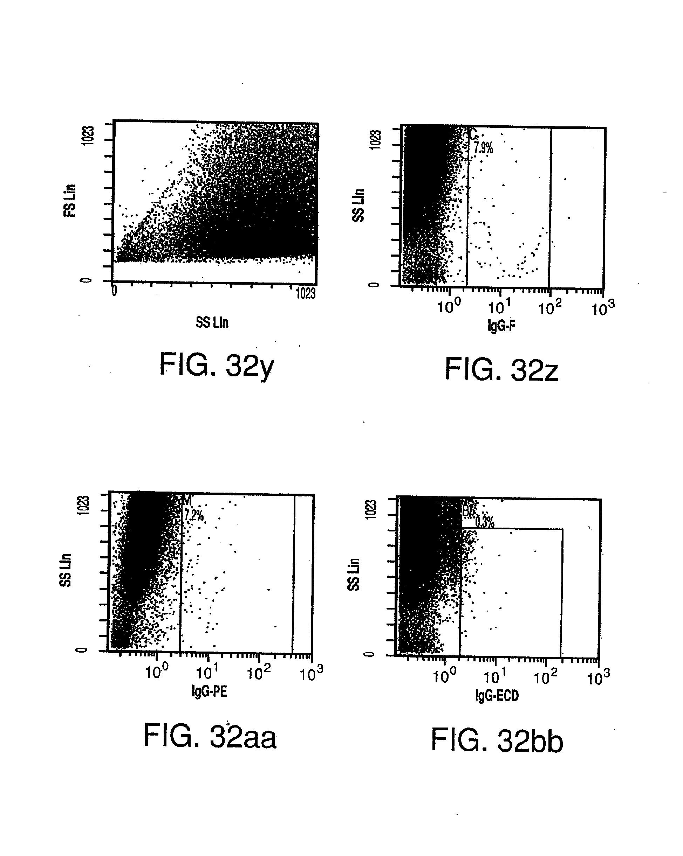

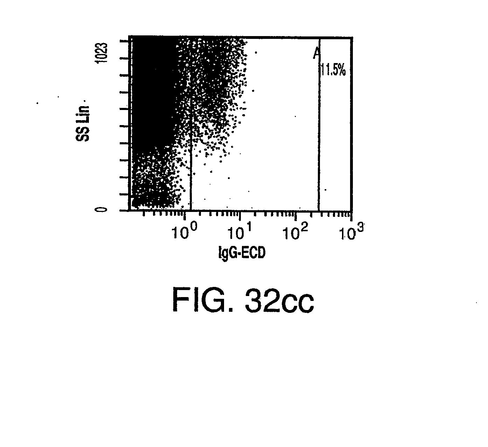

FIGS. 32a through 32cc illustrate the results of flow cytometry analysis in reference to cells in a positive fraction obtained by way of the CD117 selection methods of the invention. The positive fraction was collected as about one ml of cellular suspension and placed in an analysis tube for flow cytometry analysis. The results indicate a concentration of CD117+ cells at18.40% of the TNC population, a concentration of CD44+ cells at 93.0% of the TNC population, a concentration of CD166+ cells at 89.2% of the TNC population, a concentration of CD105+ cells at 81.4% of the TNC population, a concentration of CD90+ cells at 78.1% of the TNC population, a concentration of CD29+ cells at 73.6% of the TNC population, a concentration of CD34+ cells at 0.1% of the TNC population, and 91.9% cell viability as determined by 7AAD. The results were calculated by running the processing sample in accordance with the flow cytometry analysis of the invention and subtracting background calculations obtained with a post-processing sample run with an isotype of an IgG antibody;

FIG. 33 illustrates the results of flow cytometry analysis of cells in reference to M2-03E after several passages in culture, cryopreservation, thaw, and a post-thaw passage as described in further detail herein. The flow cytometry results showed that the cells expressed MHC I, CD44, CD29, CD90, SSEA-4, CD105, CD49f, CD9, CD166, CD166 and CXCR4 and expressed low or no CD133, MHC II, CD34, CD45, and CD38, as shown hereby;

FIGS. 34a through 34f show that menstrual stem cells M2-03E stained positive for CD117 as shown in FIG. 34a and SSEA-4 as shown in FIG. 34c, and also CD117 and SSEA-4 colocalization as shown in FIG. 34e. The negative controls did not stain positive for CD117 as shown in FIG. 34b, SSEA-4 as shown in FIG. 34d, or colocalization of CD117 and SSEA-4 as shown in 34f;

FIGS. 35a and 35b show that menstrual stem cells M2-03E grew rapidly and express multipotent markers. FIG. 35a shows that the menstrual stem cells M2-03E grew rapidly in pre-cryopreservation culture with a doubling time of about 24 hours to about 36 hours. This rapid growth allowed for expansion from 50,000 cells to 48 million cells in eight doublings. FIG. 35b shows that the menstrual stem cells M2-03E express Oct-4 at the RNA level up to about 12 passages in culture as determined by RT-PCR L, ladder; water; ESC, embryonic stem cells; P12 passage 12;

FIGS. 36a through 36g show that menstrual stem cells M2-03E differentiated into neural tissue. Menstrual stem cells were neural induced to differentiate into oligodendroglial cells expressing 04 and GalC as shown in FIGS. 36a through 36c. The menstrual stem cells also expressed Map-2 as shown in FIG. 36d, Tub-3 as shown in FIG. 36e, and Vimentin as shown in FIG. 36f. RT-PCR results of the differentiated cells show RNA level expressions typically for neural cells, RT-PCR: L, ladder; W, water; B, brain control; neural induced menstrual stem cells;

FIGS. 37a through 37d show that menstrual stem cells M2-03E differentiated into cardiac tissue. Menstrual stem cells were treated with 8uM Aza and exhibited strong expression of actin shown through immunocytochemistry in FIG. 37b. Troponin expression as shown in FIG. 37a, and connexin 43 expression as shown in FIG. 37c was stimulated by treatment of the cells with 800 uM SNAP. RT-PCR was confirmed the cardiac differentiation as shown in FIG. 37d, RT-PCR: L, ladder; W, water; H, heart control; Cardiac induced;

FIGS. 38a through 38f show that the menstrual stem cells M2-03E differentiated into mesoderm type tissues. The cells stained with oil red O for fat vacuoles indicating differentiation to adipose tissue was induced as shown in FIG. 38b in comparison to the controls as shown in FIG. 38a. The cells stained with alcian blue indicating differentiation to chondrogenic tissue when induced as shown in FIG. 38d in comparison to the controls as shown in FIG. 38c. The cells stained with alizarin red S indicating the presence of calcium deposits as shown in FIG. 38f in comparison to FIG. 38e;

FIG. 39 is a graph showing that menstrual stem cells of M2-03E express telomerase activity at passage 12 of post-thaw culture; hESC, human embryonic stem cells; MEF, mouse embryonic fibroblasts; MenSCs P12;

FIG. 40 is a graph showing the results of a study performed with 161 samples that were divided into 3 groups and collected under preset conditions to analyze variables associated with the menstrual stem cell collection methods of the present invention. The study was performed to determine the effects of using a collection media with and without antibiotics and shipping menstrual flow samples with and without ice. FIG. 40 shows that total nucleated cell count analysis averaged about 7.9 million cells per sample collected across all samples collected for the three groups;

FIG. 41 is a graph showing the results of the study referenced in FIG. 40. FIG. 41 shows that the cell viability in post-processing samples averaged about 74% viability across all samples collected for the three groups;

FIG. 42 is a graph showing the results of a study referenced in FIG. 40. FIG. 42 shows that the average temperature for all samples collected for the three groups averaged about 15°C;

FIG. 43 is a graph showing the results of a study referenced in FIG. 40. FIG. 43 shows that the average sample volume for all samples collected for the three groups averaged about 11 ml comprising about 1 ml of menstrual flow sample and about 10 ml of collection media; and

FIG. 44 is a graph showing the results of a study referenced in FIG. 40. FIG. 44 shows that the average sample age calculated from collection to receipt at the processing facility ranged from about less than 5 hours to greater than 140 hours.

### DETAILED DESCRIPTION OF EMBODIMENTS OF THE INVENTION

Understanding of the present invention will be facilitated by consideration of the following detailed description of embodiments of the present invention taken in conjunction with particular exemplary embodiments as described in the accompanying description and figures. It is to be understood that the figures and descriptions of the present invention have been simplified to illustrate elements that are relevant for a clear understanding of the present invention.

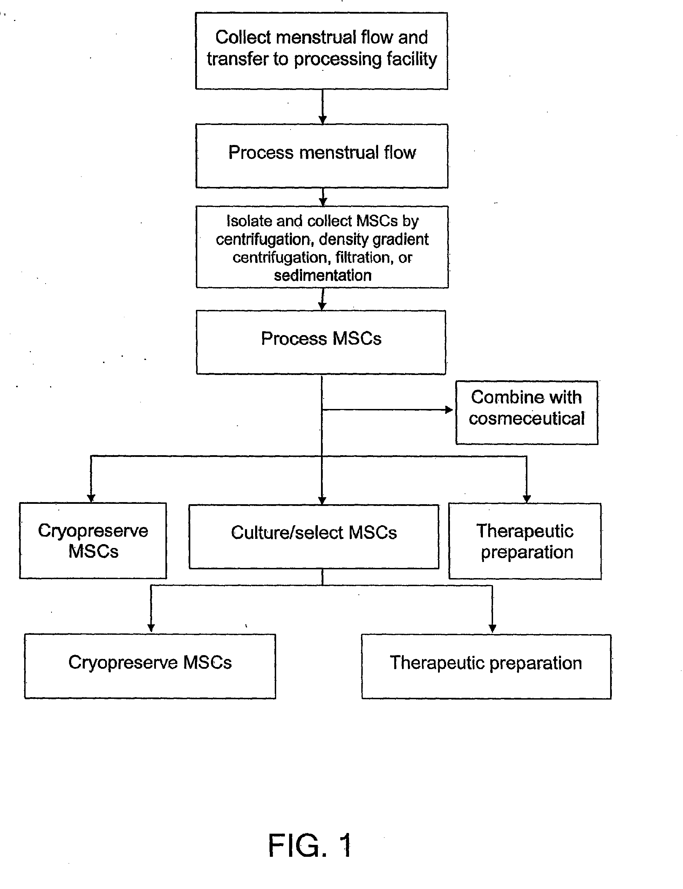

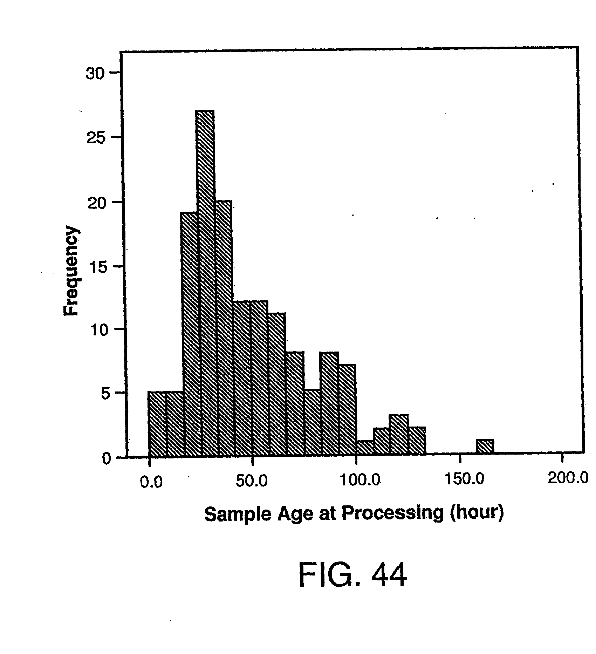

Referring now to FIGS. 1 through 44, the present invention provides methods, processes, systems, and compositions associated with the procurement of menstrual stem cells isolated from blood, cells, fluid, and tissue shed during menstruation, also referred to herein in this disclosure as "menstrual flow", and collected according to the methods, processes, and systems of the invention. As used throughout the disclosure of the invention, the phrases "endometrial/menstrual cell," "endametrial/menstrual stem cell," "menstrual stem cell," and "menstrual cell", and the terms "MSC" and "cell" are used generally throughout in reference to any one or more cells collected from menstrual flow that express at least one of the cell markers or intracellular markers including, but not limited to, CD9, CD10, CD13, CD29, CD44, CD49e, CD49f, CD59, CD81, CD105, CD166, and HLA class I, while expressing low or no levels of CD3 and MHC II. While the term "cell" is used in the singular sense, it may also be used in the plural sense to refer to more than one cell of the invention. While the aforementioned characteristics of the cell are provided as exemplary characteristics, additional cell surface characteristics of menstrual stem cells are provided throughout the disclosure of the invention herein, including, but not limited to, the characteristics provided on any table and figures hereto, and provide further disclosure regarding the menstrual stem cells of the invention.

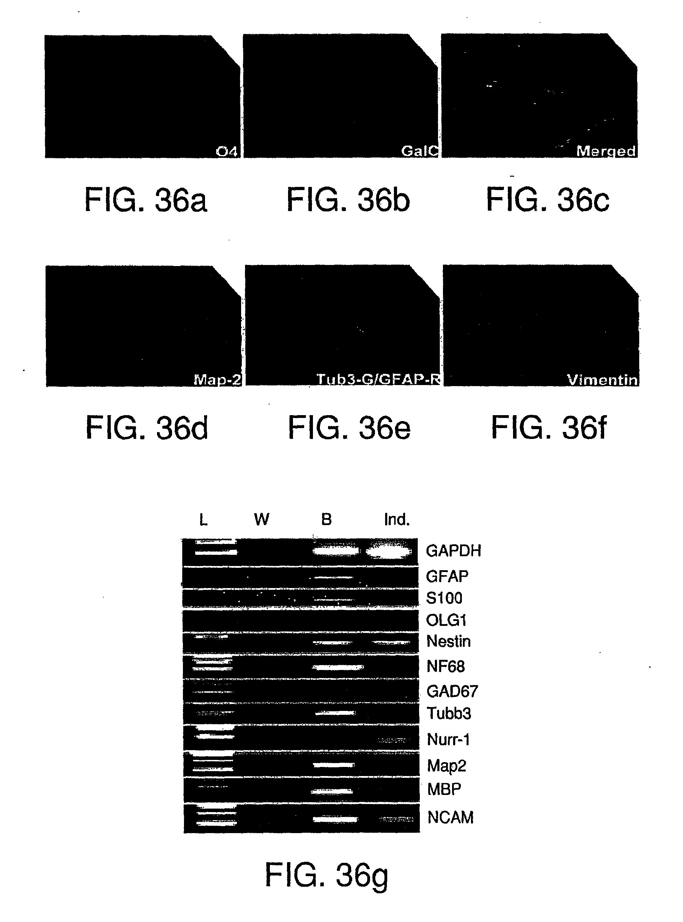

Referring now to FIGS. 36 to 38 in particular, it is recognized that some stem cells are pluripotent in nature due to the capability of the cells to differentiate into a number of distinct cell types. A pluripotent cell possesses the capacity to undergo differentiation into a number of different mammalian cell types. In particular, the MSCs of the present invention also express an ability to differentiate into various distinct cell lineages, such as, for example, neural, cardiogenic, chondrogenic, adipogenic, and osteogenic lineages, as illustrated in FIGS. 36 through 38.

It is believed that MSCs of the present invention may be capable of differentiating into any of the 260 somatic cells in the body. For example, the cells may be able to differentiate into at least hepatic, pancreatic, myogenic, osteogenic, chondrogenic, adipocytic, epithelial, neural, keratinocytes, and cardiomyocytes. The cells may also possess the capacity to differentiate when cultured with other predisposed cells, such as hepatic, pancreatic, myogenic, osteogenic, chondrogenic, adipocytic, epithelial, neural, keratinocytes, and cardiomyocytes, as seen in a co-culture system. Cells obtained from differentiation and cells obtained from co-culture may also have the potential to be used in treatment for replacement or regeneration therapies, other therapeutic applications, cosmeceutical, organ rejection therapies, and other applications.

In particular, the potential use of MSCs in therapeutic, cosmeceutical and other applications is beneficial for a number of reasons, because MSCs are, (a) easily obtained from a non-controversial source otherwise regarded as biological waste, (b) effectively collected by a donor without the need for medical training, (c) highly proliferative, (d) capable of differentiation into desired cell types, (e) capable of autologous applications, (f) capable of allogeneic applications, (g) able to provide a single cell line that may be used as a potential source multiple therapies, (h) capable of potential co-culture with other healthy cells, (i) able to be used as a source for customized regenerative healthcare solutions, and (j) capable of ready and multiple collections on a regular cycle over decades.

The use of stem cells obtained from other sources have been used in various forms of regenerative medicine for the following conditions: diabetes, myocardial infarction, valve/artery replacement, Parkinson's, Alzheimer's, MS, stroke therapy, insulin regeneration, anti-aging serums, burn/wound healing bandages, acne management, vitiligo, hair replacement, tooth regeneration, breast replacement/augmentation, liver cirrhosis, penile augmentation, sports therapy, anemia, leukemia, lymphoma, lung fibrosis, sickle cell anemia, bone replacement, osteoporosis, scoliosis, rheumatoid arthritis, spinal cord injury, knee/hip/elbow replacement, hormonal replacement therapy, PMS/depression management, autism, sterility therapy, vision regeneration, and lens replacement.

Generally, and in an embodiment, the present invention provides methods for obtaining and collecting MSCs from menstrual flow. In another embodiment, the present invention provides a system for collecting MSCs from menstrual flow. In yet another embodiment, the present invention provides a method for cryopreserving MSCs collected from menstrual flow. In yet further embodiments, products comprising populations of MSCs may be collected by various processes for obtaining and isolating MSCs from menstrual flow. In yet even further embodiments, the present invention provides enriched populations of MSCs and the processes for enriching populations of MSCs obtained from menstrual flow. In even further embodiments, the present invention provides various compositions comprising MSCs and other agents, such as, for example, compositions comprising MSCs and a cryopreservation agent, compositions comprising MSCs and at least one cosmeceutical agent, and compositions comprising MSCs and a preservation agent. These various embodiments of the present invention are described in further detail hereinafter.

An exemplary schematic of the overall methods and processes of the present invention is illustrated in FIG. 1. Generally, various steps may be used to procure menstrual flow and to process the menstrual flow to obtain MSCs that may be further processed for a particular use. In particular, the MSCs may be cryopreserved, enriched through single or multiple steps of cell selection or culture, and/or prepared for therapeutic or cosmeceutical use. Throughout the practice of the present invention, the MSCs and surrounding environment may be subjected to quality control analysis by methods and processes, such as, for example, flow cytometry to characterize collected MSCs and microbiological analysis to identify any possible contamination of samples.

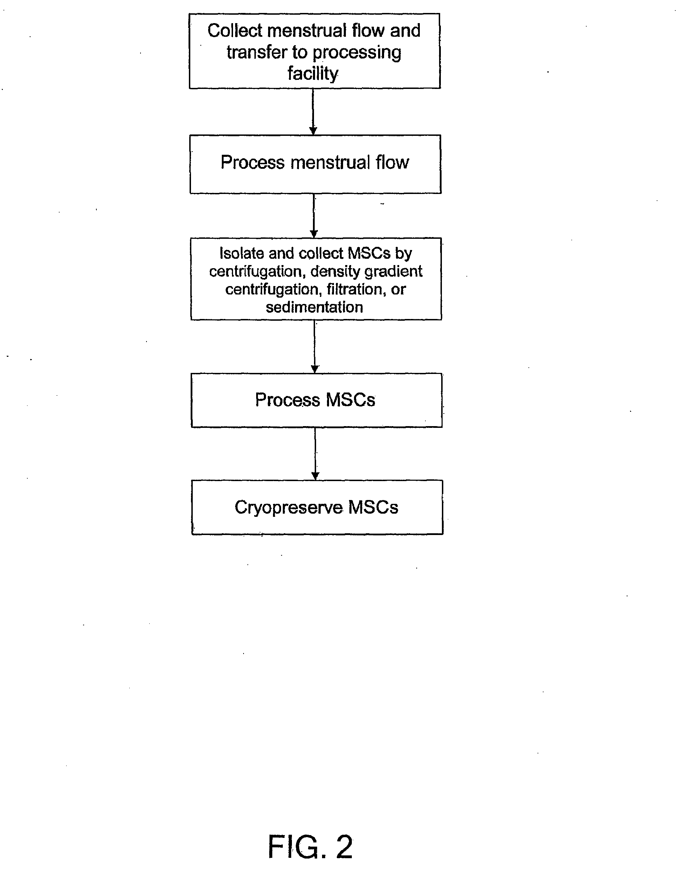

The various embodiments of the present invention depicted in FIG. 1 are described in further detail in FIGS. 2 through 28 along with the written descriptions of working examples of the invention.

Referring now to FIG. 2, the present invention provides methods and processes for cryopreserving MSCs. According to FIG. 2, menstrual flow may be collected with a collection process and then transferred to a processing facility where the menstrual flow may be processed to isolate and collect MSCs. The isolation and collection of MSCs from the menstrual flow may occur by way of centrifugation, density gradient centrifugation, filtration or sedimentation methodologies used to maximize the number of MSCs collected. Once collected, the MSCs may be further processed and combined with a cryopreservation agent in preparation for cryopreservation and are then cryopreserved. Cryopreserved MSCs may then be later thawed for use.

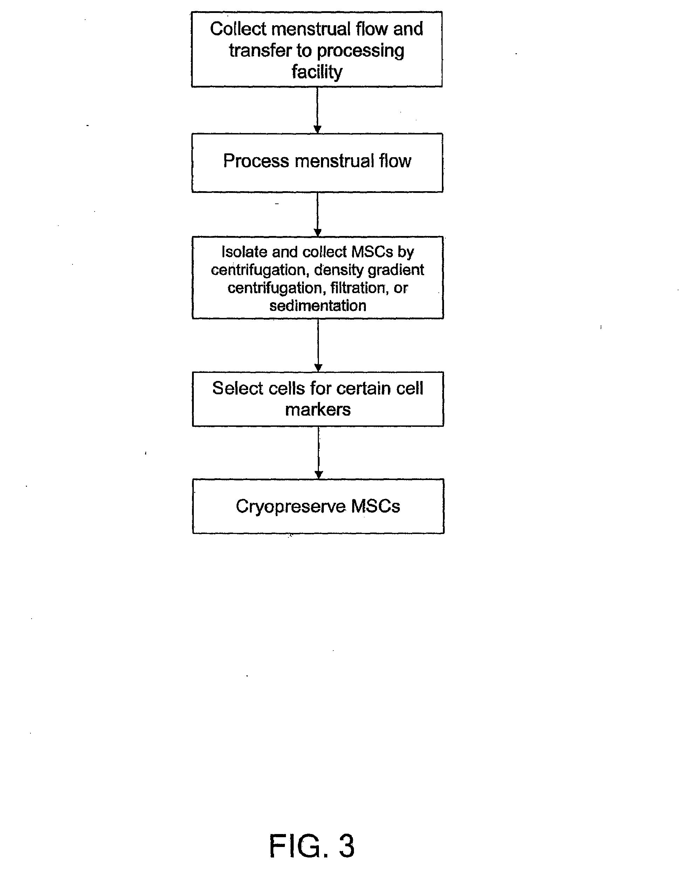

Referring now to FIG. 3, the present invention provides methods and processes for cryopreserving MSCs. According to FIG. 3, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, the MSCs may be further enriched or concentrated by selected MSCs that express certain cell markers, such as, for example, any one or more of the cell markers associated with MSCs. Selected and even unselected MSCs may be processed and combined with a cryopreservation agent in preparation for cryopreservation and then cryopreserved. Cryopreserved MSCs may then be later thawed for use.

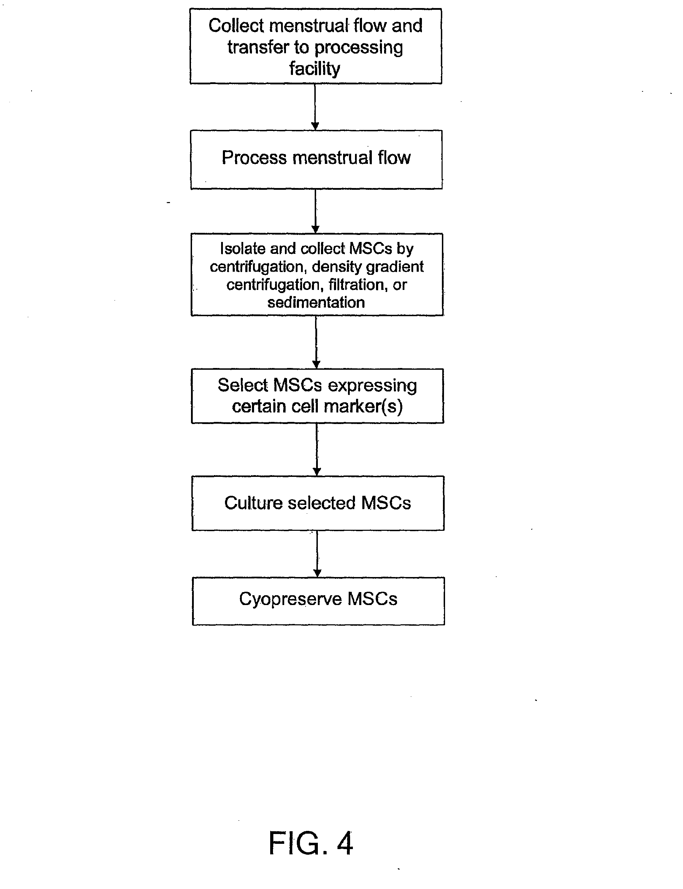

Referring now to FIG. 4, the present invention provides methods and processes for cryopreserving MSCs. According to FIG. 4, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, the MSCs may be further enriched or concentrated by selected MSCs that express certain cell markers, such as, for example, any one or more of the cell markers associated with MSCs. Selected and even unselected MSCs may then be cultured to expand the MSCs. The cultured MSCs may then be combined with a cryopreservation agent in preparation for cryopreservation and then cryopreserved. Cryopreserved MSCs may then be later thawed for use.

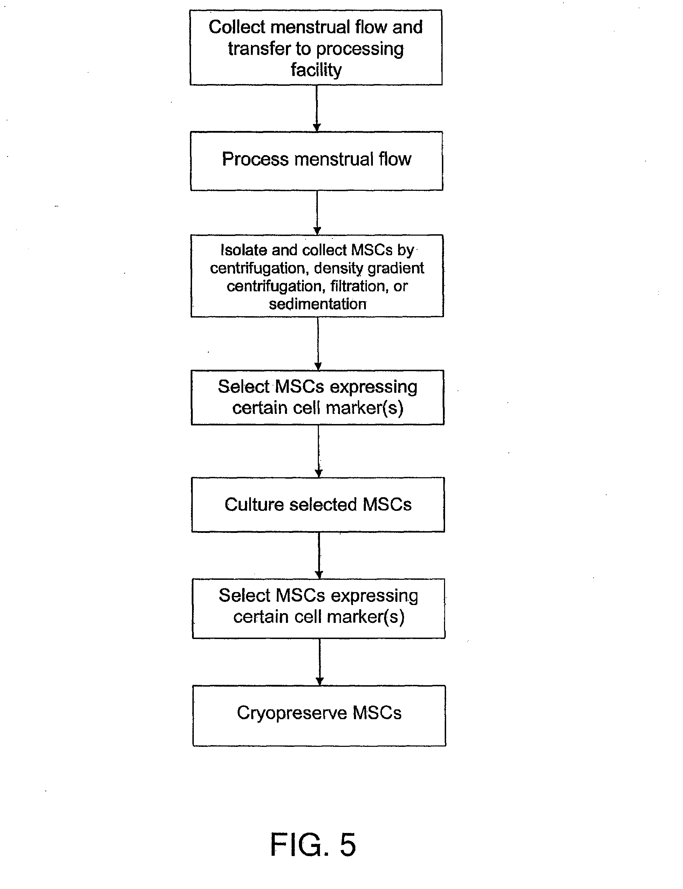

Referring now to FIG. 5, the present invention provides methods and processes for cryopreserving MSCs. According to FIG. 5, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, the MSCs may be further enriched or collected by selected MSCs that express certain cell markers, such as, for example, any one or more of the cell markers associated with MSCs. Selected and even unselected MSCs may then be cultured to expand the MSCs. The cultured MSCs may then be further selected for specific cell surface markers to enrich or collect specific MSCs. The selected and even unselected MSCs may be combined with a cryopreservation agent in preparation for cryopreservation and then cryopreserved. Cryopreserved MSCs may then be later thawed for use.

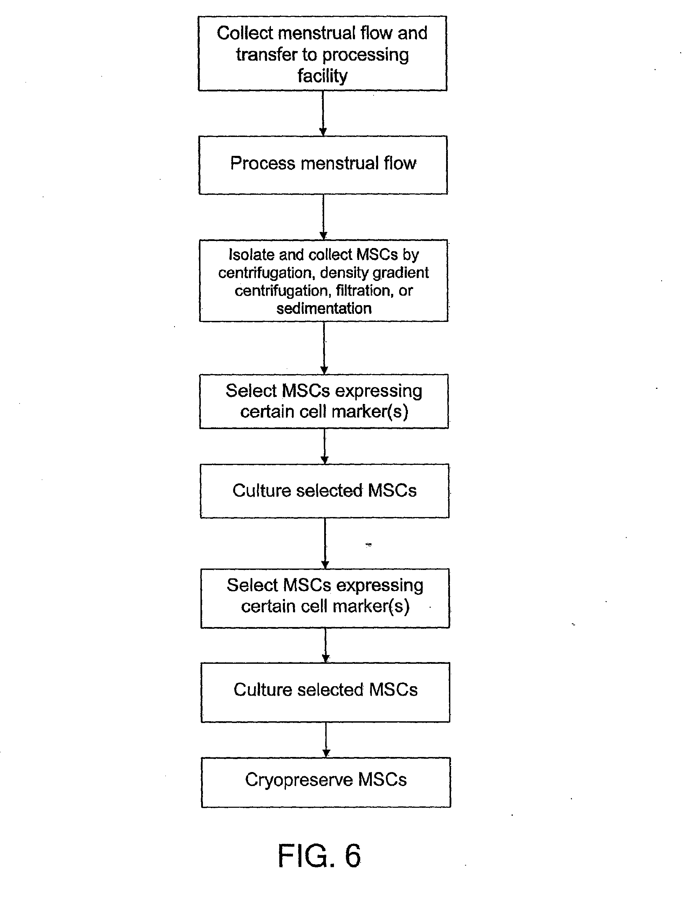

Referring now to FIG. 6, the present invention provides methods and processes for cryopreserving MSCs. According to FIG. 6, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, the MSCs may be further enriched or collected by selected MSCs that express certain cell markers, such as, for example, any one or more of the cell markers associated with MSCs. Selected and even unselected MSCs may then be cultured to expand the MSCs. The cultured MSCs may then be further selected for specific cell surface markers to enrich or collect specific MSCs. The selected and even unselected MSCs may be then cultured further to expand the number of MSCs. The MSCs may then be combined with a cryopreservation agent in preparation for cryopreservation and then cryopreserved. Cryopreserved MSCs may then be later thawed for use.

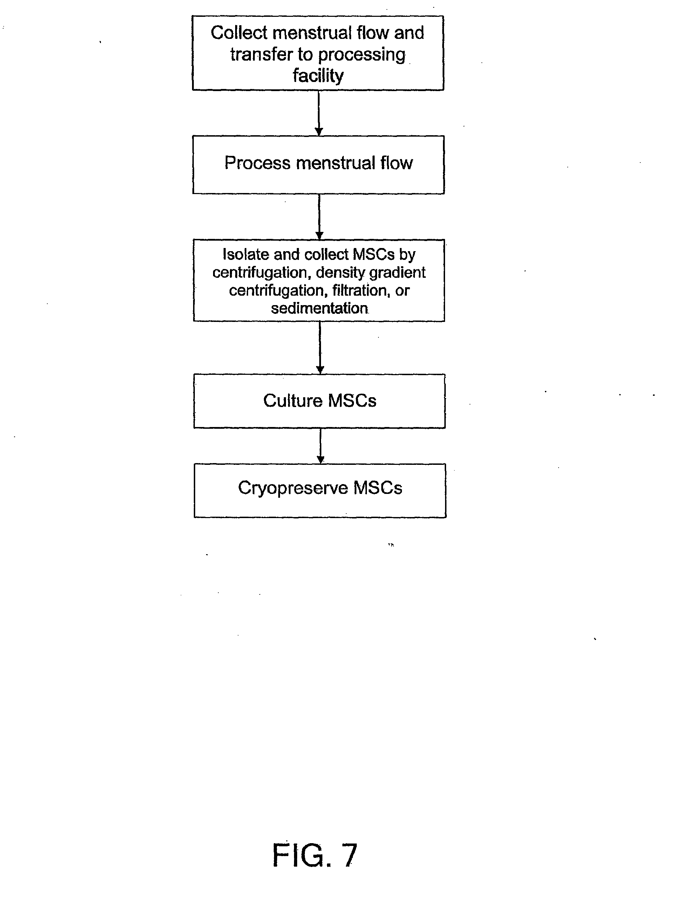

Referring now to FIG. 7, the present invention provides methods and processes for cryopreserving MSCs. According to FIG. 7, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, the MSCs may be cultured to expand the number of MSCs. Cultured MSCs may be processed and combined with a cryopreservation agent in preparation for cryopreservation and then cryopreserved. Cryopreserved MSCs may then be later thawed for use.

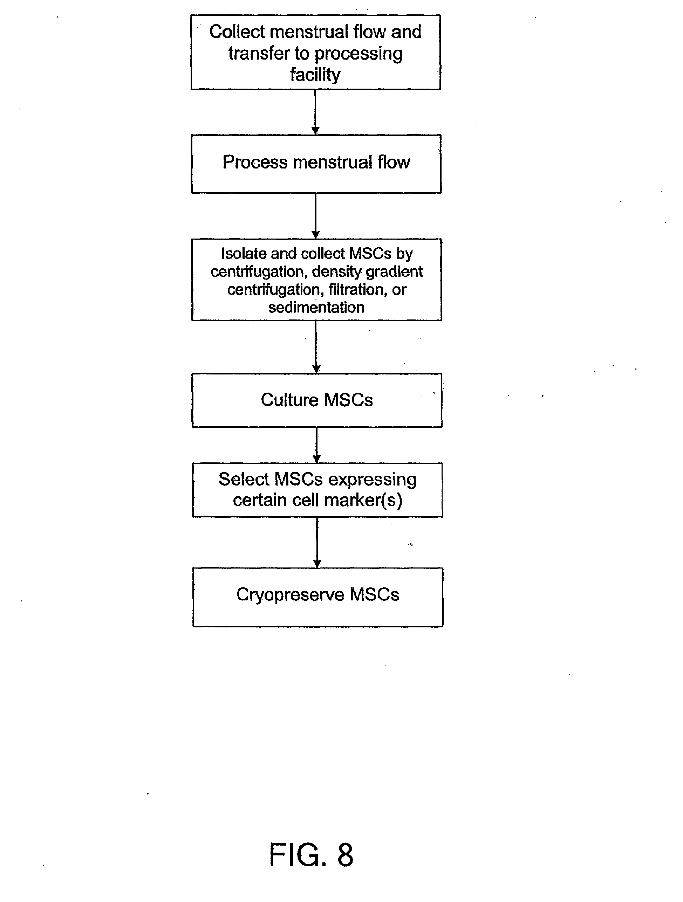

Referring now to FIG. 8, the present invention provides methods and processes for cryopreserving MSCs. According to FIG. 8, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may be enriched or concentrated by selecting MSCs that express certain cell markers, such as, for example, any one or more of the cell markers associated with MSCs. Selected and even unselected MSCs may then be separately combined with a cryopreservation agent in preparation for cryopreservation and then cryopreserved. Cryopreserved MSCs may then be later thawed for use.

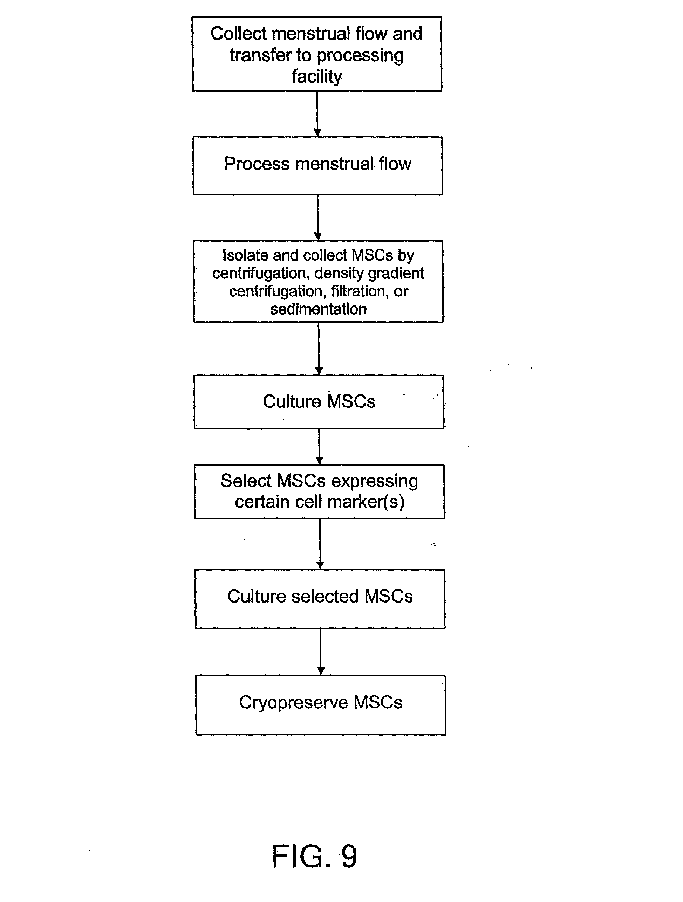

Referring now to FIG. 9, the present invention provides methods and processes for cryopreserving MSCs. According to FIG. 9, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may be enriched or concentrated by selecting MSCs that express certain cell markers, such as, for example, any one or more of the cell markers associated with MSCs. Selected and even unselected MSCs may be then be cultured to expand the MSCs. The selected and even unselected cultures of MSCs may be separately combined with a cryopreservation agent in preparation for cryopreservation and then cryopreserved. Cryopreserved MSCs may then be later thawed for use.

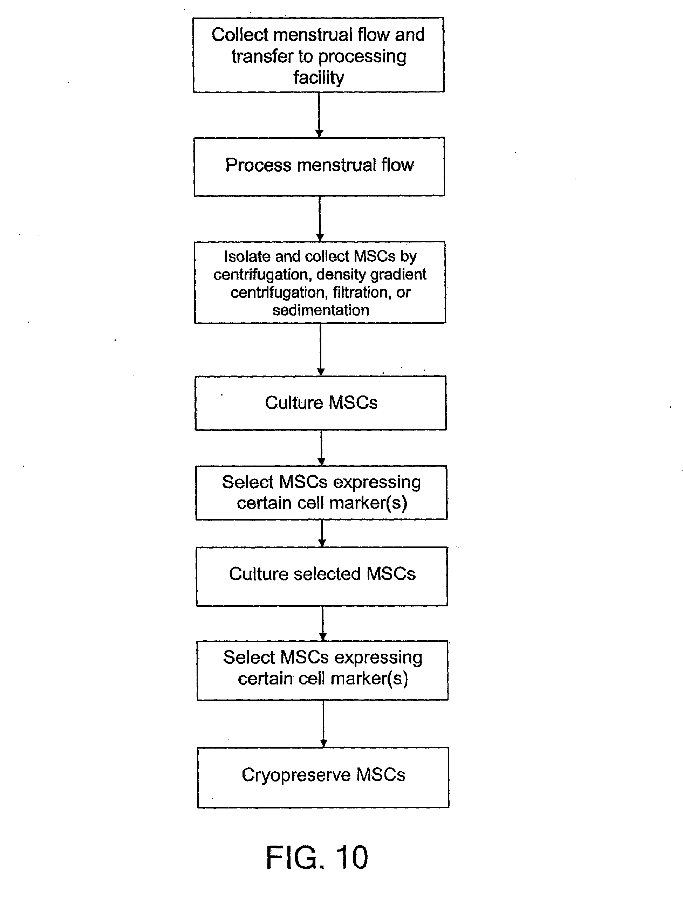

Referring now to FIG. 10, the present invention provides methods and processes for cryopreserving MSCs. According to FIG. 10, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may be enriched or concentrated by selecting MSCs that express certain cell markers, such as, for example, any one or more of the cell markers associated with MSCs. Selected and even unselected MSCs may then be cultured to expand the MSCs. The selected and even unselected cultures of MSCs may be selected further for specific cell markers. The selected and unselected cells may then be separately combined with a cryopreservation agent in preparation for cryopreservation and then cryopreserved. Cryopreserved MSCs may then be later thawed for therapeutic use. Throughout all of the descriptions of the embodiments of the present invention, the phrase "therapeutic use" includes use of MSCs for any type of stem cell therapy and also includes cosmeceutical use.

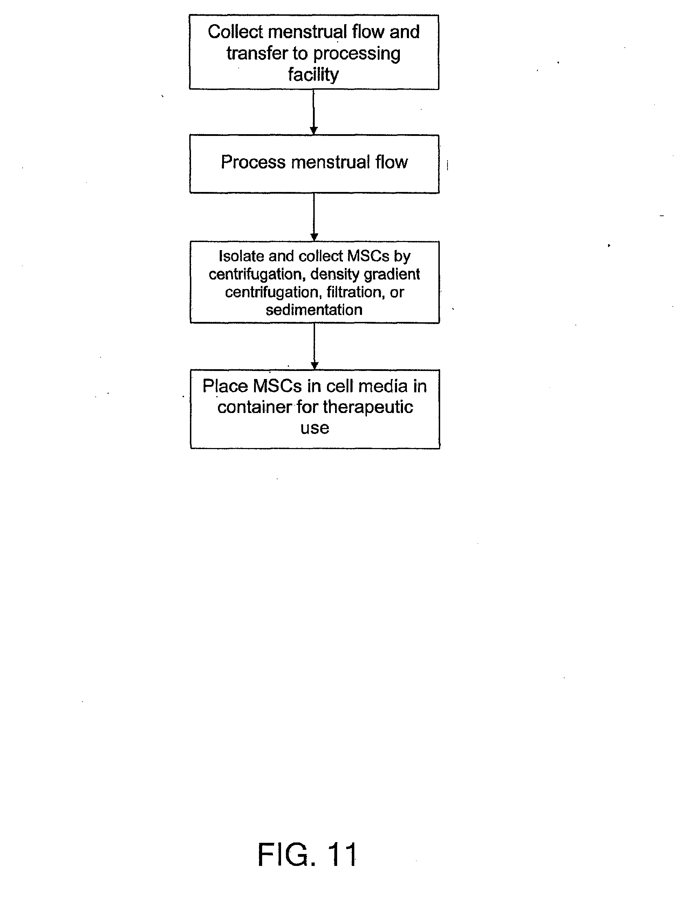

Referring now to FIG. 11, the present invention provides methods and processes for cryopreserving MSCs. According to FIG. 11, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be processed and combined with cell media to maintain cell viability in preparation for therapeutic use.

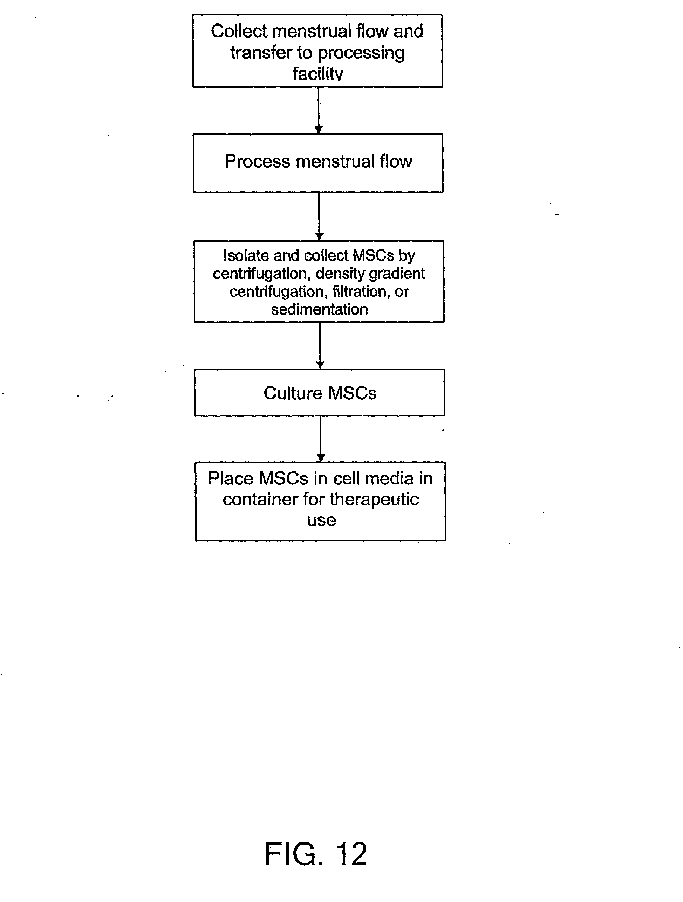

Referring now to FIG. 12, the present invention provides methods and processes for cryopreserving MSCs. According to FIG. 12, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. After culture, the MSCs may be processed and combined with cell media to maintain cell viability in preparation for therapeutic use.

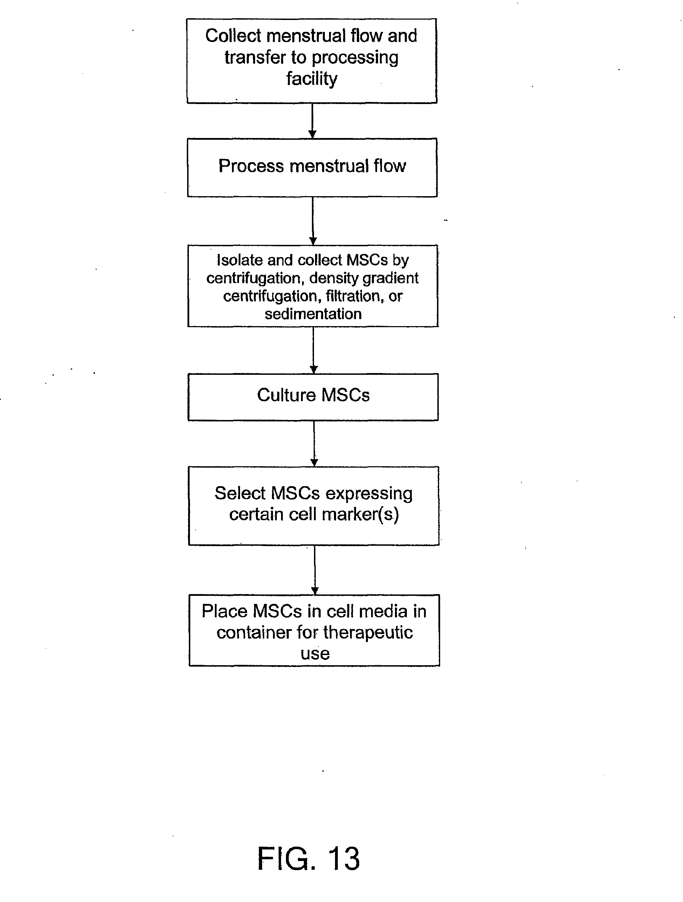

Referring now to FIG. 13, the present invention provides methods and processes for cryopreserving MSCs. According to FIG. 13, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may then may be selected for certain cell markers. The selected and unselected MSCs may then be separately combined with cell media to maintain cell viability in preparation for therapeutic use.

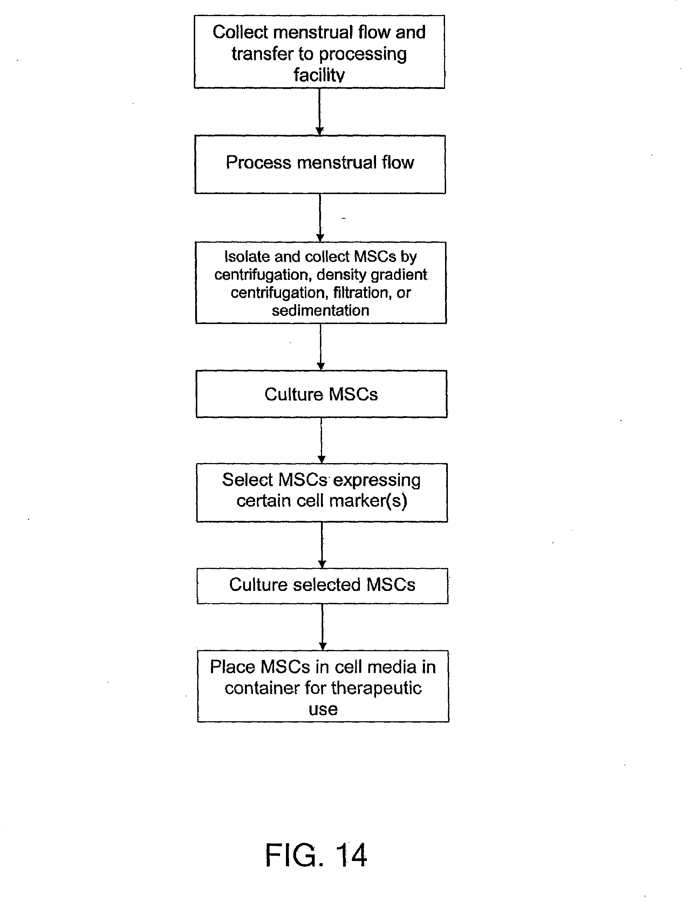

Referring now to FIG. 14, the present invention provides methods and processes for cryopreserving MSCs. According to FIG. 14, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may then may be selected for certain cell markers. The selected and unselected MSCs may then be separately cultured to expand the number of cells. The cultured MSCs may then be combined separately with cell media to maintain cell viability in preparation for therapeutic use.

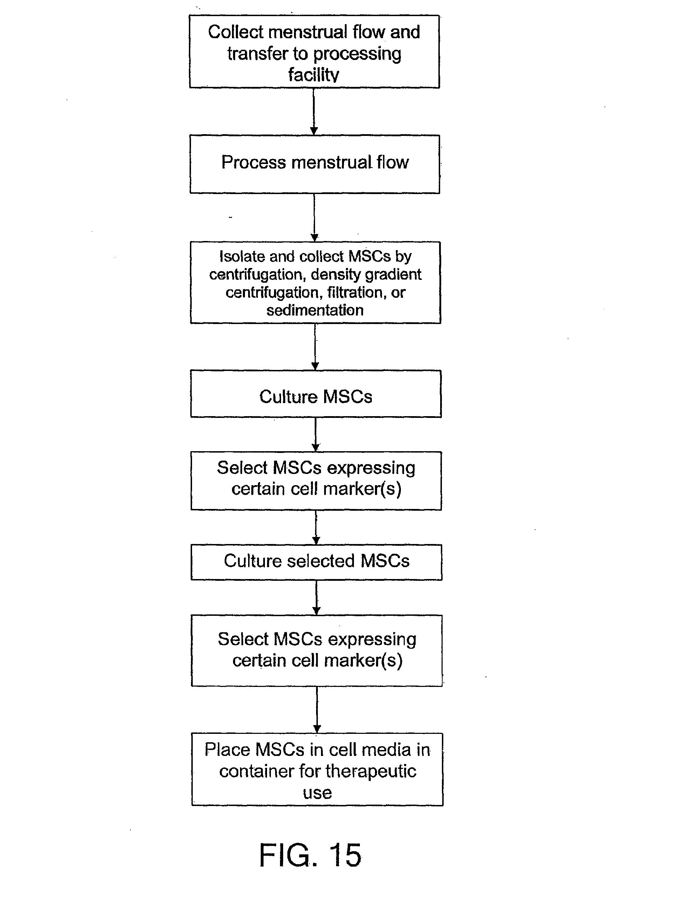

Referring now to FIG. 15, the present invention provides methods and processes for cryopreserving MSCs. According to FIG. 15, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be cultured to expand the number of MSCs. The cultured MSCs may then may be selected for certain cell markers. The selected and unselected MSCs may then be separately cultured to expand the number of cells. The separately cultured MSCs may then be selected again for certain cell surface markers. The selected and unselected MSCs may then be combined separately with cell media to maintain cell viability in preparation for therapeutic use.

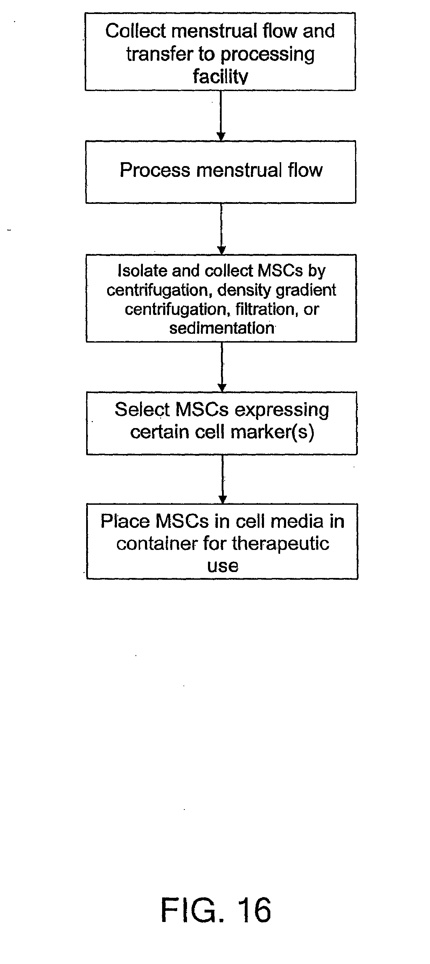

Referring now to FIG. 16, the present invention provides methods and processes for cryopreserving MSCs. According to FIG. 16, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be enriched or concentrated by selecting MSCs that express certain cell markers, such as, for example, any one or more of the cell markers associated with MSCs. Selected and even unselected MSCs may be then be separately combined with cell media to maintain cell viability in preparation for therapeutic use.

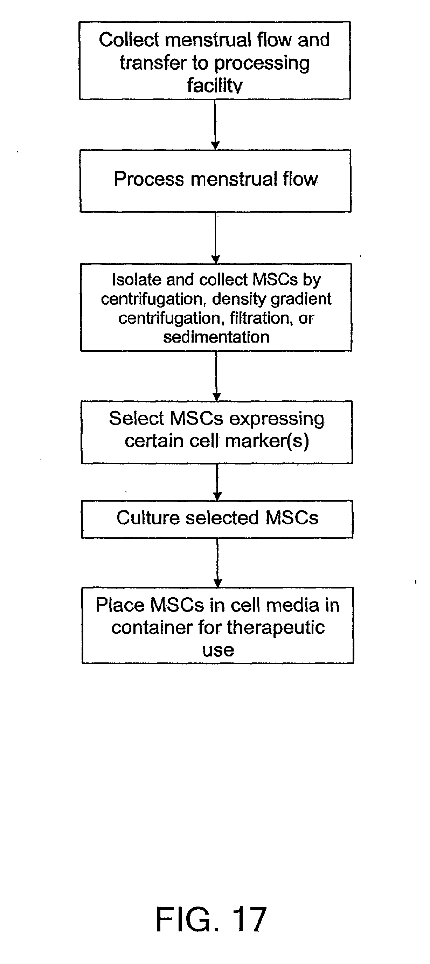

Referring now to FIG. 17, the present invention provides methods and processes for cryopreserving MSCs. According to FIG. 17, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be enriched or concentrated by selecting MSCs that express certain cell markers, such as, for example, any one or more of the cell markers associated with MSCs. Selected and even unselected MSCs may then be separately cultured to expand the number of MSCs. The cultured MSCs may then be combined with cell media to maintain cell viability in preparation for therapeutic use.

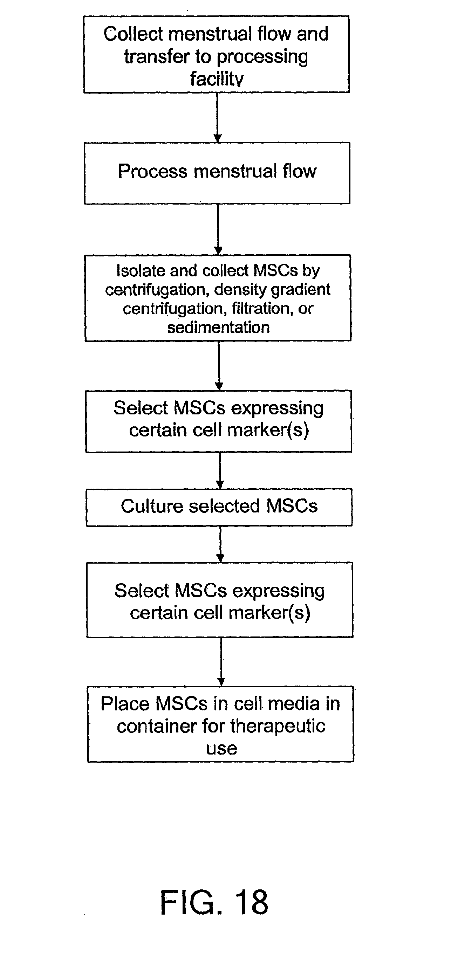

Referring now to FIG. 18, the present invention provides methods and processes for cryopreserving MSCs. According to FIG. 18, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be enriched or concentrated by selecting MSCs that express certain cell markers, such as, for example, any one or more of the cell markers associated with MSCs. Selected and even unselected MSCs may be then be separately cultured to expand the number of MSCs. The cultured MSCs may then be selected for specific cell markers. Selected and unselected MSCs may then be combined with cell media to maintain cell viability in preparation for therapeutic use.

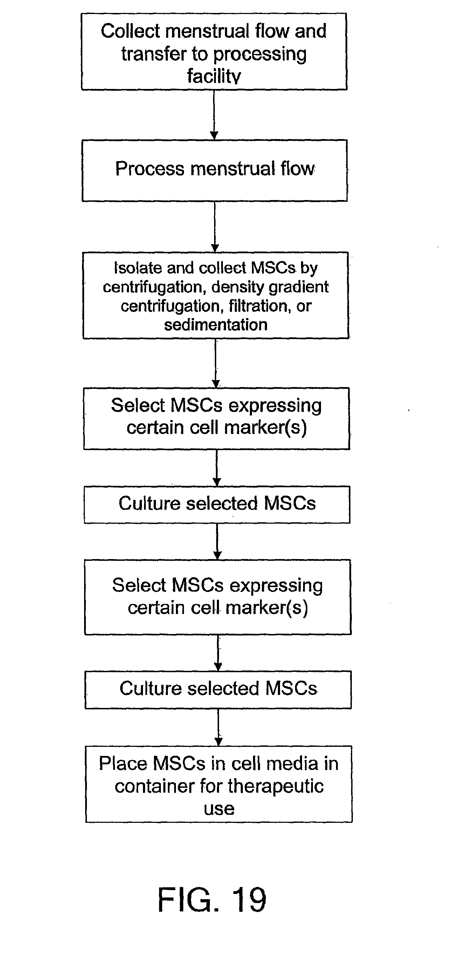

Referring now to FIG. 19, the present invention provides methods and processes for cryopreserving MSCs. According to FIG. 19, menstrual flow may be collected and transferred to a processing facility where the menstrual flow may then be processed to isolate and collect MSCs. The isolation and collection of MSCs from the menstrual flow may occur by way of centrifugation, density gradient centrifugation, filtration, or sedimentation methodologies used to maximize the number of MSCs collected. Once collected, MSCs may be enriched or concentrated by selecting MSCs that express certain cell markers, such as, for example, any one or more of the cell markers associated with MSCs. Selected and even unselected MSCs may be then be separately cultured to expand the number of MSCs. The cultured MSCs may then be selected for specific cell markers. Selected and unselected MSCs may then be cultured further to expand the number of MSCs. Cultured MSCs may then be combined with cell media to maintain cell viability in preparation for therapeutic use.

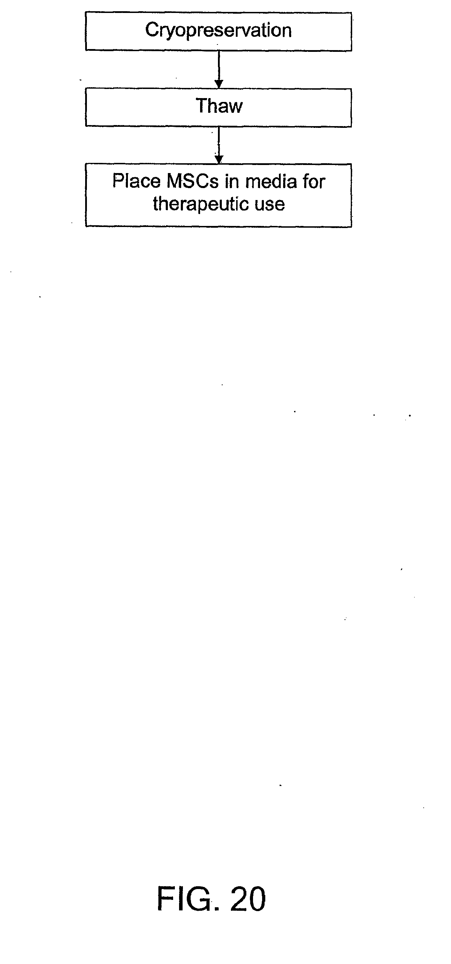

Further embodiments of the present invention are provided and described in FIGS. 20 through 28. The further embodiments include processing of MSCs after the MSCs were collected and cryopreserved according to any of the previously-described embodiments of the invention or any other cell collection and/or cryopreservation method or process.

Referring now to FIG. 20, the present invention provides methods and processes for preparing MSCs that were collected and cryopreserved according to any of the previously-described methods or processes or any other cell collection and/or cryopreservation method or process. According to FIG. 20, cryopreserved MSCs are thawed and then combined with cell media for therapeutic use.

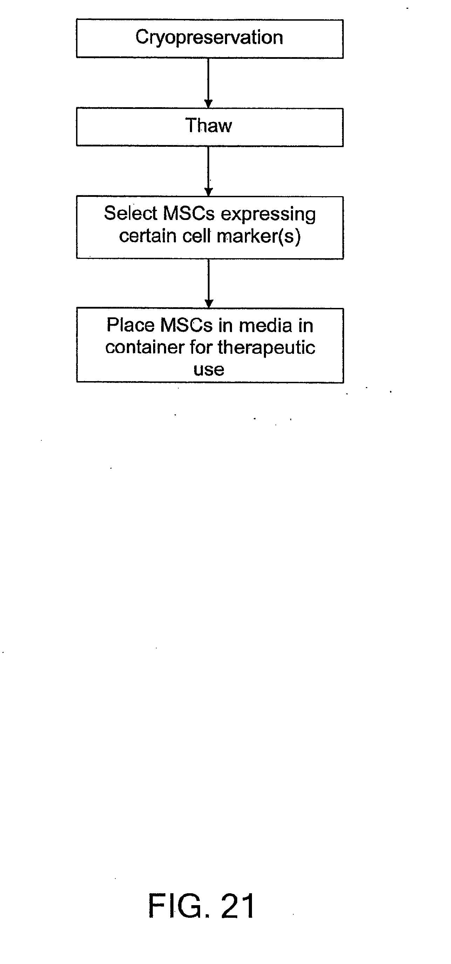

Referring now to FIG. 21, the present invention provides methods and processes for preparing MSCs that were collected and cryopreserved according to any of the previously-described methods or processes or processes or any other cell collection and/or cryopreservation method or process. According to FIG. 21, cryopreserved MSCs are thawed and then selected for certain MSC cell surface markers. Selected and unselected cells may then be combined with cell media for therapeutic use.

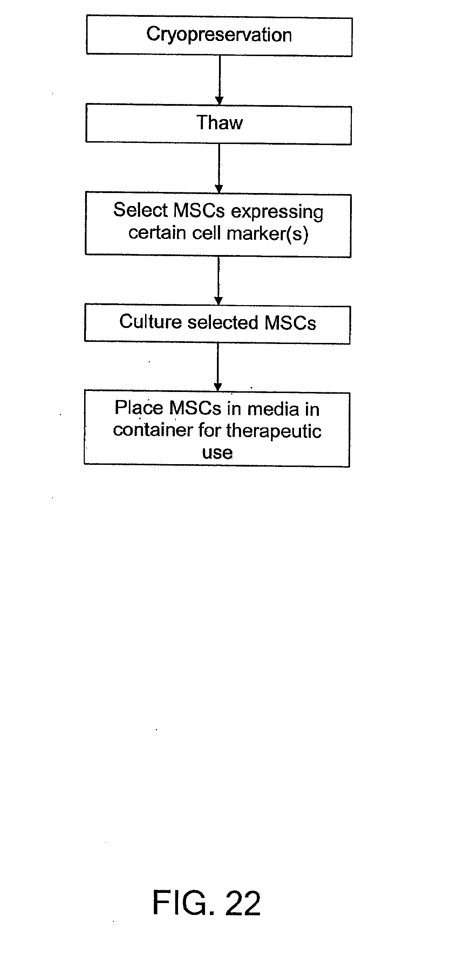

Referring now to FIG. 22, the present invention provides methods and processes for preparing MSCs that were collected and cryopreserved according to any of the previously-described methods or processes or processes or any other cell collection and/or cryopreservation method or process. According to FIG. 22, cryopreserved MSCs are thawed and then selected for certain MSC cell surface markers. Selected and unselected cells may then be cultured to expand the number of MSCs. Cultured MSCs may then be combined with cell media for therapeutic use.

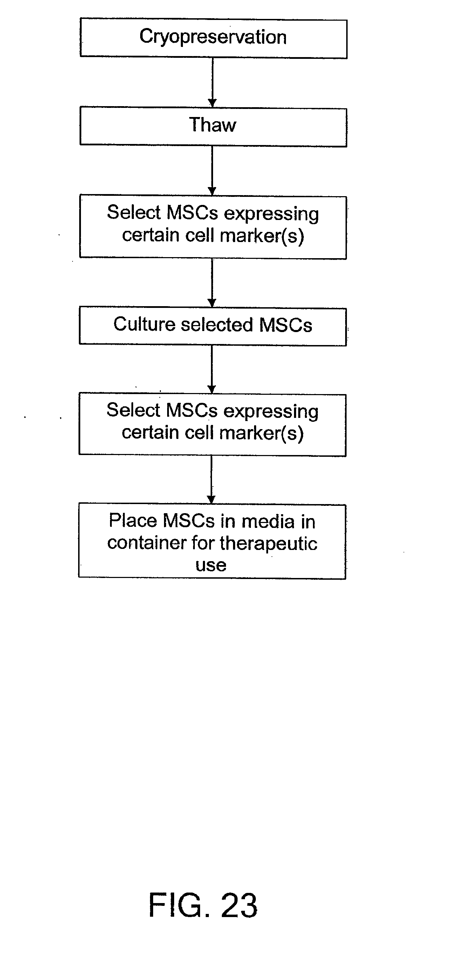

Referring now to FIG. 23, the present invention provides methods and processes for preparing MSCs that were collected and cryopreserved according to any of the previously-described methods or processes or processes or any other cell collection and/or cryopreservation method or process. According to FIG. 23, cryopreserved MSCs are thawed and then selected for certain MSC cell surface markers. Selected and unselected cells may then be cultured to expand the number of MSCs. Cultured MSCs may then be selected for certain MSC cell markers. Selected and unselected cells may then be combined with cell media for therapeutic use.

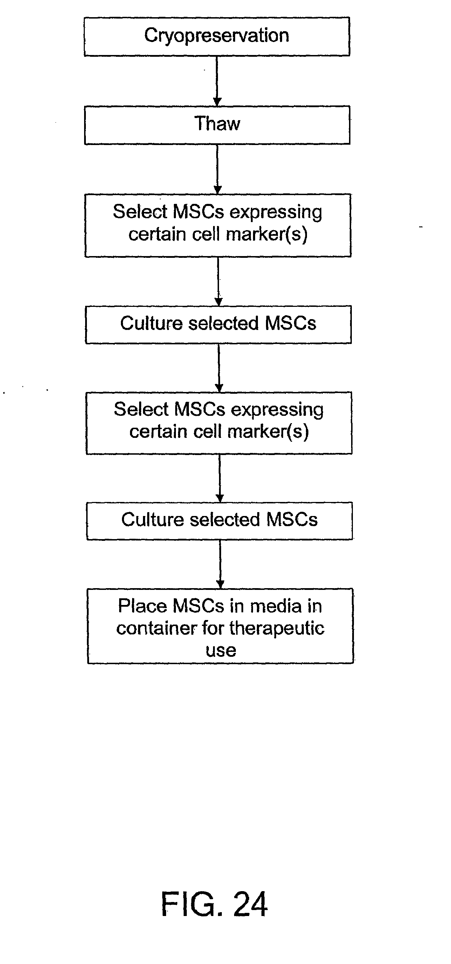

Referring now to FIG. 24, the present invention provides methods and processes for preparing MSCs that were collected and cryopreserved according to any of the previously-described methods or processes or processes or any other cell collection and/or cryopreservation method or process. According to FIG. 24, cryopreserved MSCs may be thawed and then selected for certain MSC cell surface markers. Selected and unselected cells may then be cultured to expand the number of MSCs. Cultured MSCs may then be selected for certain MSC cell markers. Selected and unselected cells may then be cultured to expand the number of MSCs. The cultured MSCs may then be combined with cell media for therapeutic use.

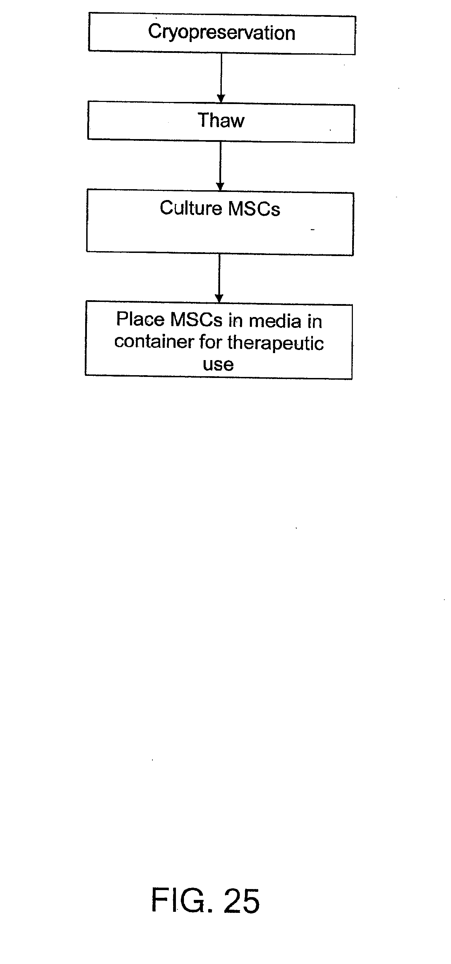

Referring now to FIG. 25, the present invention provided methods and processes for preparing MSCs that were collected and cryopreserved according to any of the previously-described methods or processes or processes or any other cell collection and/or cryopreservation method or process. According to FIG. 25, cryopreserved MSCs may be thawed and then cultured to expand the number of MSCs. Cultured MSCs may then be combined with cell media for therapeutic use.

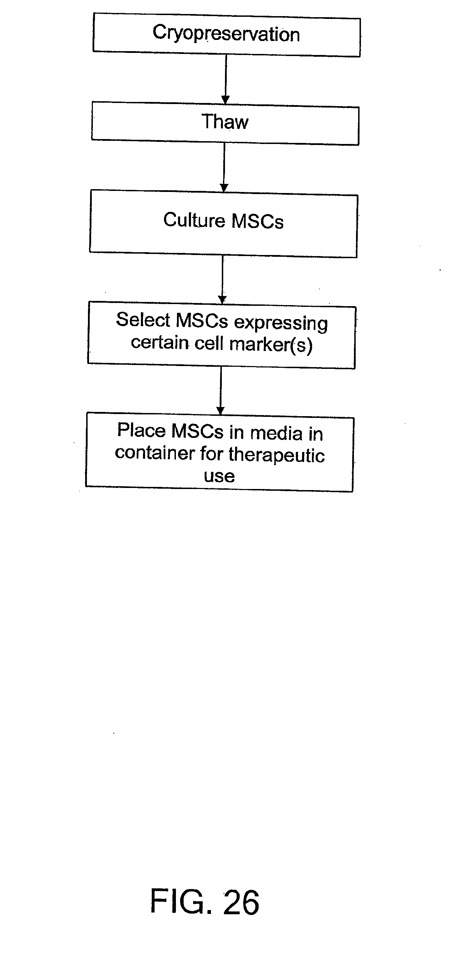

Referring now to FIG. 26, the present invention provided methods and processes for preparing MSCs that were collected and cryopreserved according to any of the previously-described methods or processes or processes or any other cell collection and/or cryopreservation method or process. According to FIG. 26, cryopreserved MSCs may be thawed and then cultured to expand the number of MSCs. Cultured MSCs may then be selected for certain cell markers. Selected and unselected MSCs may then be separately combined with cell media for therapeutic use.

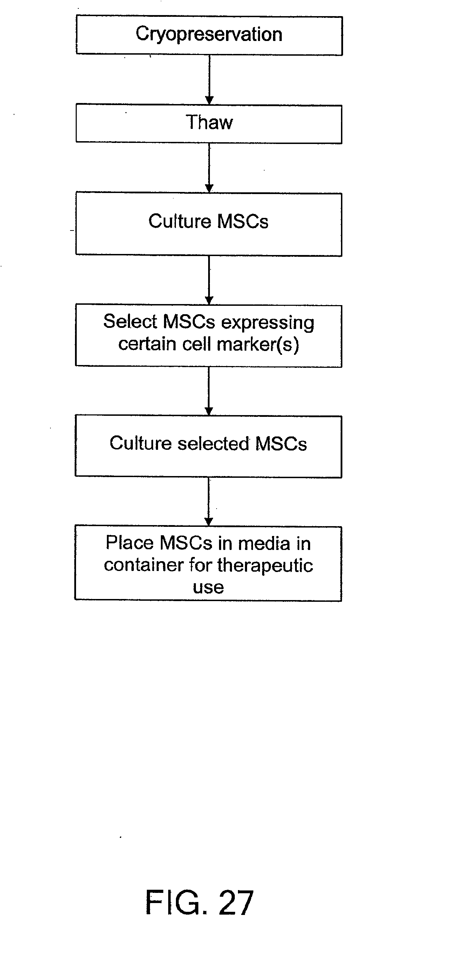

Referring now to FIG. 27, the present invention provided methods and processes for preparing MSCs that were collected and cryopreserved according to any of the previously-described methods or processes or processes or any other cell collection and/or cryopreservation method or process. According to FIG. 27, cryopreserved MSCs may be thawed and then cultured to expand the number of MSCs. Cultured MSCs may then be selected for certain cell markers. Selected and unselected MSCs may then be separately cultured to expand the number of MSCs. Cultured MSCs may then be combined with cell media for therapeutic use.

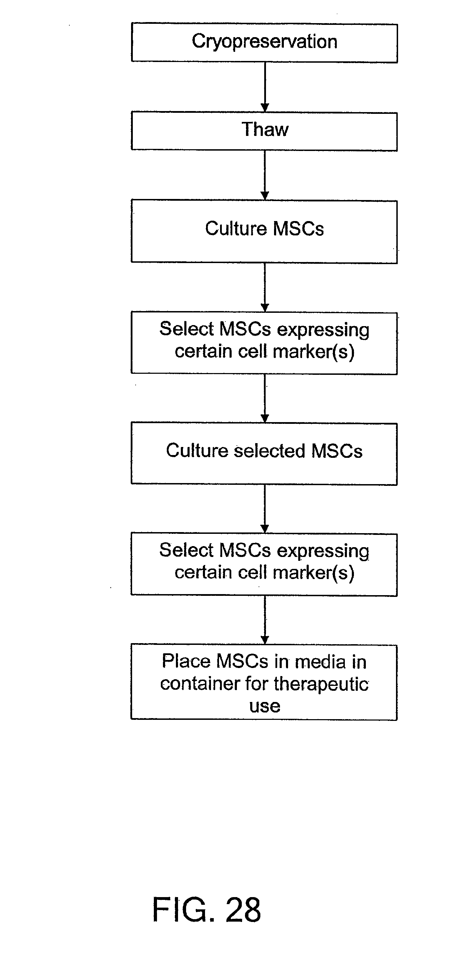

Referring now to FIG. 28, the present invention provided methods and processes for preparing MSCs that were collected and cryopreserved according to any of the previously-described methods or processes or processes or any other cell collection and/or cryopreservation method or process. According to FIG. 28, cryopreserved MSCs may be thawed and then cultured to expand the number of MSCs. Cultured MSCs may then be selected for certain cell markers. Selected and unselected MSCs may then be separately cultured to expand the number of MSCs. Cultured MSCs may then be selected for certain cell surface markers. Selected and unselected MSCs may then be combined with cell media for therapeutic use.

The aforementioned embodiments of the present invention as referenced in FIGS. 1 through 28 are described in further detail as provided and described hereinafter. According to the present invention, compositions comprising menstrual stern cells and other agents, such as, for example, cryopreservation agents, cell media, or cosmeceutical agents are also provided and described hereinafter.

**Collecting and Transporting Menstrual Stem Cells**

According to the methods and processes of the present invention, menstrual stem cells are obtained by first collecting menstrual flow, which includes menstrual stem cells, during menses using a collection device provided with a collection kit. Menstrual flow may be collected during any time of menstruation including, but not limited to, heavy, medium, or light days of a menstrual period. Optionally, a sample of peripheral blood may be collected from a donor for comparative infectious disease analysis.

A collection kit may be provided to a donor for the purposes of collecting at least one sample of menstrual flow for processing out menstrual stem cells. For example, a menstrual stern cell collection kit may be provided to collect, treat, and package at least one sample of menstrual flow for shipment. The collection kit may comprise a transport container, such as, a box, bag, or other container suitable for shipping at least one sample of menstrual flow, optionally, at a cold temperature (for example, Nanocool, Styrofoam or insulated container); a collection device such as, for example, a Mooncup, a Divacup, an Instead cup, or any other sanitary collection device suitable for collecting at least one sample of menstrual flow; at least one sterile collection container of suitable size such as, for example, a 50 ml tube or other suitable sized tube; collection media for suspending at least one menstrual flow sample; at least one plastic zipped bag; at least one absorbent towel; at least one biohazard bag; sterile 4 x 4 gauze; parafilm for wrapping the at least one collection container; and disinfecting cleansing towelettes or wipes. Prior to collecting at least one menstrual flow sample comprising menstrual stem cells, a donor's identity may be assigned an accession number that may be used through the practice of the invention to identify any sample collected by the donor.

The collection media for the collection kit may comprise a mixture of DPBS and Heparin. A suitable comparable tissue culture media with necessary pH may be used in place of DPBS. For example, Heparin may be provided at a concentration of about 1000 units per ml. In an embodiment, the collection media may comprise about 10 ml DPBS having 200 units of Heparin at a concentration of about 1000 units per ml. The media for the collection kit may be prepared by adding about 0.20 ml of Heparin at a concentration of 1000 units per ml to about 10 ml of DPBS. This volume may be placed in the sterile collection container before the sample is collected. In another embodiment, the collection media may comprise Dulbecco's Phosphate Buffer Saline (DPBS) (Mediatech or other manufacturer of a suitable replacement media) that contains no calcium, magnesium, or phenol red; antibiotics that may include Penicillin (about 100 units per milliliter), Streptomyocin (about 100 micrograms per milliliter), Amphotericin B, and/or other suitable antibiotics; and preservation-free Heparin in a range of about 10 units to about 20 units per ml of the DPBS media or suitable anticoagulant, such as, for example, ACD, CPD, CPDA, or CPDA-1. The collection media may be prepared using aseptic techniques.

The vaginal area may be cleansed with a disinfectant in preparation for collecting at least one menstrual flow sample comprising menstrual stem cells. In an embodiment, a disinfectant may be provided on a towelette. A donor then may place the collection device, which may have been previously cleaned with soap and water, within the vagina according to instructions for use of the collection device. The collection device must be placed within the vagina during a menstrual period of a donor in order to collect at least one sample of menstrual flow. In an embodiment, the collection device may be placed within the vagina during a heavy day of menstruation. In an alternative embodiment, the collection device may be placed within the vagina during a medium or light day of menstruation.

The collection device should be maintained within the vagina for an amount of time to collect at least one sample of menstrual flow. For example, the collection device may be maintained within the vagina for about 3 hours or less. Additional time to collect a sample may be necessary. After being maintained within the vagina for about 3 hours, the collection device containing a menstrual flow sample may then be removed. A menstrual flow sample may comprise a volume of about 0.1 ml to about 5 ml of menstrual blood, tissue, fluid, and menstrual stem cells. The menstrual flow sample may then be transferred to the collection media in a sterile collection tube. The transfer of the menstrual flow sample to the sterile container may occur by pouring the menstrual flow sample from the collection device into the media in a container in preparation for shipment of the menstrual flow sample to a processing facility. The container should be capped or otherwise sealed to prevent leakage of solution of the menstrual flow sample and the collection media. The seal should also prevent entry of air into the interior of the collection container. In an embodiment, the container with corresponding lid tightly closed may be wrapped with parafilm and packaged for transportation.

More than one menstrual flow sample may be collected, transferred to a container, and prepared for shipment to a processing facility. If more than one menstrual flow sample is collected, each collected sample in collection media in a collection tube may be maintained in a refrigerator until the last sample is collected. Once all menstrual flow samples are collected, the samples may then be packaged for shipment.

In an embodiment, menstrual stem cells may be procured by Papanicolaou test or any biopsy done in the endometrial or cervical area.

The at least one sample of menstrual flow comprising menstrual stem cells may be maintained at a cold temperature after procurement through shipment and processing at the processing facility. Any sealed container holding the solution of menstrual flow and media may be packaged for shipment to maintain each sample at a temperature in the range of about 1°C to about 25°C during shipment to a processing facility. In an embodiment, each sample may be maintained in a temperature range of about 1°C to about 15°C during shipment to a processing facility. In another embodiment, collected menstrual flow sample may be maintained at room or ambient air temperature after procurement and throughout shipment.

Each collection container containing a menstrual flow sample comprising menstrual stem cells and collection media may be packaged for transportation by placing the collection container in a large plastic zip bag that is sealed with the zip function of the bag. The large plastic bag with sterilized container enclosed may be placed in a second large zip bag and also sealed. Two absorbent towels may be placed in the bottom of the transport container. If a Nanocool device is being used, additional ice is not necessary. Foam bricks or other devices may be used to cool the transport container carrying the menstrual flow sample. The bag full of wet ice may be placed on top of the absorbent towels. The sterilized container in the sealed bags may be placed underneath the ice bag. The remaining absorbent towels may be placed on top of the bag of ice. The transport container is closed, secured, and sealed.

Other suitable containers may be used for transportation of the container as long as the container maintains the menstrual flow sample comprising menstrual stem cells at a temperature between about 1°C to about 25°C during shipment. For example, other suitable containers include, but are not limited to, cooled and insulated shipping containers sold by Therapak Corporation, insulated containers of any type, or a Styrofoam box designed for shipment.

A carrier may be contacted within at least two hours of shipment to pick up the transport container containing at least one menstrual flow sample in collection media. The carrier may be AirNet or other preferred courier suitable for transporting biological materials or other shipment companies such as FedEx or UPS. The transport container should arrive at the processing facility within about 5 hours to about 125 hours after collection. In an embodiment, the transport container may arrive at a processing facility between about 24 hours to about 72 hours after collection. In another embodiment, the transport container may arrive at a processing facility between about 24 hours to about 48 hours. The transport container should not be stored in a hot environment.

The processing facility receives the transport container and catalogs the identification information for each donor sample for use throughout processing the menstrual flow samples comprising menstrual stem cells. Appropriate barrier and personal protection measures may be used throughout handling any sample of menstrual flow comprising menstrual stem cells at the processing facility. Once the transport container is received at the processing facility, each container with menstrual flow sample may be removed from the transport container, placed on ice in an ice pan (or in a refrigerator), and transferred into a cleanroom in a biological safety cabinet (BSC) for isolating menstrual stem cells from menstrual flow, collecting menstrual stem cells, and later processing for use and/or cryopreservation.

**Menstrual Flow Processing at Processing Facility**

The menstrual flow sample is received by a processing facility and logged into the processing facility system. Once the menstrual flow sample is received at the processing facility it may be maintained at a temperature of about 1 °C to about 25°C throughout the processing. Each menstrual flow sample may be processed in a BSC and centrifuge using the aseptic technique. Wipe gloves with 70% IPA frequently when working in the BSC. Aseptically place all other materials needed for processing in the BSC including, but not limited to, red top tubes, needles, syringes, rack for red top tubes, BacT bottles, alcohol pads, 50 ml conicals, rack for 50 ml conicals, and pipettor prior to processing the menstrual flow sample. All sterile supplies are set up after the BSC has been disinfected. All sterile supplies used for the process may be placed in the pan or a sterile drape. The sterile supplies may include needles, syringes, and other materials placed in the BSC including red top tubes, rack for red top tubes, blood culture bottles, alcohol pads, 50 ml conical collection tubes, rack for 50 ml conical collection tubes, pipettes, and ice pan. All media and reagents are preferably placed on ice and are stored between about 1°C to about 15°C, but may also be about 2°C to about 8°C, based on manufacturer's instructions.

A buffered saline media (DPBS) or suitable replacement may be used throughout the menstrual stem cell isolation process. DPBS media comprises about 100 milliliters of DPBS, with about 5 ml to about 10 ml of Heparin (Heparin Sodium 1,000 USP Units/ml - American Pharmaceutical Partners).

On the first day that the menstrual flow sample is received at the processing facility, each menstrual flow specimen comprising menstrual stem cells may be subjected to Stage 1 processing. First, the exterior of the collection container holding the menstrual flow sample may be disinfected prior to centrifugation. A menstrual flow sample in a collection container may be subjected to centrifugation, density gradient centrifugation, filtration, and/or starch sedimentation.

**Centrifugation**

In an embodiment, the centrifugation of a menstrual flow sample may occur at about 2000 rpm for about seven minutes at about at 4°C. After centrifugation, each specimen of menstrual flow sample may then be removed from the centrifuge. The exterior of the collection container may be disinfected prior to transfer of the collection tube to a BSC to aspirate the supernatant. The pellet may be resuspended in about 10 ml of DPBS, or comparable replacement, and then subjected to Stage 2 processing. The pellet may also be processed according to an optional decontamination step as described herein.

**Density gradient centrifugation**

In another embodiment, the centrifugation of a menstrual flow sample may occur using a density gradient. For example, a density gradient may be Lymphocyte Separation Medium (Density 1.077 - 1.083 g/ml at about 20°C - Cellgro) or other suitable gradient which may have a higher or lower density. A conical collection tube containing the menstrual flow sample, collection media, and a density gradient is subject to centrifugation. For example, the conical collection tube may be centrifuged at about 14,000 rpm for about 30 minutes between about 15°C to about 30°C, preferably at about 20°C, and without a brake applied to slow the centrifuge.

As a result of density gradient centrifugation, a buffy coat layer forms at the interface between the supernatant and the density gradient. The buffy coat layer contains the desired cellular population comprising menstrual stem cells obtained from a menstrual flow sample. The supernatant above the buffy coat layer is aspirated to about 5 ml above the buffy coat layer and discarded. The buffy coat layer is removed by gently swirling the buffy coat layer and reaming the sides of the conical tube at the buffy coat layer with a 10 ml pipette. The smallest volume of density gradient as possible should be collected with the buffy coat layer. The remaining density gradient and pellet containing red blood cells may be discarded. The density gradient separation may be performed with reagents and cells at room temperature. The separation will not provide an optimal cell recovery if the cells are chilled.

The collected buffy coat layer may be placed in a 50 ml conical collection tube and the volume is brought up to about 30 ml with a wash solution. The cellular suspension may then be subject to centrifugation at about 2000 rpm for about seven minutes between about 15°C to about 30°C.

After centrifugation, the specimens may then be removed from the centrifuge and the exterior of the collection container may be disinfected prior to transfer of the collection tube to a BSC to aspirate the supernatant. The pellet may be resuspended in about 10 ml of DPBS and then subject to Stage 2 processing. The pellet may also be processed according to an optional decontamination step as described herein.

**Filtration**

In yet a further embodiment, a menstrual flow sample may be subjected to filtration in a sterile filter. The sterile filter may be about 40 microns, about 70 microns, or about 100 microns. The filtration may comprise subjecting the menstrual flow sample comprising menstrual stem cells to centrifugation at about 2000 rpm for about seven minutes between about 1°C to about 30°C.

After centrifugation, the specimens may then be removed from the centrifuge and the exterior of the collection container may be disinfected prior to transfer of the collection tube to a BSC to aspirate the supernatant. The pellet may be resuspended in about 10 ml of DPBS and then subject to Stage 2 processing. The pellet may also be processed according to an optional decontamination step described herein.

**Starch Sedimentation**

In an even further embodiment, the menstrual flow sample may be processed by starch sedimentation. In an embodiment, hydroxyethyl starch may be used at a ratio of one to five, i.e., about one milliliter of starch to about 5 ml of menstrual flow sample. Each menstrual flow sample may be mixed according to the aforementioned ratios. The mixture may be subjected to centrifugation to assist with the sedimentation at about 50 g for about 6 minutes. The mixture may also be allowed to sediment with or without the aforementioned centrifugation step for about thirty minutes or up to about two hours. After sedimentation, the buffy coat comprising menstrual stem cells are removed and subjected to centrifugation to concentrate the menstrual stem cells. Centrifugation comprises subjecting the menstrual stem cells suspended in DPBS to centrifugation at about 2000 rpm for about seven minutes between about 1°C to about 30°C.

After centrifugation, the specimens may then be removed from the centrifuge and the exterior of the collection container disinfected prior to transfer of the collection tube to a BSC to aspirate the supernatant. The pellet may be resuspended in about 10 ml of DPBS and then subject to Stage 2 processing. The pellet may also be processed according to an optional decontamination step described herein.

**Decontamination**

A sample of collected menstrual stem cells as collected according to any one of the aforementioned steps of centrifugation, density gradient centrifugation, filtration, or starch sedimentation may be subjected to an optional decontamination process. The excess fluid about a pellet may be removed after first calculating the total volume of the sample. The pellet may be resuspended in about 5 ml of decontamination media.

Decontamination media may be used to decontaminate menstrual flow samples. Decontamination media comprises at least one antibiotic in a suitable media. By way of a nonlimiting example, the antibiotics may comprise at least one of Vancomycin, Claforan, Amikacin, Gentamycin, and Amphotericin B. The suitable media may comprise Hanks Balanced Salt Solution (HBSS) or other media. The antibiotics may require reconstitution if provided in desiccated form.

In an embodiment, the decontamination media may comprise the following concentrations of antibiotics 50 ug/ml of Vancomycin, 250 ug/ml Claforan, 100 ug/ml Amikacin, 120 ug/ml Gentamycin, and 2.7 ug/ml Amphotericin B. Other concentration levels of antibiotics may be suitable. For example, the decontamination media may be made by adding about 10 ml of HBSS to about a 1 g vial of Vancomycin to obtain a concentration of about 100 mg/ml. About 1 ml of 100 mg/ml concentration of Vancomycin is added to about 199 ml of HBSS to obtain a concentration of about 500 ug/ml. About 1 ml of about 500 ug/ml concentration of Vancomycin will be added to a final vial for preparing the decontamination media. About 2 ml of HBSS is added to about a 500 mg/vial of Claforan to obtain a concentration of about 250 mg/ml. About 1ml of the about 250 mg/ml Claforan solution is added to about 99 ml of HBSS to obtain a concentration of Claforan of about 2500 ug/ml. About 1ml of the about 2500 ug/ml concentration of Claforan will be added to the final vial for the decontamination media. About 10 ml of HBSS is added to about a 1 g vial of Amikacin to obtain a concentration of about 100mg/ml. Add about 1ml of the about 100mg/vial concentration of Amikacin to about 99 ml of HBSS to get about 1000 ug/ml. About 1ml of Amikacin will added to the final vial to prepare the decontamination media. About 10 ml of HBSS is added to about a 1 g vial of Gentamycin to obtain a concentration of about 100 mg/ml. About 1ml of the about 100 mg/ml concentration of Gentamycin is added to about 99 ml of HBSS to obtain about a 1000 ug/ml concentration. About 1.2 ml of about 1000 ug/ml solution of Gentamycin will be added to the final vial to prepare the decontamination media. About 2 ml of Amphotericin B is added to about 8 ml of sterile water to prepare a concentration of about 50 ug per ml of Amphotericin B. About 0.540 ml (27ug) of Amphotericin B to prepare a final concentration of Amphotericin B.

With the solutions of antibiotics, decontamination media may be prepared in a 50 ml sterile tube by adding about 1 ml of 500 ug/ml Vancomycin, about 1 ml of 2500 ug/ml of Claforan, about 1 ml of 1000 ug/ml of Amikacin, about 1.2 ml of 1000 ug/ml of Gentamycin, and about 0.540 ml (27ug) of Amphotericin B to make up about 4.74 ml of media. About 0.200 ml heparin 1000 units per ml is added the antibiotic solution and about 5.06 ml of HBSS is added to the antibiotic solution to create a final volume of 10 ml of decontamination media. Media may be stored at about 2°C to about 8°C until use.

The pellet may be resuspended in about 5 ml of decontamination media. Cells may require filtration if clots or other macromolecules are present. If the resuspended cells require filtration due to clots or for any other reason, the sample may be filtered using a 100 micron filter. For filtration, the suspension may be filtered using a 100 micron filter, or smaller depending upon clot size in the suspension, and a sterile 50 ml conical tube. Once the suspension passes through the filter, the filter may be washed using additional antibiotic media.

Filtered or unfiltered cells in the decontamination media may be incubated for decontamination. In an embodiment, the filtered or unfiltered cells in decontamination media may be incubated at about 2°C to about 8°C for about 20 hours to about 30 hours in a refrigerator in a clean room. During incubation, the cells may be placed on a rotator or inverted for the first 5 hours of incubation at about once per hour. At the end of the decontamination process, the cellular suspension comprising menstrual stem cells is then subjected to Stage 2 processing.

The cell suspension comprising menstrual stem cells, with or without being subject to the decontamination process, may be processed according to Stage 2 processing. Stage 2 processing comprises subjecting the cellular suspension comprising menstrual stem cells in either DPBS or decontamination media to centrifugation. In an embodiment, the centrifugation of cellular suspension may occur at about 2000 rpm for about seven minutes at about 4°C. The exterior of a tube holding menstrual stem cells may be disinfected after centrifugation and prior to transfer to the BSC. Once in the BSC, the supernatant may be removed leaving a pellet of cells at the bottom of the tube. The cells may be resuspended with about 10 ml of DPBS, mixed, and centrifuged at about 2000 rpm for about seven minutes at about 4°C. After centrifugation, the container may be decontaminated prior to transfer to the BSC.

Bacteriological analysis of the menstrual stem cells may occur during this point in processing. In the BSC, and using sterile technique, about 5 ml of supernatant above the pellet comprising menstrual stem cells may be removed using a sterile syringe. About 4 ml of the supernatant may be placed in an anaerobic blood culture bottle and about 1 ml of supernatant may be placed in an aerobic blood culture bottle. The bottles may be configured for use in a BacT/Alert system. The bottles may then be incubated using the BacT/Alert system according to manufacturer's instructions. The BacT/ALERT blood culture bottle and system are provided as an example of a suitable testing system for the practice of the invention. Other suitable automated or manual blood culture specimen bottles and systems may be used as long as it is in compliance with 21 C.F.R. Section 610.12 or validated for performance similar to this CFR Section.

The remaining supernatant may be removed from above the pellet. The pellet comprising cells may be resuspended in DPBS up to about 5.1 ml and mixed. About 100 µl of the cells may be aliquoted to a tube for post-processing testing to obtain cell count and viability. Cell count may be performed using an automated or manual hemocytometer. Cell viability may be performed using trypan blue or other method.

The remaining cells suspended in DPBS may be prepared for cryopreservation, cell culture, cell selection, or therapeutic or cosmeceutical use.

**Cryopreservation**

For cryopreservation, the remaining cells suspended in DPBS may be placed on ice for at least about 15 minutes in preparation for cryopreservation. The preparation may comprise adding cryopreservation media to the menstrual stem cells and then subjecting the mixture to several temperature reduction steps to reduce the temperature of the menstrual stem cells to a final temperature of about -90°C utilizing a controlled rate freezer or other suitable freezer system (dump-freeze monitored or a freeze container (Nalgene)). Suitable control rate freezers include, but are not limited to, Cryomed Thermo Forma Controlled Rate Freezer 7454 (Thermo Electron, Corp.), Planar Controlled Rate Freezer Kryo 10/16 (TS Scientific), Gordinier, Bio-Cool - FTS Systems, and Asymptote EF600, BIOSTOR CBS 2100 series.

Cryopreservation media may be prepared comprising media and DMSO. About 3 ml of DPBS may be added to a container, such as, for example, a 50 ml conical tube. About 1 ml of Human Serum Albumin (HSA) may be added to the about 3 ml of DPBS and then chilled for about ten minutes on ice. About 1 ml of the chilled 99% DMSO is added to the HSA and DPBS to prepare the final cryopreservation media. Cryopreservation media and the cell sample may then be placed on ice for about 15 minutes before the cryopreservation media is added to the cell sample. Batch processing may be used for aliquoting cryopreservation media into a cell sample. For example, a single aliquot of about 100ul of cell suspension may be combined with about 3 ml of DPBS, 1 ml of HSA, and about 1 ml of 99% DMSO. About 2 aliquots of about 200 ul of cell suspension may be combined with about 6 ml of DPBS, 2 ml of HSA, and about 2 ml of 99% DMSO. About 5 aliquots of cell sample may be combined with about 15 ml of DPBS, about 5 ml of HSA, and about 5 ml of 99% DMSO. About 10 aliquots of cell sample may be combined with about 30 ml of DPBS, about 10 ml of HSA, and about 10 ml of 99% DMSO.

In an alternative embodiment, other cryopreservation media may be used. For example, cryopreservation media with cryopreservation agents may be used to maintain a high cell viability outcome post-thaw, such as, for example, CryoStor CS10 or CS5 (Biolife), embryonic cryopreservation media supplemented with propanediol and sucrose (Vitrolife), or SAGE media (Cooper Surgical). Glycerol may be used with other cryopreservation agents, such as, DMSO, or may be used alone at a concentration of about 10% in a media with suitable protein.

Cryopreservation media may be added to the cell sample. Cryopreservation media may be added to menstrual stem cells suspended in DPBS drop by drop until the volume of total mixture of suspended menstrual stem cells and cryopreservation media is brought to the final desired volume of cryopreservation cell mixture. The final cryopreservation cell media may be transferred into 2 x 5ml bar-coded cryoquats with the QC sample stored in the cap of the 5 ml vial. Use a pipettor to remove about 200 µl aliquots of the sample and aliquot into the QC caps. After the caps have been filled, add the remaining 4.8 ml of specimen into the 5 ml vial. Samples should be sent for infectious disease and other analysis. The cryovials should be placed in the CRYOMED Freezer and subject to temperature reduction by controlled rate to a temperature at or below about -85°C. The cryopreserved specimens should be transferred to a bunker for storage in the vapor of Liquid Nitrogen at -150°C or less. Any sample that tests positive for an infectious disease should be quarantined. Any sample that tests negative for infectious disease may be transferred to a different permanent storage.

The following temperature reduction steps may be programmed in the controlled rate freezer, first reducing the mixture of cryopreservation agent and menstrual stem cells. The menstrual stem cells combined with cryopreservation agent may be subjected to controlled rate temperature reductions in preparation for final storage in a freezer. The controlled rate reductions may be designed to maintain cell viability. A Cryo-Med Freezer (Thermo Electron Corp.), liquid nitrogen cylinder, and portable Cryo-Med Freezer may be used for controlled rate reductions in preparation for final storage in freezer. The cells may be subject to controlled rate reductions in cryovials or cryobags to reach a temperature of about -90°C.

For a sample of menstrual stem cells collected in a cryobag, the cells may be subject to the following controlled rate reduction profile: wait at about 4°C, 1.0°C per minute to -6.0°C (sample), 25.0°C per minute to -50.0°C (chamber), 10.0°C per minute to -14.0°C (chamber), 1.0°C per minute to -45.0°C (chamber), 10.0°C per minute to -90.0°C (chamber), and end (sample at or below -85.0°C).

For a sample of menstrual stem cells collected in a cryovial, the cells may be subject to the following controlled rate reduction profile: wait at 4.0°C, 1.0°C per minute to -3.0°C (chamber), 10.0°C per minute to -20.0°C (chamber), 1.0°C per minute to -40.0°C (chamber), 10.0°C per minute to -90.0°C (chamber), and end.

Once the mixture of cryopreservation agent and menstrual stem cells is at or below about -85°C, the cryopreservation vials are transferred to a cryogenic storage unit and stored in the vapor of liquid Nitrogen at a temperature at or below about -135°C or alternatively vials may be stored in the liquid phase of liquid nitrogen. For example, a suitable cryogenic storage unit includes, but is not limited to, LN2 Freezer MVE 1830 (Chart Industries).

**Flow Cytometry Analysis**

Any collected sample of menstrual stem cells may be tested for the total cell count and cell viability by Trypan blue via dye exclusion. Samples of menstrual stem cells may also be analyzed for the presence of cells expressing cell markers.

The total cell count and cell viability of pre-processing, post-processing, and any other sample of cells may be quantified by a hand count with a hemocytometer, a flow cytometer, or other means suitable for obtaining cell count, such as, for example, ViCell (Beckman Coulter) or software suitable to count cells displayed on a microscopic image.

Pre-processed cells may be analyzed by flow cytometry. 1X NH4CL lysing solution (StemKit) may be prepared, if red cells are present, by adding about 36 ml distilled water to a 50 ml tube and about 4 ml of 10X lysing solution. About 100ul of pre-processed sample per client may be added to two tubes to run the analysis. One tube is for CD34+/viability analysis and the second tube is for the isoclonic control. About 20 ul of CD45-FITC/CD34-PE may be added to the bottom of each tube. About 20 ul of 7-AAD Viability dye should be added into the tubes. The mixture may be vortexed and then incubated at about 15°C to about 30°C for at least 20 minutes protected from light. About I ml of 1x NH4CL lysing solution may then be added to each tube and vortexed. The mixture may be incubated at about 15°C to about 30°C for about 20 minutes. About 100 uL of Stem-Count Fluorospheres may be added to each tube and vortexed. The sample should then be run on a Flow Cytometer for analysis.

Post-processed cells may be analyzing by flow cytometry. 1X NH4CL lysing solution may be prepared from a StemKit by adding about 36 ml distilled water and 4 ml 10X lysing solution into a 50 ml tube. About 50 ul of post-processed sample per client may be added to two tubes to run the analysis. One tube is for CD34+/viability analysis and the second tube is for the isoclonic control. About 10 µL of 7-AAD Viability dye may be added to each tube. About 10µL of CD45-FITC/CD34-PE may be added to the first tube. About 10 µL of CD45-FITC/CTRL-PE may be added to the second tube. The mixture may be vortexed and then incubated at about 15°C to about 30°C for at least 20 minutes protected from light. About 1 ml of 1x NH4CL lysing solution may then be added to each tube and vortexed. The mixture may be incubated at about 15°C to about 30°C for about 20 minutes. About 100 uL of Stem-Count Fluorospheres may be added to each tube and vortexed. The sample should then be run on a Flow Cytometer for analysis.

Fresh samples of menstrual stem cells and thawed samples of menstrual stem cells that were previously cryopreserved may also be analyzed by flow cytometry to analyze cell surface markers, cell viability, and other cell characteristics. Fresh samples may also be analyzed according to the following protocol after cell lysis.

Cryopreserved menstrual stem cells must be thawed according to the thawing process described herein. In cases where a cell sample must be thawed, the cell may require flow cytometry analysis immediately after thaw or after cells are cultured to assess a certain cell passage. The cryopreserved samples may be agitated in a 37°C water bath without letting the cells completely thaw. The cells may be transferred into about 1 ml of chilled wash media and mixed by inversion. The sample may be centrifuged at about 2000 rpm for about seven minutes. The supernatant may be removed and the cells resuspended in about 100 ul of wash media (25% HSA, DNAse, Heparin and HBSS w/Ca+ and Mg+). The resuspended cells may then be centrifuged in a Blood Bank Serofuge for about 1 minute. The supernatant may be decanted and the cells resuspended in about 1.2 ml Sheath fluid and vortexed.

The cells in Sheath fluid may be analyzed for any number of cell surface markers. As an example, and not a limitation, about a 100 ul sample of cells in Sheath fluid may be added to each tube containing the following reagents in either 10ul or 20 ul volumes per reagent in each tube and then vortex the tube to mix the reagents and sample as described in Table A.

**Table A: Flow Cytometry Load Schematic**

| **TUBE** | **FITC** | **PE** | **ECD** | **PC5** |
|---|---|---|---|---|
| **1** | IgG | IgG | IgG | IgG |
| **2** | HLA-I | CD133 | HLA-II | 7AAD |
| **3** | CD9 | CD54 | CD45 | CD10 |
| **4** | CD59 | CD63 | CD34 | CD13 |
| **5** | CD49e | CD81 | None | CD49f |
| **6** | CD44 | CD117 | None | CD38 |
| **7** | CD29 | CD105 | CD41 | CD3 |
| **8** | CD19 | CD166 | None | CD90 |
| **9** | NANOG | SSEA3 | None | 7AAD |
| **10** | CD14 | SSEA4 | None | 7AAD |
| **11** | None | CD56 | None | 7AAD |

Incubate at room temperature (15-30°C) for 20 minutes. Protect from light. If running a fresh sample containing RBC's, add 500 µl of lyse solution and incubate at room temperature for another ten minutes and protect from light. If running a density gradient or a thawed sample, do not lyse. If sample was not lysed, wash after 20 minutes incubation with 1 ml of wash media. Centrifuge for 1 minute and then decant the supernatant. If sample was lysed, centrifuge sample for 1 minute and decant lyse. Add 1 ml of wash media, vortex, centrifuge again, and then decant again. Add 500 uL of Sheath fluid to each tube, vortex, and run on a FC500 Flow Cytometer.

Prior to cell marker analysis, a total cell count may be performed on fresh cell samples or thawed samples. Any number of positive controls may be set up for flow cytometry analysis using a Kasumi-3 control cell or other control cells.

The materials for cell count and cell viability analysis includes, but is not limited to, a flow cytometer, Isoflow Sheath Fluid, Coulter Clenz Cleaning Agent, and reagents including, but not limited to, CD45-FITC/CD34-PE, CD45-FITC/Isoclonic Control-PE, 7-AAD Viability Dye, Stem-Count Fluorospheres, Ammonium Chloride (NH4CL) Lysing Solution 10x Concentrated, and 22% Bovine Albumin Solution. See Stem Kit™ CD34+ HPC Enumeration Kit Package Insert - Version 03 (PNIM2390); Beckman Coulter Product Corrective Action, CXP 2.0 & 2.1 Panel Interruption -3/10/06, PCA-M-D-1013; 14.3 StemLab, Build Number 200706260856, Version 3.2.1. Materials for Flow Cytometry also include, but are not limited to, Isoflow Sheath Fluid; Coulter Clenz Cleaning Agent; and the following reagents about 20-25°C prior to use: CD117-PE, CD29-FITC, CD34-ECD, CD44-FITC, CD45-ECD, CD90-PC5, CD105-PE, CD166-PE, IgG-FITC, IgG-PE, IgG-ECD, IgG1-PC5, HLA-I-FITC, CD133-PE, HLA-II ECD, CD9-FITC, CD54-PE, CD10-PC5, CD59-FITC, CD63-PE, CD13-PC5, CD49e-FITC, CD81-PE, CD49f-PC5, CD44-FITC, CD38-PC5, CD29-FITC, CD105-PE, CD41-ECD, CD3-PC5, CD19-FITC, NANOG-FITC, SSEA3-PE, SSEA4-PE, CD14-FITC, CD56-PE, 7-AAD Viability Dye, Ammonium Chloride (NH4CL) Lysing Solution 10x Concentrated, Wash Media (comprising HBSS (Hanks with Ca+ and Mg+) 500ml, Heparin 5 ml, Human Serum Albumin 25% 50 ml, DNASE - 1 ampoule), Kasumi-3 cell line -CD117+ cells, Timer, and Vortex Mixer.

In an alternative embodiment, the cells may be analyzed by an alternative flow cytometry method similar to the previously described flow cytometry methods including the use of controls and methods for preparing fresh, pre-processing, post-processing, or thawed samples of menstrual stem cells. Cell markers may also be assessed by immunohistochemical analysis.

The total cell count for each sample may be analyzed before flow cytometry. If the sample contains >=3.5 X 10⁶ cells, the full panel may be assessed. If the sample contains < 3.5 X 10⁶ cells, less tubes may be necessary.

If a flow test needs to be run on a frozen sample that needs to be thawed, agitate vials in 37°C water bath without letting cells completely thaw. The thawed sample may be processed according to the methods for the previously discussed flow cytometry analysis methods and added to a number of tubes depending upon the number of cells according to the follow Table B.

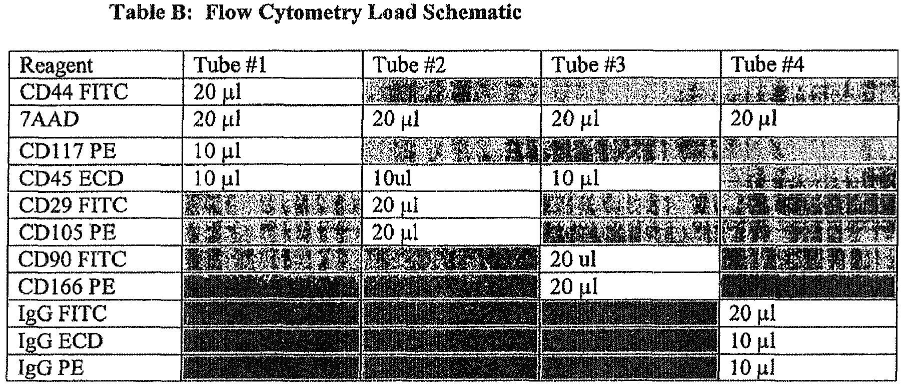

Each tube may be incubated at about 15°C to about 30°C for about 20 minutes. Protect from light. If running a fresh sample containing RBC's, add 500 µl of lyse solution and incubate at the same temperature for about ten minutes and protect from light. If running a density gradient or a thawed sample, do not lyse, If sample was not lysed, wash after about 20 minutes incubation with 1 ml of wash media and centrifuge for about one minute and then decant the supernatant. If sample was lysed, centrifuge sample for about one minute and decant lyse. Add about 1 ml of wash media, vortex, centrifuge again and then decant again. Add about 1 ml of Sheath fluid, vortex, and run on the FC500 or a suitable replacement flow cytometer.

**Cell Selection**

Cells may be selected for at least one of the cell markers for menstrual stem cells as described herein. Cells may also be subjected to a negative selection step to remove undesired cells. Throughout the cell selection process, the aseptic technique may be used. Cell selection may be used for fresh, post-processing, thawed cells that were previously cryopreserved, and cells in culture. Cell selections may be performed on as little as 2.5 million cells and up to 10 million cells. Cells may also be selected from a sample comprising less than 2.5 million cells or a sample comprising more than 10 million cells.

Materials for the cell selection include, but are not limited to, DNase, Pulmozyme (Genentech. Inc.) - 1 ampoule, any Anti-cell surface mark to be selected negatively or positively for example, Anti-CD117 antibody (Santa Cruz 104D2 or YB5.58 for BD Bioscience), Goat anti-mouse IgG microbead, and magnetic field.

A cellular suspension comprising menstrual stem cells obtained from a fresh sample, post-processing sample, thawed sample, or cultured sample of menstrual stem cells may be centrifuged at about 300 g for about 10 minutes at about 4°C. The supernatant may be removed without disturbing the cell pellet. The pellet may be resuspended with about 100ul of working buffer and about 20 ul of anti-cell surface antibody. In an embodiment, the anti-cell surface antibody is Anti-CD 117 antibody. The cells in solution may be incubated on ice for about 20 minutes to about 25 minutes. After incubation, about 2 ml of working buffer may be added to the cells and gently mixed. The mixture may be centrifuged for about 10 minutes at about 300 g at about 4°C. After centrifugation, the supernatant may be aspirated without disturbing the pellet. The pellet may be resuspended in about 80ul of working buffer. About 20 ul of goat anti-mouse IgG may be added to the cell suspension and gently mixed. The mixture may be incubated for about 30 minutes on ice. After incubation, the cells may be washed by adding about 2 ml of working buffer and then mixing the solution. The cells may be centrifuged at about 300 g for about 10 minutes at about 4°C.

A column may be used to separate selected cells from the unselected cells. A column may be prepared by wetting it in about 500 ul of working buffer. After centrifugation, the supernatant may be aspirated without disturbing the pellet. The pellet may be resuspended in about 500 ul of working buffer. To avoid cell adherence, additional DNase may be added to the cells. The cellular suspension may be added to the column using a pipette. Antibody labeled cells (positive fraction) should attach to the column subjected to a magnetic field provided by a MACS separator. Unlabeled cells (negative fraction) should flow through the column and be collected.

After the cellular suspension flows through the column and is collected as a negative fraction, the column may be washed at least 3 times using 500 ul of working buffer per wash. Each wash may completely flow through the column prior to the next wash. Each wash may be collected with the negative fraction. About 100ul of the negative fraction may be removed for analysis. A cell count using the Hemacytometer and viability using Trypan Blue or another method may be performed. Phenotype analysis may occur using flow cytometry as previously discussed or using another flow cytometry method. The negative fraction may be prepared for cryopreservation or placed in culture for further cell growth and expansion and later processing.

After the negative fraction is collected and the column is washed, another tube may be placed under the column to collect the positive fraction. About one ml of working buffer may be added to the column and remove the magnetic field form the column. The working buffer and positive fraction should be collected. A plunger may be used to remove as many labeled cells as possible from the column for the positive fraction. About 100 ul of the positive fraction may be removed for analysis including, but not limited to, cell count and viability using Trypan Blue or flow cytometry. The positive fraction may be cryopreserved, cultured, or prepared for therapeutic or cosmeceutical use.

The steps of selecting menstrual stem cells expressing MSC cell markers may occur according to any of the embodiments of the present invention as shown in FIGS. 3 through 6, 8 through 10, 13 through 19, 21 through 24, and 26 through 28. In particular, the selection may occur (a) prior to cryopreservation, (b) before and/or after at least one step of cell culture, or (c) after thawing cryopreserved cells. The steps of selecting menstrual stem cells expressing certain cell markers provides a population of enriched cells expressing the selected cell marker, which may be used for further cell culture, cryopreservation, or therapeutic or cosmeceutical use according the methodologies of the invention.

In an embodiment, the step of selecting menstrual stem cells expressing CD117 from a population of cells comprises labeling menstrual stem cells with anti-human CD117 antibodies and then labeling the CD117 stem cell-anti-human CD117 antibody complexes with magnetically-labeled antibodies capable of binding to the anti-human CD117 antibodies. Additionally, the method comprises labeling any cell expressing CD117 with anti-human CD117 antibodies and then labeling the CD117 cell-anti-human CD117 antibody complexes with magnetically-labeled antibodies capable of binding to the anti-human CD117 antibodies. The method of selecting menstrual stem cells expressing CD117 may include selecting any cell expressing CD117 that is collected in accordance with any of the methodologies of the present invention. The step of isolating menstrual stem cells comprises exposing the complexes comprising CD117 cells, anti-human CD 17 antibodies, and magnetically-labeled antibodies to a magnetic field to draw the magnetically-labeled antibodies and the rest of the complex to the column and washing all other CD117 negative cells through the column for analysis.

Throughout the steps of selecting menstrual stem cells expressing CD117, the cellular suspension of cells and working buffer (MACS® Separation running buffer with Dnase, Miltenyi) may be maintained at a cold temperature. Other magnetic separation kits may be suitable for use (R&D Systems). The cellular suspension may comprise a population of cells suspended in a wash solution if the steps of selecting endometrial/menstrual cells expressing CD117 occurs (a) prior to cryopreservation, (b) before and/or after at least one step of cell culture, or (c) after thawing cryopreserved cells as demonstrated throughout the embodiments of the invention.

The cellular suspension may be centrifuged at about 300 g for about 10 minutes. The pellet may be suspended in a working buffer with anti-human CD117 antibodies. For example, the working buffer may comprise, for example, PBS at about pH 7.2, bovine serum albumin, EDTA and about 0.09% azide (or suitable solution) (BD Biosciences). The pellet may be suspended, for example, in about 100 ul of working buffer and about 5 ug of purified antibodies having affinity for human CD117. The antibody may be monoclonal or polyclonal. The antibody may be purified IgG or other antibody capable of binding human CD 117. The antibody may be a mouse anti-CD117 antibody. For example, the antibody may be a monoclonal mouse anti-human CD117 antibody (available as 104D2 from Santa Cruz or YB5.58 from BD Biosciences).

The solution comprising the cells, working buffer and anti-CD117 antibodies are incubated for an incubation period. For example, the incubation period may comprise between about 20 minutes to about 25 minutes on ice. The incubation period may, alternatively, be shortened to less than about 20 minutes if the temperature is at least about 2°C to about 8°C or about 5 minutes to about 10 minutes if at least at room temperature. After the incubation period, the solution with the cells may be washed with working buffer to remove unbound antibody and then centrifuged. For example, the centrifugation may occur at about 300 g for about 10 minutes. After centrifugation, the supernatant is aspirated and may be saved for analysis, and the pellet is suspended in working buffer. For example, the volume of the working buffer may be about 80 ul.

A second batch of antibodies having microbeads affixed thereto and having an affinity for the anti-human CD117 antibody are added to the working buffer used to suspend the pellet. The microbeads may comprise, for example, iron oxide and polysaccharide. The microbeads may be biodegradable. The microbeads are available through Miltenyi Biotec. For example, the second batch of antibodies are specific for an antibody having affinity for human CD117, such as, for example, goat anti-mouse IgG antibody. The antibody may be monoclonal or polyclonal. The antibody may be capable of binding to the light chain and/or the heavy chain of mouse antibodies. The antibody may be for example a goat anti-mouse IgG microbead conjugate available through Miltenyi Biotec as product 130-048-401. A two (2) ml vial of the aforementioned goat anti-mouse IgG may be used for approximately 1.0x10^9 of total unseparated cells.

The cellular suspension is incubated for a second incubation period. For example, the incubation period may be in a range of about 30 minutes to about 35 minutes. Alternatively, the incubation period may be less than about 30 minutes where the incubation occurs at about 2°C to about 8°C or about 5 to about 10 minutes where incubation occurs at about room temperature. After the incubation period is complete, the cells are washed with working buffer, such as for example, about 2 ml of working buffer, and the cells are then centrifuged. For example, the centrifugation may occur at about 300 g for about 10 minutes. The supernatant may be aspirated and saved for analysis, and the pellet containing cells is suspended in working buffer, such as for example, about 500 ul of working buffer.

Cell Separation

The CD 117 stem cells may be separated from a cellular suspension in working buffer using an MS column to separate the CD117 stem cells. For example, an MS Column (Miltenyi Biotec) or other suitable column may be used. Alternatively, other suitable methods to separate cells may be used. A MiniMACS kit available through Miltenyi Biotec comprising a unit, multistand, MS columns and microbeads may be used for CD117 cell selection. The MS column may be prepared by rinsing it with working buffer. For example, the volume of working buffer used to rinse the column may be about 500 ul. The column is placed in a magnetic field of a MACS separator available through Miltenyi Biotec or suitable separator providing a magnetic field.

The cellular suspension in working buffer is added to the column with a pipette or other device capable of transferring a volume of liquid. The CD117 cells labeled with anti-human CD117 antibodies, which are bound with antibodies attached to microbeads, are held in the column due to the magnetic field of the MACS separator. Any unlabeled cells, along with the working buffer, should flow through the column and may be collected in a sterile tube for cell phenotyping and cell count. The unlabeled cells, which flow through the column, may be identified as a negative fraction. The column may be washed with working buffer after adding the cellular suspension. For example, the column may be washed at least 3 times or any amount of time that causes all or substantially all of the unlabeled cells to pass through the column. The effluent from the washing steps may be collected for cell phenotyping and count. The effluent may also be identified as a negative fraction.

The labeled CD117 stem cells may be collected from the column after the column is washed. The labeled CD117 cells are collected by placing a sterile tube under the column and removing the column from the magnetic field. Once the column is removed from the magnetic field, the labeled CD117 cells pass through the column and into the sterile tube. Residual labeled CD117 stem cells in the column may be washed out by adding working buffer to the column to wash the cells through the column and, optionally, by stripping the column with a plunger to release the cells. The collected labeled CD117 cells may be identified as the positive fraction. In order to obtain a more purified population of labeled CD117 cells, the positive fraction may, optionally, be run through a column at least one more time following the previously disclosed washing procedure. The positive fraction may be centrifuged at about 300 g for about 10 minutes and the supernatant aspirated. The pellet may be suspended in about 5 ml of working buffer.

The positive fraction and the negative fraction are analyzed with a hemocytometer to obtain a total count of viable cells. The negative fraction is analyzed by flow cytometry for phenotyping. Optionally, the positive fraction may be analyzed by flow cytometry for phenotyping.

The positive fraction containing stem cells expressing MSC cell markers, such as, for example, CD117 or other cell marker, may be prepared for cryopreservation in accordance with the methods of the present invention and described herein in further detail. About one ml of human serum albumin, about 3 ml of DPBS and about one ml of DMSO are added to the about 5 ml of the positive fraction. Alternatively, other culture media may be used in the step of preparing cells for cryopreservation, such as, for example, complete media, bovine serum albumin, fetal calf serum, fetal bovine serum, protein plasma fraction, or autologous serum. The solution containing MSCs is mixed and cooled on ice for about 10 minutes. About one ml of DMSO is added as a cryopreservative. Alternatively, about 1 ml of a mixture of about 6% HES hydroxyethyl starch and about 5% DMSO may be used as a cryopreservative. The resulting solution is aliquoted into cryovials. Alternatively, the resulting solution may be aliquoted into any container suitable for cryopreservation, such as, for example, a cryopreservation bag. The cryovials are then cryopreserved in a controlled rate freezer (Cryomed) in accordance with controlled rate freezer protocol of the present invention as described herein in further detail. Once the solution containing MSCs reaches the target temperature of about -90°C, the cryovials are transferred into a long term storage freezer and stored at about -135°C or less. Alternatively, the cryovials or other suitable cryopreservation container may be placed into a monitored dump freeze and frozen to about -80°C and then transferred into the vapor phase of liquid nitrogen in a long term storage freezer at about -135°C or less.

Alternatively, the positive fraction may be used to seed culture flasks and culture the cells in accordance with the methods of the present invention. MSCs may then be selected from the cell cultures and cryopreserved in accordance with the methods of the present invention.

The cells obtained in accordance with the present invention may be selected or isolated by other suitable processes and methods at any point in the practice of the invention wherein CD117 selection is employed. For example, rather than using the CD117 selection processes and methods described herein, the cells of the present invention, whether obtained pre-cryopreservation, post-thaw, or from cell culture, may be concentrated by serum deprivation using serum deprivation media in culture for about 2 weeks to about 4 weeks or other suitable time period at suitable temperatures and conditions. The serum deprivation media may be used with or without ES-FBS.

**Preparation for Cell Culture**

The cellular suspension comprising menstrual stem cells collected in accordance with the methodologies of the present invention may be cultured further in preparation for a further selection step, cryopreservation, or therapeutic or cosmeceutical use. The cellular suspension comprising menstrual stem cells may be prepared for cell culture after concentration according to the present invention or after being cryopreserved and thawed. When applicable, the thawing step comprises preparing aliquots of about 15 ml of density gradient media available as Histopaque (Sigma-Aldrich) or other suitable media at about room temperature for each vial containing about 5 ml of cryopreserved cells to be thawed and then preparing about 25 ml aliquots of Chang's complete media, DMEM complete media, or IMDM complete media or other suitable media for each vial containing about 5 ml of cryopreserved cells to be thawed. As described herein, fresh cell samples from post-processing, density gradient, and cell selection, may also be seeded in culture

Chang's complete media comprises about 325 ml of MEM alpha media (Gibco) as product 12571-063, add 20% Chang's Media to include about 90 ml of Chang B (basal) C110 (18% v/v) (Irvine Scientific), about 10 ml of Chang medium C from Supplement C106 (2% v/v) (Irvine Scientific), about 5 ml Penicillin/Streptomycin (liquid prepared with 10,000 units/ml Penicillin G Sodium and 10,000 ug/ml Streptomycin sulfate in 0.85% saline (Gibco - 15140-122), about 5 ml of L-glutamine 200 mM (100X) (Gibco - 25030-081), and about 75 ml of ES-Fetal Bovine Serum (15% v/v) (Gibco -10439-024). Chang medium may be purchases as complete media (Irvine Scientific).

Cryopreserved cells may be thawed using materials, including, but not limited to, Complete Media, DNAse (Genentech, Inc. #50242-100-40), TrypLE™ Express (Gibco, #12605-010), and DPBS (Mediatech, MT21-031 CV).

Cryopreserved cells must be removed from storage in vapor of liquid Nitrogen and placed in a 37°C water bath. The cells may be agitated in the water bath and not allowed to completely thaw. The partially thawed cells may be transferred to chilled complete Chang's media with DNAse and mixed by inversion. DNAse may be added at about 10 drops per 100 ml of Chang's media. Each about 5 ml cell preparation may be added to about 25 ml of chilled Chang's media. The suspension may be centrifuged at about 120 g for about 5 minutes. The supernatant may be aspirated and the cells resuspended in an appropriate media in preparation for cell culture.

Alternatively, and if thawing is not necessary, such as, for example, when the cellular suspension comprising menstrual stem cells is cultured in the absence of the step of cryopreservation, the cellular suspension may be diluted by placing the about 5 ml aliquot into the about 25 ml aliquot of chilled Chang's complete media containing about one mg of DNAse available through Pulmozyme.

The diluted cell suspension may be mixed by inversion. The suspension is centrifuged at about 840 g for about 7 minutes. The supernatant is aspirated while not disturbing the pellet. The pellet is brought up to a total volume of about 30 ml Chang's complete media. A small amount of the cell suspension is removed for analysis that includes cell count with a hemocytometer and viability testing using trypan blue or other suitable viability testing methodology. The about 30 ml suspension is overlaid on a density gradient solution available as Histopaque (Sigma-Aldrich) or other suitable media, and is centrifuged at about 420 g for about 30 minutes without a brake. The tube is removed from the centrifuge without disrupting the buffy coat. The supernatant is aspirated and the buffy coat is collected. The buffy coat is brought up to about 20 ml with Chang's complete media and is washed at about 840 g for about 7 minutes. The supernatant is aspirated, and the pellet is suspended in Chang's complete media up to about 10 ml but may also up to about 20 ml or about 30 ml, or even less than about 10 ml. An aliquot of the suspension, such as for example, about 100 ul, is removed to perform a cell count and viability analysis.

**Culture of Cryopreserved Cells**

Cells may be plated at about 2000 cells per cm² with about a 48 hour passage time. If a higher concentration of cells is used, passages may occur at about 24 hour intervals. A non-tissue culture treated T75 flask may be used to plate about 150,000 cells in about 15 ml of complete media. Plated cells may be incubated in a 5% CO2 incubator until subconfluent at about 80%.

Cells may be subject to any number of passages. When cells are ready to be dissociated from the flask, media may be aspirated from the flask. The flask may then be rinsed with about 5 ml of phosphate buffered solution (PBS) without calcium or magnesium. PBS may then be removed after the attached cells are washed once. About 2 ml of Trypsin-like enzyme or other suitable enzymatic solution may be added to a T75 flask (one ml per T25 flask), pre-warmed at about 37°C. The flask may be agitated to coat the cells with the enzyme-like solution. The flask with the enzyme-like solution may be incubated for about 5 minutes at about 37°C in an incubator. After incubation, the flask may be tapped on a solid surface to dislodge the cells. The contents of the flask may be diluted with about 2 ml of complete media to rinse and stop the reaction. Cells may be washed with about 2ml to about 5 ml DPBS and transferred to a 50 ml centrifuge tube for washing. The tube may be centrifuged at about 120 g for about 5 minutes. The supernatant may be discarded after centrifugation, and the harvested cells may be suspended in Chang's complete media. About 7 ml of cellular suspension may be used for culture in a T25 Flask and about 15 ml for a T75 Flask.

Chang's complete media for Cell Culture comprises about 325 ml of MEM alpha media (Gibco #12571-063), about 90 ml of Chang B (basal media) (18% v/v) (Irvine Scientific, C110), about 10 ml of Chang C (2% v/v) (Irvine Scientific, Supplement C106), about 5 ml Penicillin/Streptomycin (10,000 units/ml Penicillin G Sodium & 10,000 ug/ml Streptomycin sulfate (Gibco #15140-122)), about 5 ml of L-glutamine 200 mM (100X) (Gibco, #25030-081), and about 75 ml of ES-Fetal Bovine Serum (15% v/v) (Gibco #10439-024).

**Alternative embodiment of thawing and culturing methods.**

After cells are removed from liquid nitrogen vapor, agitate the cryovial in 37°C water bath without letting cells completely thaw. Partially thawed cells may be transferred to chilled Chang's media with DNAse and mixed gently by inversion. For each 5 ml of cell suspension, about 25 ml of chilled Chang's media may be used. DNAse may be added to the Chang's media at about 10 drops for 100 ml of chilled Chang's media. The cellular suspension may be centrifuged at about 120 g for about 5 minutes. Supernatant may be aspirated and the cells may be placed in the previously discussed antibiotic media. After about at least 12 hours incubation at about 37°C, the cells may be washed using Chang's Complete Media.

The washed cells may then be seeded for cell culture. In an embodiment, thawed cells may be plated at about 40,000 cells per cm² or 1 million cells per T-25. Cells may be plated at 2000 cells per cm² with a 48 hour passage time. If more cells are plated, passages may occur within about 24 hour intervals. A non-tissue culture treated T75 flask may be used to plate about 150,000 cells in about 15 ml of complete media. Cells may be incubated in a 5% CO2 incubator until subconfluent to 80%. When cells are ready for dissociation from the flask, the media may be aspirated from the flask. The flask may then be rinsed with about 5 ml of phosphate buffered solution without calcium or magnesium. PBS may be removed after the attached cells have been washed at least once. About two ml of Trypsin-like enzyme may be added to a T75 flask (one ml for a T25 flask), pre-warmed at about 37°C, and the flask may be agitated to coat the cells with the enzyme. The flask with the enzyme may be incubated for about 5 minutes at about 37°C in an incubator. After incubation, the flask may be gently tapped on a solid surface to dislodge the cells. The contents of the flask may be diluted with about 2 ml of complete media to rinse and stop the reaction. Wash cells with about 2 ml to about 5 ml DPBS and transfer the resuspended cells to a 50 ml centrifuge tube for washing. The tube may be centrifuged at about 120 g for about 5 minutes. The supernatant may be discarded after centrifugation, and the harvested cells may be suspended in Chang's complete media and used for the next passage. About 7 ml may be used for a T25 Flask and about 15 ml for a T75 Flask.

**Other Cell Culture Methods**

The cells in suspension may be seeded at about 40,000 cells per cm² into an untreated culture flask in Chang's complete media, DMEM complete media (with high glucose or low glucose), or other suitable media. The flask may be incubated in about 5% CO₂ in a CO₂ incubator (Thermo Electron Corp. or Bioscience Technologies), or any other suitable incubator system at a temperature of about 37°C. The cell cultures are monitored for turbidity and pH change. If the pH is high, about 50% of the media should be changed.

The flask may be incubated initially for about 7 days or until the media is significantly out of range as noted by the color of the phenol red indicator in the media. If the pH remains stable after about 7 days, the media is changed with fresh media (also referred to herein as "virgin media"), as necessary. Almost all media is changed. After the media change at day 7, the cells may become confluent by day 8 to day 21. Once attaining about 70-80% confluence, the cells may be sub-cultured. Cell cultures are sub-cultured using the trypsin-like enzyme such as TrypLE™ Express (Gibco) or any other suitable enzyme to provide enough cells to perform the cell selection in accordance with the present invention. For example, cell selection may occur with about 10 million cells. Cell selection may also occur with greater than or less than about 10 million cells.

In accordance with the invention, cells may be collected from the cell culture at a suitable time. In order to collect the cells, adherent cells should be dissociated from the flask. In order to dissociate the cells from the flask, the media is aspirated via an automated pipette. The flask is then rinsed with about 5 ml of Phosphate Buffered Saline (PBS) without calcium or magnesium. The PBS is then removed from the flask with attached cells that have been washed at least once. About one ml of a Trypsin-like recombinant enzyme such as TrypLE™ Express (Gibco), or any other suitable enzyme, should be added, preferably pre-warmed at about 37°C, to the cell culture in the flask. The flask is agitated to coat the cells with the enzyme. The flask with enzyme should be incubated for about 5 minutes at about 37°C. After incubation, the flask should be gently tapped on a solid surface to dislodge the cells. The flask should be diluted with about 2 ml of Chang's complete media and the cells transferred to a 15 ml centrifuge tube for washing with Chang's complete media, DMEM complete media (with high glucose or low glucose), or other suitable media. The tube should be centrifuged for about 7 minutes at about 100 g. The supernatant is aspirated and discarded. The pellet is suspended in a suitable volume of Chang's complete media, DMEM complete media (with high glucose or low glucose), or other suitable media.

At this point, the cells may be selected from the cell culture in accordance with cell selection methodologies of the present invention. Once selected, the cells may be plated on a Petri dish, seeded into a culture flask, or cryopreserved in accordance with present invention.

The cells may be plated in a 9 cm² Petri dish using Chang's complete media (about 15% FBS). Alternatively, the cells may be placed in a culture flask with a vented cap. If the pH of the media becomes high, the cells may be washed with Chang's complete media. When necessary after suitable growth, the cells may be dissociated from the Petri dish or culture flask using a trypsin-like enzyme and then placed in an untreated culture flask using Chang's complete media. After suitable growth, the cells may be dissociated using a trypsin-like enzyme such as TrypLE™ Express (Gibco) and then seeded in a fresh untreated culture flask. This process may be repeated in order to maintain desired cell growth. The cells may be washed with fresh media, or about 50% of the media or other suitable amount may be replaced with fresh media if the pH of the media is high. At this point, the cells may be selected from the cell culture in accordance with the cell selection methodology of the present invention. The selected cells may be plated on a Petri dish, seeded into a culture flask, or cryopreserved in accordance with present invention.

Current Good Manufacturing Practices (cGMP) standards and current Good Tissue Practices (cGTP) established by the United States Food and Drug Administration in the Code of Federal Regulations are followed throughout the practice of the invention.

The aforementioned steps of the processes, methods, and systems of the present invention may be alternatively embodied as illustrated by FIGS. 2 through 28.

**M2-01 Cells**

In an example, menstrual stem cells were collected according to the practices of the invention. In particular, about 0.4 ml of a menstrual flow sample designated and referred to herein as M2-01 was collected by a donor during a menstrual cycle. A sterilized collection device was placed within the donor's vagina for time enough to collection a sample of menstrual flow. The about 0.4 ml of menstrual flow sample was poured from the collection device into about 10 ml of collection media in a collection tube. The collection media comprised DPBS (Mediatech) without calcium, magnesium or phenol red, Penicillin at about 100 units per milliliter, Streptomycin at about 100 micrograms per milliliter, and preservation-free Heparin in a range of about 10 units to about 20 units per milliliter of the DPBS. The menstrual flow sample and collection media were sealed in the collection tube, packed on ice in a collection container, and transported by shipment to a processing facility.

The menstrual flow sample arrived at the processing facility within about 24 hours after collection. The collection container comprising the about 10.0 ml of menstrual flow sample in collection media with antibiotics was disinfected upon arrival at the processing facility, transferred into a clean room, and placed on ice. The collection container was centrifuged at about 2000 rpm for about seven minutes at a temperature of about 18°C and was then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results were negative for aerobic and anaerobic microorganisms.

The remaining supernatant was aspirated and discarded. The pellet was suspended in about 2 ml of DPBS. About 1 ml of cellular suspension was collected as a post-processing sample and analyzed by cell count, flow analysis, and viability. The total cell count performed using a Hemocytometer indicated that about 7.5 million cells were collected in the post-processing sample. Flow cytometry analysis was performed according to the methods of the present invention using a FC500 Flow Cytometer to identify certain cell surface markers present on menstrual stem cells in the post-processing sample. As show in FIGS. 29a to 29j, the results indicate that CD117⁺ cells were collected at a concentration of about 0.4% of the cell population. Additionally, the results indicated that CD45 negative cells were collected at a concentration of about 58% of the cell population. The results indicated that CD44+ cells were collected at a concentration of about 17% of the cell population, CD166+ cells were collected at a concentration of about 3% of the cell population, CD105+ cells were collected at a concentration of about 8.0% of the cell population, CD90+ cells were collected at a concentration of about 6.8% of the cell population, CD29+ cells were collected at a concentration of about 0.1% of the cell population, and results indicated that CD34+ cells were collected at a concentration of about 0.1% of the cell population. The flow cytometry analysis was also performed using 7AAD to assess cell viability. The results indicate that the menstrual stem cells were about 95% viable.

The remaining cellular suspension was cultured according to the methods of the present invention. In particular, part of the cellular suspension was overlaid on 15 ml of a density gradient solution with 1.083 g/ml (Histopaque, Sigma-Aldrich). The cellular suspension was centrifuged at about 420 g for about 30 minutes without a brake. The supernatant was aspirated, and the buffy coat was removed and placed in a tube. Chang's complete media was added to the buffy coat to bring the total volume up to about 30 ml. The solution was washed twice at about 840 g for about 7 minutes. After centrifugation on the second wash, the supernatant was removed leaving a pellet, and about 15 ml of Chang's complete media was added to the tube to suspend the pellet. About 100 uL of suspension was removed to perform cell count analysis with a hemocytometer and viability analysis using trypan blue.

About one million cells were seeded at about 40,000 cells per cm² into a T25 untreated culture flask in Chang's media on day 1. Flasks were incubated in about 5% CO₂ at about 37°C temperature. On day 8, the cells went through passage 1 and the media was changed.

As used throughout this disclosure, a passage generally involved dissociating cells from the flask by aspirating the media with an automated pipette. The flask was rinsed with about 5 ml of Phosphate Buffered Saline (PBS) without calcium or magnesium. The PBS was then removed after the flask with cells attached had been washed once. About one ml of Trypsin-like enzyme (TrypLE™ Express - Gibco) pre-warmed at about 37°C was added to the flask and the flask was agitated to coat the cells with the enzyme. The flask with the enzyme was incubated for about 5 minutes at about 37°C in an incubator. After incubation, the flask was gently tapped on a solid surface to dislodge the cells. The contents of the flask were diluted with about 2 ml of complete media, and the cells were transferred to a 15 ml centrifuge tube for washing. The tube was centrifuged for about 7 minutes at about 100 g. The supernatant was discarded after centrifugation, and the harvested cells were suspended in Chang's complete media and placed into at least one new T25 or T75 untreated cell culture flask.

Additional passages of the cell culture occurred on days 10, 14, 19, 21, 24, 26, 29, 32, and 35. On day 8, about 165,000 cells were harvested and seeded in Chang's media in untreated tissue culture flasks according to the culturing methods of the present invention. On day 10, about 270,000 cells were harvested and seeded in Chang's media in untreated tissue culture flasks. On Day 14, about 840,000 cells were harvested and seeded in Chang's media in untreated tissue culture flasks. On Day 19, about 2.2 million cells were harvested and seeded in Chang's media in untreated tissue culture flasks. On day 21, about 5 million cells were harvested, of which about 3.5 million cells were used for cell phenotype. On day 24, about 3.0 million cells were harvested and seeded in Chang's media in untreated tissue culture flasks. On day 26, about 4.9 million cells were harvested and seeded in Chang's media in untreated tissue culture flasks. On day 29, about 14.8 million cells were harvested and seeded in Chang's media in untreated tissue culture flasks wherein about 11.1 million cells were used for positive selection of cells expressing CD117 and about 3.5 million cells were used for phenotypic analysis, and about 200,000 cells were seeded into culture. On day 32, about 1.3 million were harvested, of which about 1 million cells were used for flow phenotype and about 300,000 cells were seeded in Chang's media in untreated tissue culture flasks.

On day 29, and as previously discussed, the cells harvested from culture were subjected to CD117 selection according to the selection methodologies of the present invention as described herein. Prior to CD117 stem cell selection, an aliquot of each cell culture was removed for analysis with results shown in Table C.

In particular, the harvested cells were labeled with mouse anti-human CD-117 antibodies. About 10 million harvested cells were centrifuged and the supernatant was aspirated. The pellet was suspended in about 100 ul of the working buffer that contained about 5 ug of purified mouse anti-human CD117 monoclonal antibody (IgG₁) with product 104D2 (Santa Cruz). The working buffer available through BD Biosciences contains PBS at about pH of 7.2, bovine serun albumin, EDTA and about 0.09% azide. The solution containing cells and antibodies was incubated for about 20 minutes to about 25 minutes on ice. The cells were washed with working buffer to remove unbound antibodies, and the solution containing the cells and antibodies were centrifuged at about 300 g for about 10 minutes. After centrifugation, the supernatant was aspirated and discarded, and the pellet was suspended in about 80 ul of working buffer.

The CD117 stem cell-antibody complexes were labeled with goat anti-mouse antibodies capable of binding to the heavy chain and/or the light chain of mouse IgG. Magnetic microbeads are attached to the goat anti-mouse antibodies. The goat anti-mouse IgG antibodies were added to the suspended pellet in working buffer and incubated on ice for about 30 minutes to about 35 minutes. After incubation the cells were washed with about 2 ml of working buffer and centrifuged at about 300 g for about 10 minutes. The supernatant was aspirated and the final cells were suspended in about 500 ul of working buffer.

The cell suspension comprising harvested stem cells and solutions, including working buffer, was kept cold throughout the experiment to prevent non-specific labeling of cells.

An MS Column (Miltenyi Biotec) was prepared by rinsing about 500 ul of working buffer through the column. Minimacs available through Miltenyi Biotec was used for CD117 stem cell selection. The column was placed in the magnetic field of the MACS separator available through Miltenyi Biotec. The cell suspension was added to the column via pipette. The unlabeled cells and working solution flowed through the column and were collected in a sterile tube for phenotyping and cell count analysis. The tube with the collected effluent was labeled as a negative CD117 cell fraction. The column was washed 3 times with working buffer after adding the cells suspension. A sterile tube was placed under the column, which was removed from the magnetic field to allow the positive fraction to pass through the column and into the sterile tube. About one ml of working buffer was added to the column, and a plunger was pushed through column to release the positive fraction of the cells. The tube with the collected effluent was labeled as a positive CD117 cell fraction.

The positive and negative fractions were analyzed with a hemocytometer to obtain the total number of viable cells. The negative fraction was analyzed by flow cytometry. M2-01 cells were analyzed at various steps throughout processing and culture with the results shown in FIGS. 30a to 30e and as summarized on the following Table C:

**Table C: M2-01 Flow Cytometry and Culture**

| **Passage #** | | | | | | | **CD105** | | | |
|---|---|---|---|---|---|---|---|---|---|---|
| | **CD117-PE** | **CD44-FITC (POS)** | **CD45-ECD (NEG)** | **7AAD-TEST** | **7AAD-ISO** | **CD166** | | **CD29** | **CD34** | **CD90** |
| M2-01 P0 | 0.40% | 17.70% | 58.90% | 95.40% | 95.10% | 3.70% | 8.00% | 0.10% | 0.10% | 6.80% |
| M2-01 P4 | 40.30% | 88.30% | 77.10% | 97.50% | 97.50% | 94.70% | 16.10% | 81.10% | 0.10% | 84.90% |
| M2-01 P7 | 2.10% | 89.20% | 87.70% | 95.20% | 95.60% | 91.00% | 33.00% | 82.90% | 0.20% | 67.90% |
| M2-01 P8 | 2.50% | 70.80% | 94.50% | 97.30% | 95.30% | | | | | |
| M2-01 POS FRAC | 7.20% | 93.60% | 83.30% | 95.80% | 91.90% | | | | | |
| M2-01 NEG FRAC | 4.50% | 94.10% | 86.40% | 94.70% | 92.80% | 92.00% | 45.60% | 90.40% | 0.10% | 79.60% |

**M2-02C Cells**

In another example, menstrual stem cells were collected according to the practices of the invention. In particular, a menstrual flow sample of about less than 1.0 ml designated and referred to herein as M2-02C was collected by a donor during a menstrual cycle. A sterilized collection device was placed within the donor's vagina for time enough to collect a sample of menstrual flow. The menstrual flow sample was poured from the collection device into about 10 ml of collection media in a collection tube. The collection media comprised DPBS (Mediatech) without calcium, magnesium or phenol red, Penicillin at about 100 units per milliliter, Streptomycin at about 100 micrograms per milliliter, and preservation-free Heparin in a range of about 10 units to about 20 units per milliliter of the DPBS media. The menstrual flow sample and collection media were sealed in the collection tube, packed on ice in a collection container, and transported by shipment to a processing facility.

The menstrual flow sample arrived at the processing facility within about less than 24 hours after collection. The collection container comprising the about 10.0 ml of menstrual flow sample and collection media with antibiotics upon arrival at the processing facility was disinfected, transferred into a clean room, and placed on ice. The collection container was centrifuged at about 2000 rpm for about seven minutes at a temperature of about 18°C and then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results were negative for aerobic and anaerobic microorganisms.

The remaining supernatant was aspirated and discarded. The pellet was suspended in about two ml of buffered saline. About one ml of cellular suspension was collected as a post-processing sample and analyzed by flow cytometry analysis to obtain cell count, flow analysis, and viability.

The total cell count of the processing sample was performed using a Hemocytometer. About 19.7 million cells were collected in the processing sample. Flow cytometry analysis was performed to identify cell surface markers using a FC500 Flow Cytometer. As shown in FIGS. 3 1a to 31iii, the results indicated that CD 117⁺ cells were collected at a concentration of about 0.2% of the cell population. Additionally, the results indicated that CD45 negative cells were collected at a concentration of about 97% of the cell population. The results indicated that CD44+ cells were collected at a concentration of about 1% of the cell population, CD166+ cells were collected at a concentration of about 0.1 % of the cell population, CD105+ cells were collected at a concentration of about 1% of the cell population, CD90+ cells were collected at a concentration of about 0.4% of the cell population, CD29+ cells were collected at a concentration of about 0.5% of the cell population, and results indicated that CD34+ cells were collected at a concentration of about 0% of the cell population. The results also indicate that about 99% of cells collected were viable as determined by 7AAD.

All of the suspended cells were transferred to one 50 ml conical collection tube, and the volume of the cellular suspension was brought up to a total volume of about 31 ml using the wash solution. About half of the 30 ml of the cellular suspension was concentrated by density gradient centrifugation. Cells were processed by methods including density gradient centrifugation and volume reduction, concentration, and wash. The cellular suspension was brought up to about 30 ml and was underlayed with a density gradient of about 15 ml of Lymphocyte Separation Medium (Density 1.077 - 1.080 g/ml at about 20°C - Cellgro). The cellular suspension and the density gradient were subjected to centrifugation at about 14,000 rpm for about 30 minutes without a brake at a temperature at about 20°C. A buffy coat layer formed and contained the menstrual stem cell population. The supernatant above the buffy coat layer was aspirated and discarded. The buffy coat layer was removed along with some wash solution and as little volume of density gradient as possible. The remaining density gradient and pellet containing red blood cells was discarded.

The buffy coat layer was placed in a 50 ml conical collection tube and the volume was brought up to about 30 ml with wash solution. The cellular suspension was subjected to centrifugation at about 2000 rpm for about seven minutes at room temperature of about 20°C. After centrifugation, about one ml of the supernatant near the top of the conical collection tube was removed and used to inoculate a BacT/ALERT blood culture bottle. The blood culture bottle was incubated at about 37°C in a BacT/ALERT system for about fourteen days. The results indicate that the supernatant and the cellular suspension did not contain any aerobic or anaerobic microorganisms. The remaining supernatant was aspirated while carefully leaving the pellet comprising the menstrual stem cells.

About one million cells were seeded at about 40,000 cells per cm² into a T25 untreated culture flask in 15% Chang's complete media. Flasks were incubated in about 5% CO₂ at 37°C temperature. At day 6, and at passage 1, cells were harvested at about 80,000, day 11, 324,000 cells, day 17, 600,000 cells, day 9, 2.9 million (2.3 million provided for cell phenotype), day 22, 1.2 million, day 24, 2.6 million, day 29, 26.8 million cells (3.5 for cell phenotype, 10 million for CD117 selection, 131 million cryopreserved, 200,000 cells seeded for culture). Aliquots of cells and media were collected throughout the cell culture for phenotyping and validity assessment. Phenotyping and validity assessment was performed in accordance with the methods described herein for all of the cell culture experiments relating to all of the cell lines. Cell counts during cell culture were performed using a hemocytometer relating to all of the cell lines. The data collected from the assessment showed that Cell Line M2-02C was initially positive for CD117, CD166, CD105, CD29, CD90, and CD44, and negative for CD45 with a high percentage of viability.

**Table D: Flow cytometry results for M2-02C during cell culture**

| **Passage #** | **CD117-PE** | **CD44-** | **CD45-ECD** | **7AAD TEST** | **CD105** | **CD 29** | **CD 34** | **CD 90** | **CD166** |
|---|---|---|---|---|---|---|---|---|---|
| | | **(POS)** | **(NEG)** | | | | | | |
| M2-02A | 0.2% | 0.6% | 96.7% | 98.9% | 1.1% | 0.3% | 0.0% | 0.0% | 0.6% |
| M2-02B | 0.2% | 0.9% | 93.2% | 98.4% | 2.3% | 1.0% | 0.0% | 0.5% | 1.2% |
| M2-02C | 0.1% | 0.5% | 98.0% | 99.5% | 0.9% | 0.5% | 0.1% | 0.4% | 0.2% |
| M202 P3 | 15.5% | 97.1% | 99.9% | 99.0% | 96.0% | 97.3% | | 67.0% | 95.8% |
| M202 P5 | 5.9% | 97.1% | 100.0% | 91.1% | 47.9% | 97.7% | | | |
| M2-02C P6 | | 96.3% | 88.8% | 99.5% | 86.6% | 92.2% | 10.3% | 81.8% | 94.8% |
| M2-02C P7 | 14.6% | 95.9% | 84.3% | 99.3% | 92.4% | 95.1% | 18.9% | 91.5% | 93.4% |
| M2-02C P8 | 6.3% | 98.1% | 97.4% | 99.4% | 52.2% | 90.4% | 0.5% | 83.2% | 98.2% |
| M2-02C-P9 | 12.7% | 97.5% | 90.8% | 99.2% | | | | | |
| M2-02C P10 | 12.7% | 97.6% | 91.5% | 99.0% | | | | | |
| M202C P11 | 34,9% | 97.3% | 72.6% | 98.7% | | | | | |
| M202C P12 | 24.6% | 96.7% | 87.3% | 98.9% | 93.7% | 98.3% | 10.7% | 90.0% | 91.8% |
| M2-02C P14 | 21.1% | 97.8% | 60.5% | 98.2% | 95.7% | 98.0% | 21.2% | 88.3% | 95.7% |
| M2-02C-P17 | 35.1% | 92.0% | 75.0% | 95.9% | 84.0% | 91.0% | 37.6% | 96.0% | 98.8% |
| M2-02C P18 | 18.4% | 88.8% | 70.1% | 98.1% | | | | | |
| M2-02C P19 | 14.9% | 92.1% | 67.1% | 97.1% | | | | | |
| M202C-P21 | 16.5% | 89.5% | 70.8% | 98.6% | | | | | |
| M202C P26 | 40.1% | 92.2% | 40.1% | 92.9% | | | | | |
| M2-02C P28 | 14.9% | 78.5% | 72.7% | 94.9% | | | | | |
| | | | | | | | | | |
| M2-02C-P3 thaw | 37.4% | 89.6% | 86.6% | 97.5% | | | | | |
| M202C THAW P4 | | | 100.0% | 99.7% | 98.4% | 98.7% | | | 92.9% |
| M202C-THAW-P6 | 10.6% | 97.6% | 99.8% | 98.1% | 96.4% | 97.2% | | | 88.2% |
| M2-02C FICOL P4 | 0.0% | 90.1% | 82.0% | 99.1% | | | | | |
| M2-02C FICOL-P8 | 21.4% | 91.6% | 84.4% | 97.4% | 94.2% | 91.1% | 13.0% | 88.8% | 94.1% |
| M2-02C FICOL-P9 | 9.7% | 95.2% | 97.2% | 98.3% | | | | | |
| M2-02C-FICOL-P10 | 13.8% | 92.3% | 89.0% | 98.0% | | | | | |
| M2-02C FICOL P12 | 12.3% | 90.5% | 94.2% | 99.0% | | | | | |
| M2-02C P13 FICOL | 5.4% | 88.9% | 79.1% | 94.8% | | | | | 77.8% |
| M2-02C FICOL P14 | 23.3% | 94.3% | 69.9% | 98.6% | | | | | |
| M2-02C P15 | 20.2% | 92.3% | 73.7% | 98.4% | 32.0% | 95.5% | 10.4% | 94.9% | 85.3% |
| FICOL | | | | | | | | | |
| M2-02C-P18 FICOL | 0.0% | 89.0% | 67.5% | 98.5% | 28.8% | 94.0% | 21.3% | 95.6% | 65.7% |
| M202C P21 FICOL | 4.9% | 85.9% | 57.0% | 96.6% | 90.3% | 92.3% | 28.7% | 88.1% | 75.4% |
| M202C FICOL P26 | 18.0% | 67.9% | 50.3% | 90.1% | 20.0% | 74.6% | 39.7% | 57.3% | 38.3% |
| M202C FICOL P28 | 0.2% | 65.7% | 69.3% | 93.7% | | | | | |
| | | | | | | | | | |
| M2-02C FICOL NEG | 17.4% | 98.4% | 89.8% | 99.0% | 84.6% | 89.5% | 19.7% | 95.6% | 86.4% |
| M2-02C FICOL POS | 13.7% | 89.1% | 66.3% | 92.2% | | | | | 78.4% |
| M2-02C FICOL POS | | | | | 97.9% | 97.1% | 8.0% | 90.7% | |
| M2-02C-FICOL-P5 POS | 0.0% | 78.0% | 98.4% | 89.1% | | | | | |
| M2-02C-P9 FICOL POSITIVE | 1.7% | 87.5% | 67.1% | 95.9% | | | | | |
| M2-02C-P10 FICOL POS | 14.8% | 93.2% | 69.7% | 98.8% | | | | | |
| M202C-P12 POS FICOL | 29.0% | 93.5% | 74.0% | 97.4% | | | | | |
| M2002C P13 POSI-FICOL | 2.8% | 90.6% | 65.5% | 98.7% | | | | | |
| M202-P14 FICOL POS | | | | | 91.0% | 92.7% | 13.6% | 66.4% | 80.3% |
| M202C FICOL P17 POS | 34.4% | 82.9% | 39.0% | 93.5% | | | | | |
| M2-02C-P18 POS FICOL POS FXN | 0.0% | 69.7% | 68.9% | 94.8% | | | | | |
| M2-02C-P19 POS FICOL | 14.4% | 93.5% | 57.8% | 99.0% | | | | | |
| | | | | | | | | | |
| M2-02C P2 POS | | | 87.3% | 98.8% | 86.9% | 94.5% | 14.3% | 87.7% | 89.6% |
| M2-02C-P3 POS | 5.1% | 82.6% | 93.8% | 99.0% | | | | | 92.6% |
| M2-02C P4 POS | | 97.6% | 99.1% | 98.7% | | | | | |
| M2-02C-P5 POS | 10.1% | 96.0% | 74.0% | 99.4% | 98.0% | 97.4% | 4.7% | 82.6% | 89.5% |
| M2-02C-P7 POS | 3.8% | 92.3% | 79.4% | 99.2% | | | | | |
| M2-02C-P8 POS | 5.4% | 90.0% | 66.5% | 96.5% | 93.2% | 91.3% | 24.2% | 91.6% | 76.6% |
| M202C-P11 POS | 23.6% | 89.6% | 79.1% | 97.1% | | | | | |
| M202C-P15 POS FRAC | 12.2% | 83.9% | 85.8% | 95.5% | 84.5% | 85.7% | 16.2% | 81.2% | 73.6% |
| | | | | | | | | | |
| M2-02C NEG | 17.8% | 93.4% | 77.3% | 97.8% | 99.0% | 98.3% | 96.9% | 94.9% | 96.1% |

The results of the flow cytometry analysis for the cultured M2-02C at Passage 4 are presented in FIGS. 32a through 32iii. The cultured sample comprises about one million cells seeded in accordance with the invention. The results indicated at Passage 4 that CD117⁺ cells were collected at a concentration of about 0.0% of the cell population. Additionally, the results indicated that CD45- cells were collected at a concentration of about 82% of the cell population. The results indicated that CD44+ cells were collected at a concentration of at about 93% of the cell population. The results also indicated that about 99% of cells collected were viable as determined by 7AAD. At passage 8 for M2-02C, the results indicated that CD117⁺ cells were collected at a concentration of about 14% of the cell population. Additionally, the results indicated that CD45- cells were collected at a concentration of about 84% of the cell population. The results indicated that CD44+ cells were collected at a concentration of about 93.3% of the cell population, CD166+ cells were collected at a concentration of about 93% of the cell population, CD105+ cells were collected at a concentration of about 90% of the cell population, CD90+ cells were collected at a concentration of about 87% of the cell population, CD29+ cells were collected at a concentration of about 85% of the cell population, and results indicated that CD34+ cells were collected at a concentration of about 0.2% of the cell population. The results also indicate that about 97% of cells collected were viable as determined by 7AAD.

The cells in cell culture were subjected to CD117 stem cell selection at passage 4 in accordance with the methodologies of the present invention and as described herein. The harvested cells were labeled with mouse anti-human CD-117 antibodies. About ten million harvested cells were centrifuged and the supernatant was aspirated. The pellet was suspended in about 100 ul of the working buffer that contained about 5 ug of purified mouse anti-human CD117 monoclonal antibody (IgG₁) with product 104D2 (Santa Cruz). The working buffer available through BD Biosciences contains PBS at about pH of 7.2, bovine serum albumin, EDTA and about 0.09% azide. The solution containing the cells and antibodies was incubated for about 20 minutes to about 25 minutes on ice. The cells were washed with working buffer to remove unbound antibodies, and the solution containing the cells and antibodies were centrifuged at about 300 g for about 10 minutes. After centrifugation, the supernatant was aspirated and discarded, and the pellet was suspended in about 80 ul of working buffer.

The CD117 stem cell-antibody complexes were labeled with goat anti-mouse antibodies capable of binding to the heavy chain and/or the light chain of mouse IgG. Magnetic microbeads are attached to the goat anti-mouse antibodies. The goat anti-mouse IgG antibodies were added and incubated on ice for about 30 minutes to about 35 minutes. After incubation the cells were washed with about 2 ml of working buffer and centrifuged at about 300 g for about 10 minutes. The supernatant was aspirated and the final cells were suspended in about 500 ul of working buffer.

The cell suspension comprising harvested stem cells and solutions, including working buffer, was kept cold throughout the experiment to prevent non-specific labeling of cells.

An MS Column (Miltenyi Biotec) was prepared by rinsing about 500 ul of working buffer through the column. Minimacs available through Miltenyi Biotec was used for CD117 stem cell selection. The column was placed in the magnetic field of the MACS separator available through Miltenyi Biotec. The cell suspension was added to the column via pipette. The unlabeled cells and working solution flowed through the column and were collected in a sterile tube for phenotyping and cell count analysis. The tube with the collected effluent was labeled as a negative CD117 cell fraction. The column was washed 3 times with working buffer after adding the cells suspension. A sterile tube was placed under the column, which was removed from the magnetic field to allow the positive fraction to pass through the column and into the sterile tube. About one ml of working buffer was added to the column, and a plunger was pushed through column to release the positive fraction of the cells. The tube with the collected effluent was labeled as a positive CD 117 cell fraction.

The positive and negative fractions were analyzed with a hemocytometer and flow cytometry at various steps throughout other processing with density gradient, CD117 selection, and subsequent culture, with the results shown generally for positive and negative fractions in Table D and the positive fractions in Table E below.

**M2-03E Cells**

In yet another example, menstrual stem cells were collected according to the practices of the invention. In particular, a sample of menstrual flow designated and referred to herein as M2-03E was collected by a donor during a menstrual cycle. A sterilized collection device was placed within the donor's vagina for time enough to collection a sample of menstrual flow. The menstrual flow sample was poured from the collection device into about 10 ml of collection media in a collection tube. The collection media comprised DPBS (Mediatech) without calcium, magnesium or phenol red, Penicillin at about 100 units per milliliter, Streptomycin at about 100 micrograms per milliliter, and preservation-free Heparin in a range of about 10 units to about 20 units per milliliter of the DPBS media. The menstrual flow sample and collection media were sealed in the collection tube, packed on ice in a collection container, and transported by shipment to a processing facility.

The menstrual flow sample arrived at the processing facility within about less than 24 hours after collection. The collection container comprising the about 10.0 ml of menstrual flow sample and collection media with antibiotics was disinfected upon arrival at the processing facility, transferred into a clean room, and placed on ice. The collection container was centrifuged at about 2000 rpm for about seven minutes at a temperature of about 18°C and then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results were negative for aerobic and anaerobic microorganisms.

The remaining supernatant was aspirated and discarded. The pellet was suspended in about two ml of buffered saline. About one ml of cellular suspension was collected as a post-processing sample and analyzed by flow cytometry analysis to obtain cell count, flow analysis, and viability.

The total cell count processing sample was performed using Hemocytometer. The results indicated that about 5.2 million cells were collected in the post-processing sample in accordance with the invention. Flow cytometry analysis was performed on a FC500 Flow Cytometer. The results indicated that CD117⁺ cells were collected at a concentration of about 0.5% of the cell population. Additionally, the results indicated that CD45 negative cells were collected at a concentration of about 88.4 % of the cell population. The results indicated that CD44+ cells were collected at a concentration of at about 2.1 % of the cell population. The results also indicated that about 97.0% of cells collected were viable as determined by 7AAD.

About 4 million cells were seeded at about 40,000 cells per cm² into a T75 untreated culture flask in Chang's media on day 0. Flasks were incubated in about 5% CO₂ at about 37°C temperature. At passage 1 on day 10, the media was changed. Additional passages occurred on days 14, 16, 19, 22, and 26. On day 26 at passage 5, cells were harvested for CD117 selection according to the selection methods of the present invention.

The harvested cells were labeled with mouse anti-human CD-117 antibodies. About 10 million harvested cells were centrifuged and the supernatant was aspirated. The pellet was suspended in about 100 ul of the working buffer that contained about 5 ug of purified mouse anti-human CD117 monoclonal antibody (IgG₁) with product 104D2 (Santa Cruz). The working buffer available through BD Biosciences contains PBS at about pH of 7.2, bovine serum albumin, EDTA, and about 0.09% Azide. The solution containing the cells and antibodies was incubated for about 20 minutes on ice. The cells were washed with working buffer to remove unbound antibodies, and the solution containing the cells and antibodies were centrifuged at about 300 g for about 10 minutes. After centrifugation, the supernatant was aspirated and discarded, and the pellet was suspended in about 80 ul of working buffer.

The CD117 stem cell-antibody complexes were labeled with goat anti-mouse antibodies capable of binding to the heavy chain and/or the light chain of mouse IgG. Magnetic microbeads are attached to the goat anti-mouse antibodies. The goat anti-mouse IgG antibodies were added and incubated on ice for about 30 minutes. After incubation the cells were washed with about 2 ml of working buffer and centrifuged at about 300 g for about 10 minutes. The supernatant was aspirated and the final cells were suspended in about 500 ul of working buffer.

The cell suspension comprising harvested stem cells and solutions, including working buffer, was kept cold throughout the experiment to prevent non-specific labeling of cells.

An MS Column (Miltenyi Biotec) was prepared by rinsing about 500 ul of working buffer through the column. Minimacs available through Miltenyi Biotec was used for CD117 stem cell selection. The column was placed in the magnetic field of the MACS separator available through Miltenyi Biotec. The cell suspension was added to the column via pipette. The unlabeled cells and working solution flowed through the column and were collected in a sterile tube for phenotyping and cell count analysis. The tube with the collected effluent was labeled as a negative CD117 cell fraction. The column was washed 3 times with working buffer after adding the cells suspension. A sterile tube was placed under the column, which was removed from the magnetic field to allow the positive fraction to pass through the column and into the sterile tube. About one ml of working buffer was added to the column, and a plunger was pushed through the column to release the positive fraction of the cells. The tube with the collected effluent was labeled as a positive CD117 cell fraction.

The positive and negative fractions were analyzed with a hemocytometer to obtain the total number of viable cells.

The positive fraction was cultured in Chang's media. About 900,000 million cells were seeded at about 3,000 cells per cm² into 4 - T25 untreated culture flasks in Chang's media on day 0. Flasks were incubated in about 5% CO₂ at about 37°C temperature. On day 3, the cells went through passage 1 and the media was changed. Additional passages occurred on days 3, 6 and 8. On day 8 at passage 3, about 24 million cells were harvested and about 20.5 million cells were cryopreserved according to the cryopreservation methods of the present invention. The remaining CD117 positively selected cells were seeded into culture and went through several more passages. At passage 5 of the culture of the positively selected cells, the cells exhibited the cell surface markers as listed on Table E

**Table E: M2-02C/M2-03E Flow Cytometry Analysis**

| | POS | POS |
|---|---|---|
| | **M2-02C POS FRAC P4** | **M2-03E POS FRANC P5** |
| | %POS | %POS |
| HLA-I | 99.3 | 95 |
| CD133 | 0 | 0 |
| HLA-II | 1.8 | 35.5 |
| CD9 | 91.7 | 28.5 |
| CD54 | 11.5 | 0 |
| CD45 | 6.4 | 0 |
| CCD10 | 41.9 | 2 |
| CD59 | 100 | 98.6 |
| CD63 | 95.2 | 2 |
| CD34 | 16.8 | 86.2 |
| CD13 | 98.9 | 98.7 |
| CD49e | 92.1 | 93.8 |
| CD49f | 93.4 | 87 |
| CD81 | 99 | 55.6 |
| CD44 | 100 | 98.6 |
| CD117 | 35.3 | 0 |
| CD38 | 0 | 0 |
| CD29 | 100 | 98 |
| CD105 | 100 | 93.3 |
| CD90 | 73 | 98.2 |
| CD166 | 99.1 | 99.4 |
| NANOG | 0 | 0 |
| SSEA3 | 0 | 0 |
| SSEA4 | 12.5 | 0 |
| CD3 | 0 | 0 |
| CD19 | 0 | 0 |
| CD14 | 0 | 0 |
| CD56 | 1.6 | 0.2 |
| CD41 | 98.2 | 98.2 |

The cryopreserved cells were later thawed and analyzed according to the cell culture, flow cytometry methods, karyotype analysis, differentiation methods, and RNA extraction and RT-PCR methods described in R. Gonzalez et al., Pluripotent marker expression and differentiation of human second trimester MSC, Biochem. Biophys, Res. Commun. (2007).

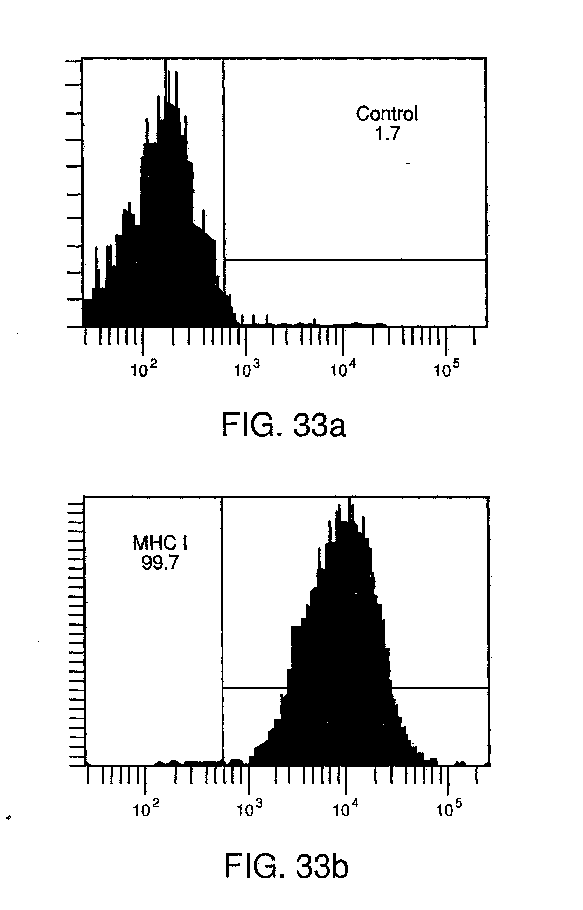

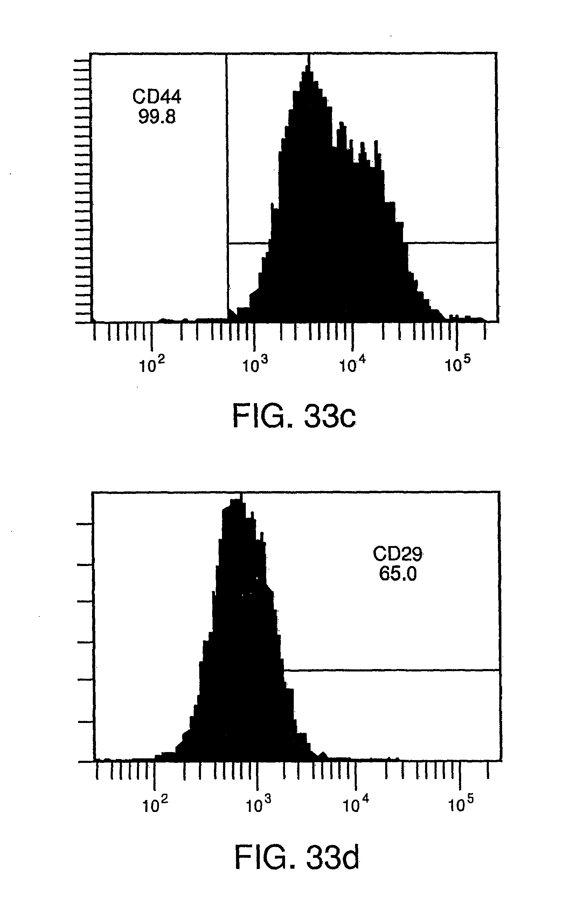

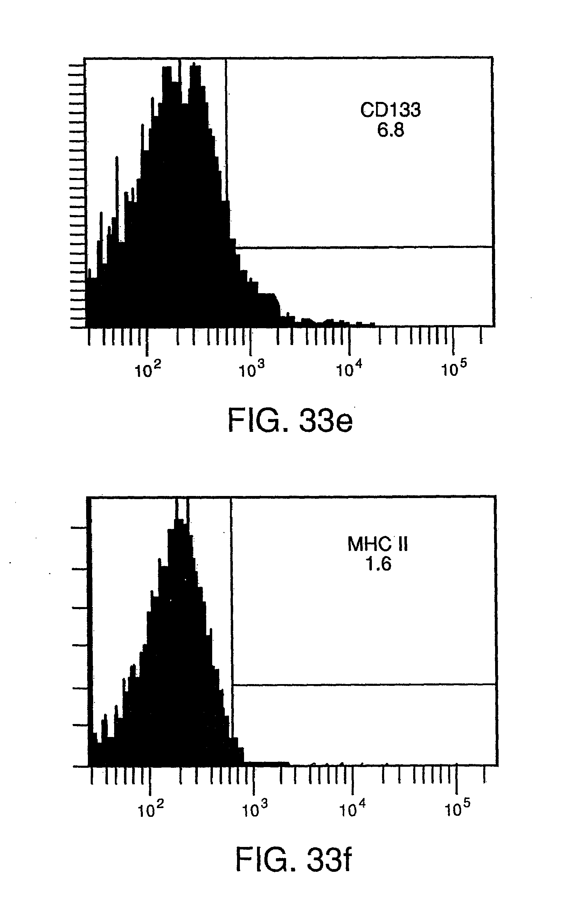

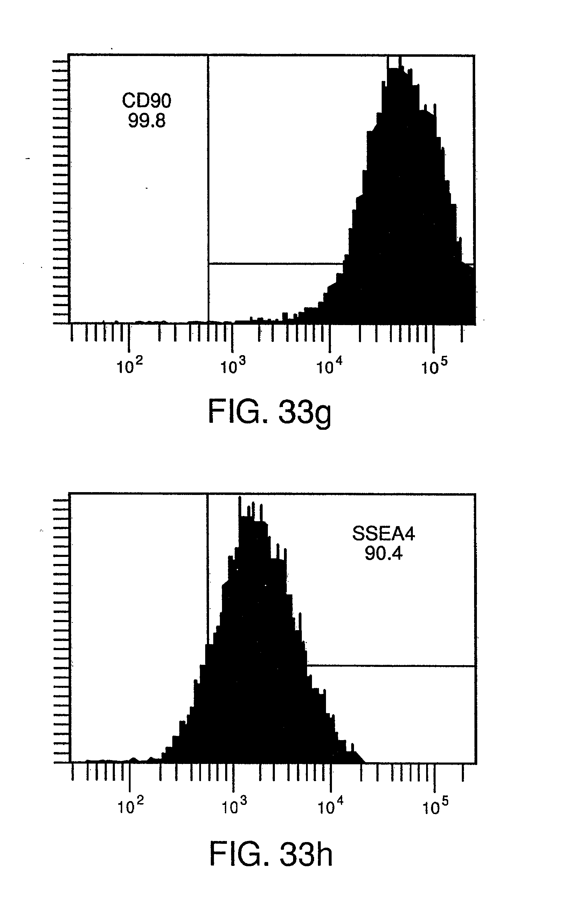

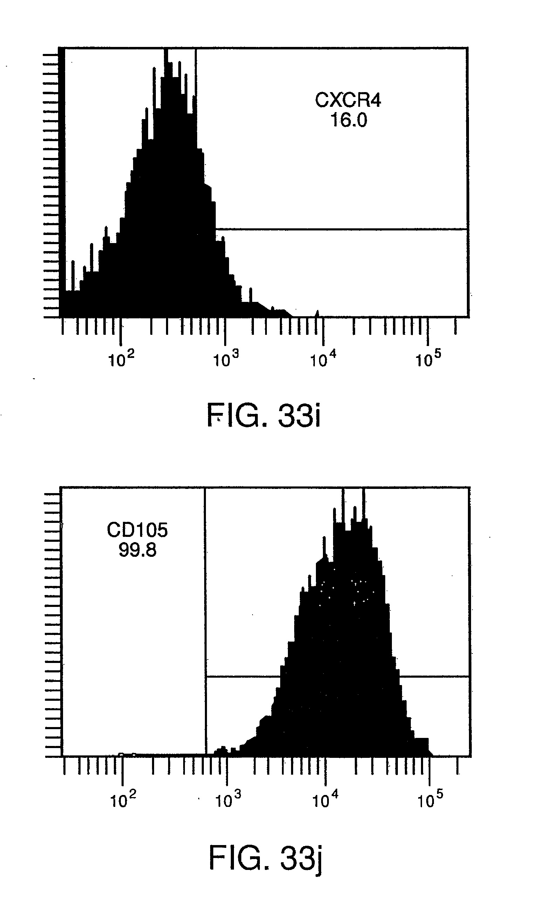

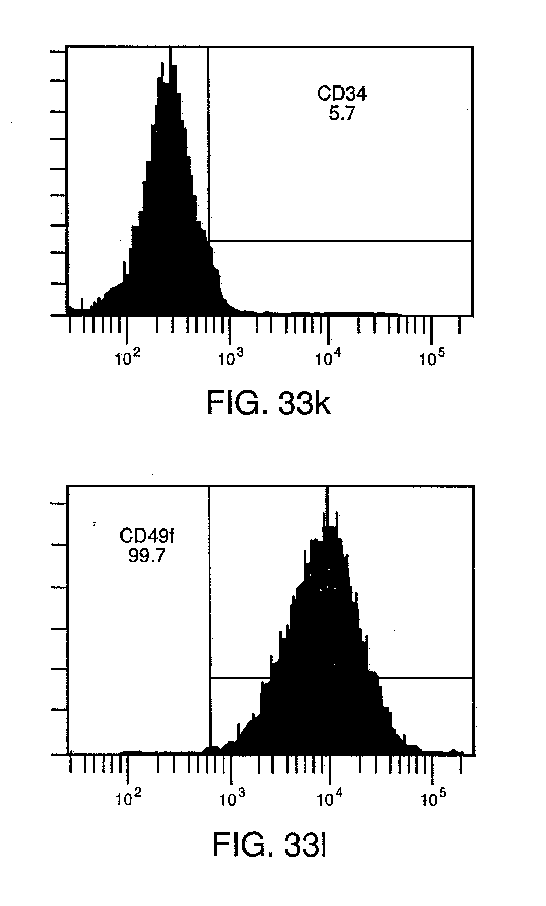

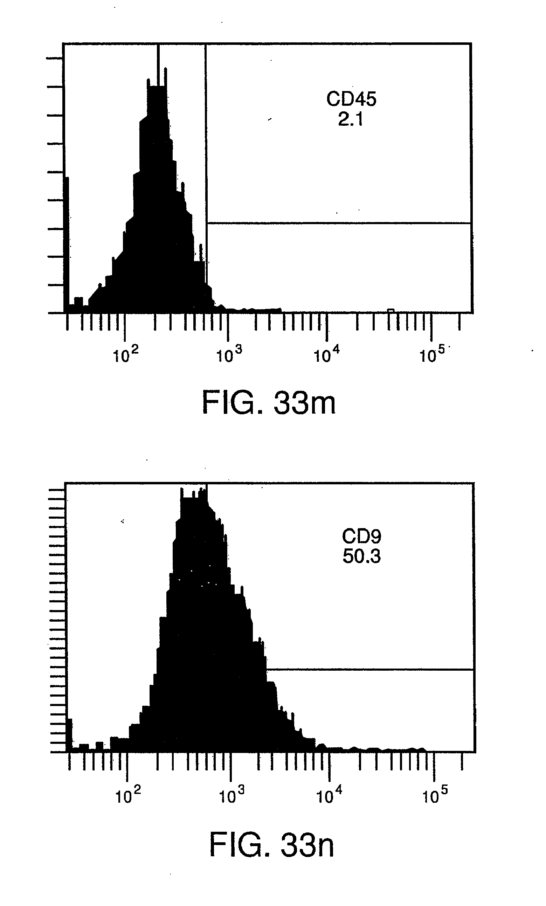

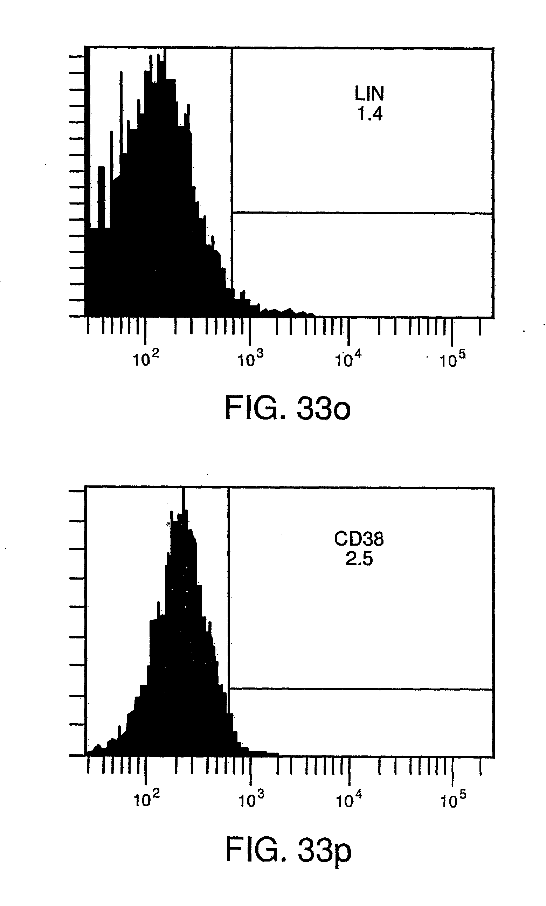

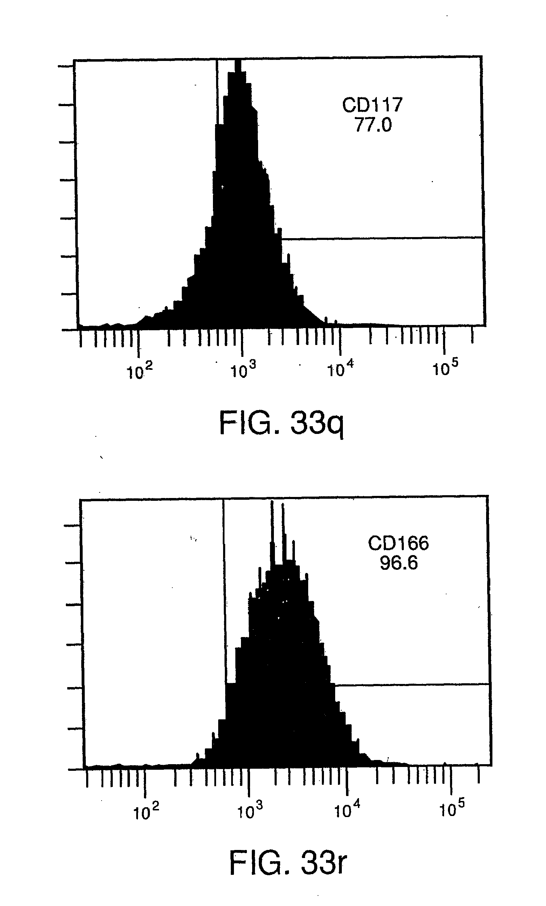

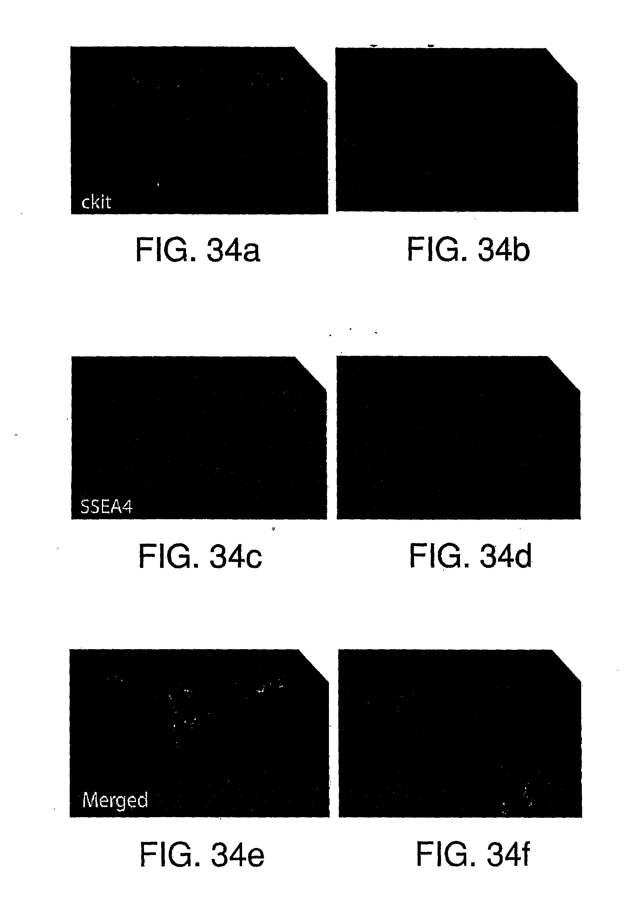

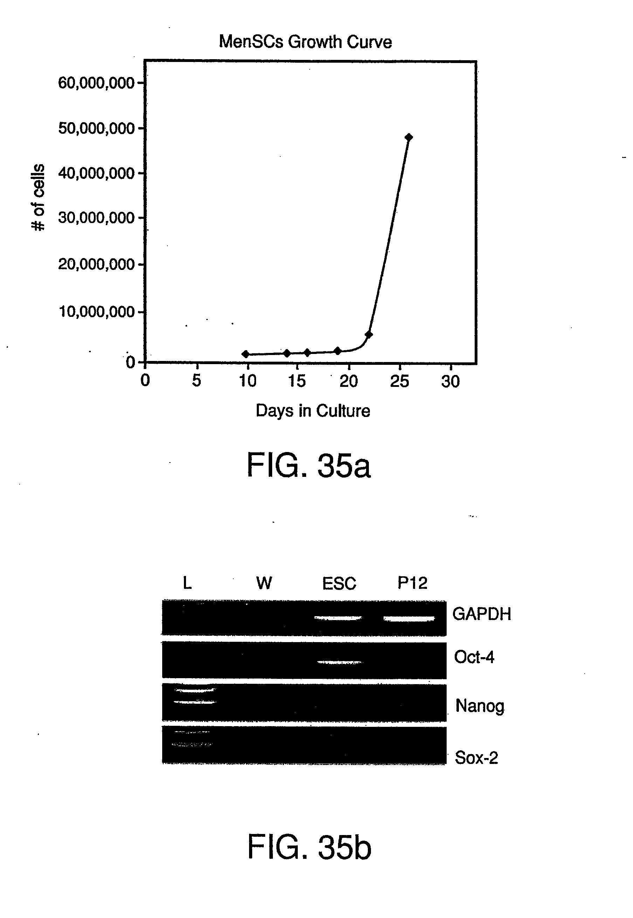

The cryopreserved cells were thawed and cultured for one passage according to the culture techniques of Gonzalez. Cells were analyzed by the flow cytometry methods, the karyotype methods, and the differentiation methods of Gonzalez at the first passage. Flow cytometry results as shown in FIG. 33 indicate that the cells were positive for CD117 and SSEA-4, as shown specifically in FIG. 34, and also CD44, CD105, CD166, CD90, CD49f, MHC I, CD29, and CD9 and demonstrated co-localization on isolated cells. The cells were also positive for CXCR4. The cells were negative for CD38, CD133, CD45, CD34, MHC II, and LIN. As shown in FIG. 35a, the cells doubled at a rate of about 24 to about 36 hours in culture. As also shown in FIG. 35b, the initial cell culture started with 50,000 cells and grew to 48,000,000 by day 26 of culture. RT-PCR analysis showed that the cells express the multipotent marker Oct-4 at passage 12, but not SOX-2 or Nanog as also shown in FIG. 35b.

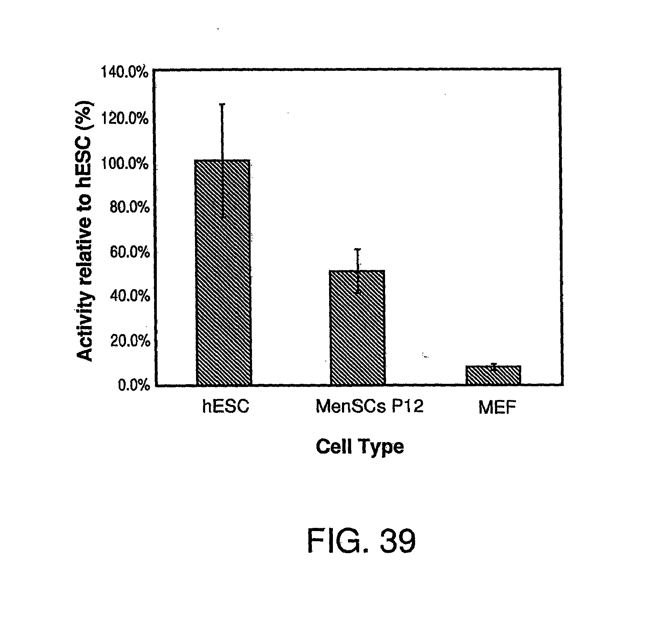

The karyotype analysis according to the methods of Gonzalez indicated that the cells were apparently a normal female karyotype and maintained diploid cells in the absence of chromosomal aberration. The cells show greater than 50% telomerase activity at passage 12 as shown in FIG. 39.

The RT-PCR results are shown in FIG. 35b and illustrate that the cells express at least the pluripotent marker Oct-4.

The results of the differentiation analysis according to the Gonzalez methods show that the cells have the ability to differentiate into neural lineages, cardiogenic lineages, chondrogenic lineages, osteogenic lineages, and adipogenic lineages.

According to the results of the Gonzalez methods for neural lineage differentiation, the cells exhibited the capability to differentiate into neural tissues by expressing the markers 04 and GalC, which are expressed by oligodendroglial cells as shown in FIG. 36. Neural induced cells also expressed Tub3, Map2, and Vimentin, which are expressed by neuronal cells and neural progenitors as shown in FIG. 36. When cultured in media containing FDF for 4 days, and followed by the addition of FGF, PDGF, and EGF for about 7 days, then cultured in FGF and PDGF excluding EGF for 5 days, the cell expressed 04 and GalC, the neuronal marker Map-2, Vimentin, and the astrocyte marker GFAP, as shown in FIG. 36. RT-PCR illustrates that the cells express neural markers at the RNA level including Nestin, NCAM, and Nurr-1. The RT-PCR data shows that the cells have a potential for differentiation into multiple neural phenotypes at a rate of 45-50%.

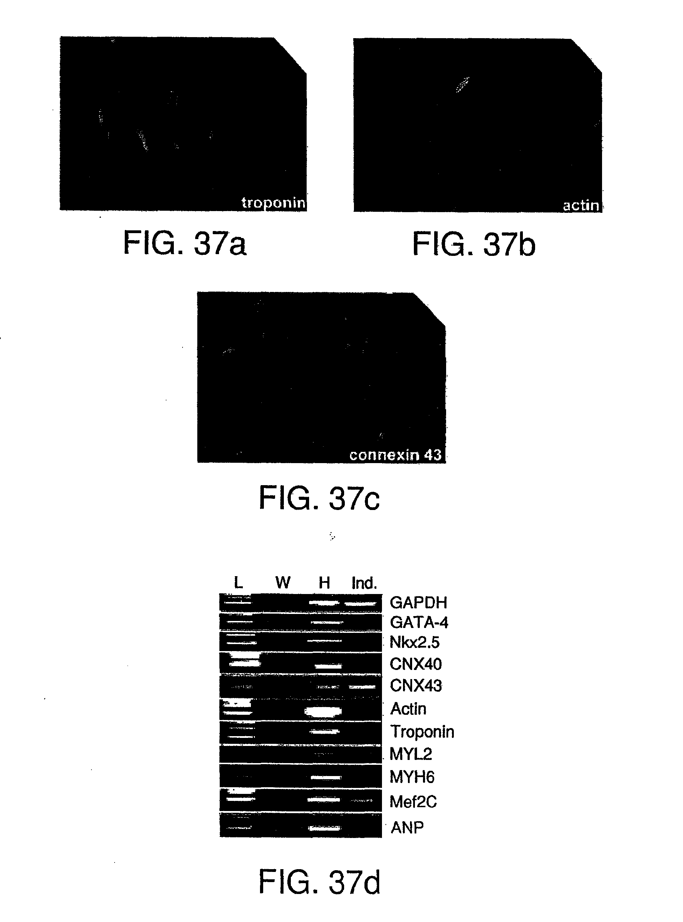

According to the results of the Gonzalez methods for cardiogenic lineage differentiation, the cells exhibited the capability to differentiate into cardiogenic lineages. Cardiac induced cells expressed actin and troponin, which are expressed in cardiac cells as also shown in FIG. 37.

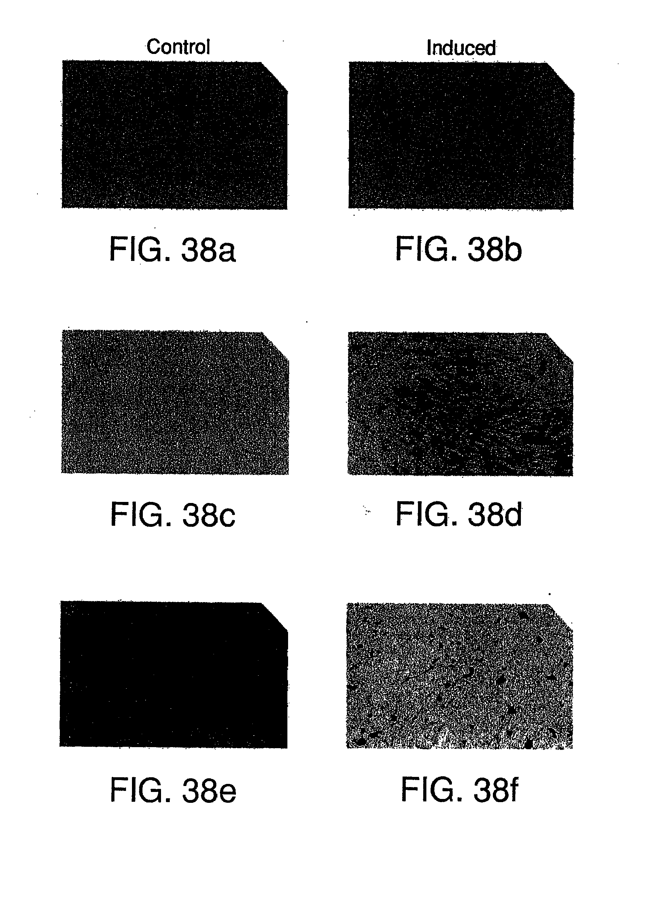

According to the results of the Gonzalez methods for chondrogenic lineage differentiation, the cells exhibited the ability to differentiate into chondrogenic lineages as shown in FIGS. 38c and 38d. Chondrogenic differentiated cells stained positive with alcian blue for sulfated proteoglycans, which are expressed by chondrogenic tissue types.

According to the results of the Gonzalez methods for adipogenic lineage differentiation, the cells exhibited the ability of the cells to differentiate into adipogenic lineages as shown in FIGS. 38e and 38f. Adipogenic differentiated cells stained positive with oil red O for fat vacuoles, which are expressed by adipogenic tissue, and as also shown specifically in FIG. 38f.

According to the results of the Gonzalez methods for osteogenic lineage differentiation, the cells exhibited low staining with alizarin red S for calcium deposits that are typical of osteogenic tissue as shown in FIG. 38a and 38b.

According to the results of the Gonzalez methods for cardiac lineage differentiation, the cells exhibited the ability to differentiate into a cardiac lineage as shown in FIG. 37. The cells stained positive for the cardiac markers actin, troponin, and connexin 43 after differentiation of cells using either 8 uM Aza or 800 uM SNAP as shown in FIG. 37. RT-PCR data demonstrates that the cells express cardiac markers at the RNA level, when the cells are overgrown with cell aggregates. The data shows that the cells express cardiogenic markers at the cellular and molecular level at a rate of about 50 to about 60%.

**Study Examples**

A study was performed to analyze results of alternative methods of collecting menstrual flow samples. The study focused on determining total nucleated cell counts and cell viability of menstrual stem cells collected according to the menstrual flow collection methods using a collection cup and collection media with and, alternatively, without antibiotics. The study also focused on the temperature of menstrual flow samples after arrival at a processing facility between about 4 hours to about 112 hours after collection where menstrual flow samples were transported on ice or, alternatively, at room temperature. There were 161 total menstrual flow samples that were collected and analyzed according to the study. The total menstrual flow samples were treated as follows: (a) 46 menstrual flow samples were collected using a collection device, placed in a collection media comprising antibiotics, and transported on ice, all according to the invention, (b) 34 menstrual flow samples were collected using a collection device, placed in collection media without antibiotics, and shipped at room temperature, all according to the invention, and (c) 81 menstrual flow samples were collected using a collection device, placed in collection media without antibiotics, and transported on ice, all according to the invention.

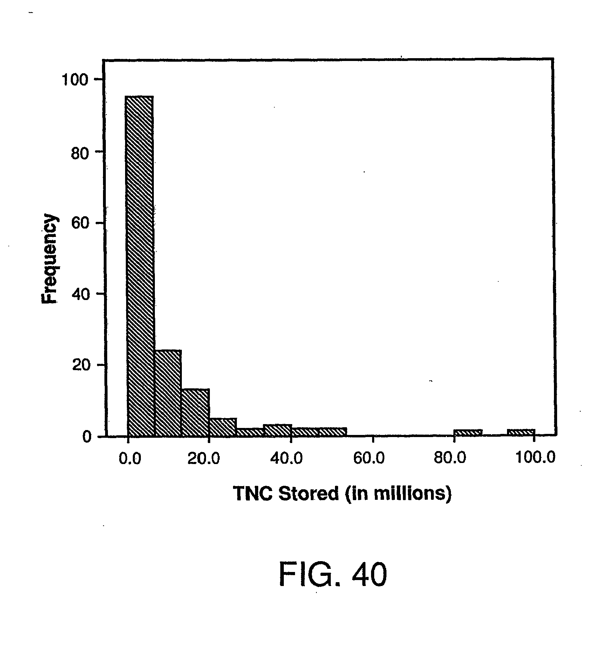

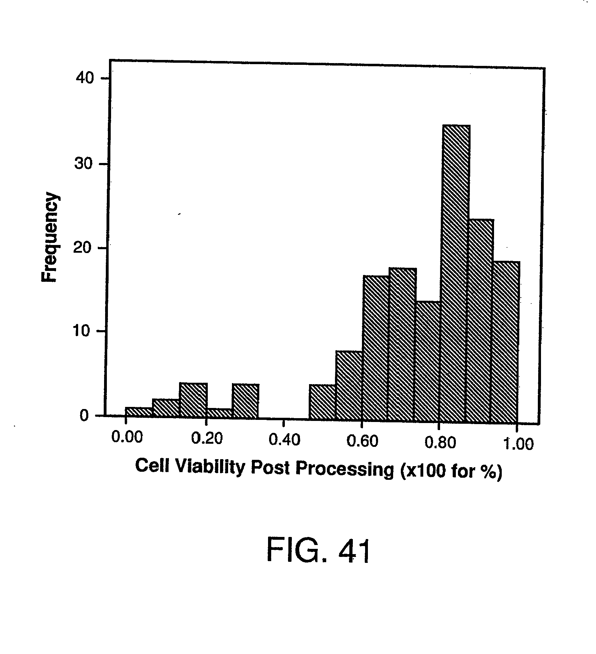

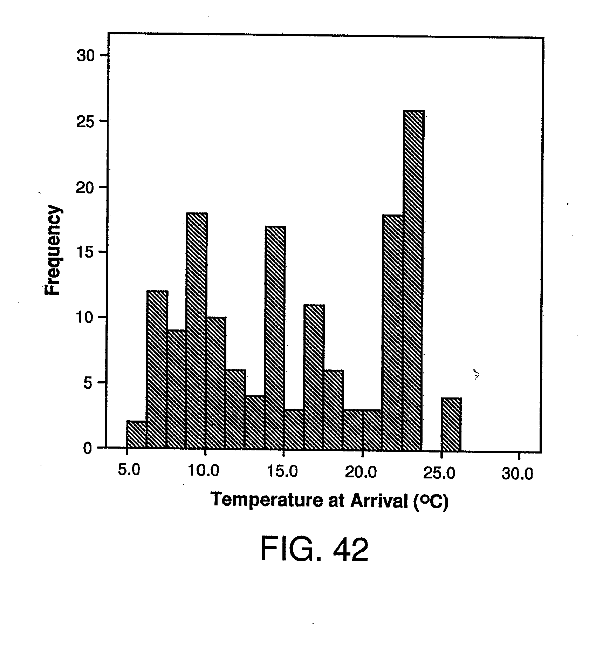

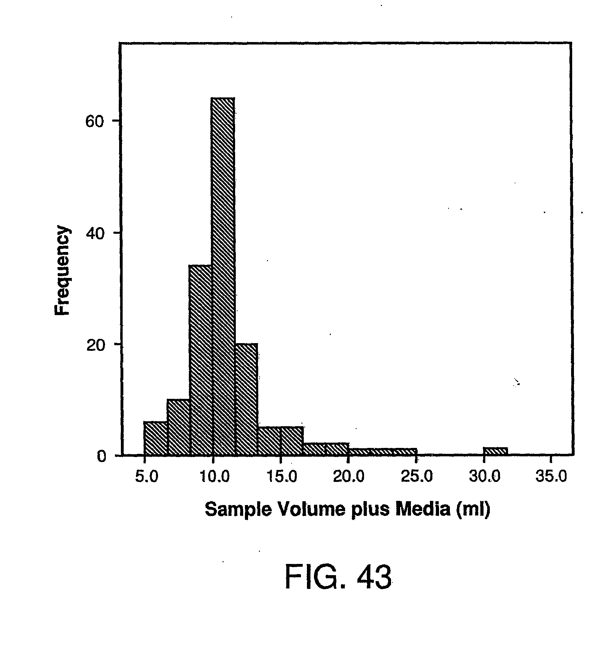

Each sample upon arrival at the processing facility was analyzed to assess temperature and total menstrual flow sample volume at arrival. Total nucleated cell count using a hemocytometer and cell viability by Trypan Blue Exclusion Dye was assessed post-processing. Each sample was subjected to Stage 1 processing and the optional decontamination process and Stage 2 processing prior to assessment of total nucleated cell count and cell viability analysis. The average cell count assessed for all samples was about 7.9 million cells as shown in FIG. 40. The viability range was from about 6% to about 100% per sample. The average cell viability for all samples collected was about 74% as determined by Trypan Blue Dye Exclusion as shown in FIG. 41. The temperature measured at menstrual flow sample arrival ranged from about 5.8°C to about 25°C for all samples analyzed by the study. The average temperature measured at menstrual flow sample arrival was about 15°C as shown in FIG. 42. The total volume of menstrual flow samples in collection media ranged from about 5 ml to about 30 ml. The average volume of menstrual flow sample in collection media was about 11 ml as shown in FIG. 43.

The 46 menstrual flow samples that were collected using a collection device and placed in a collection media comprising antibiotics and transported on ice were also assessed. The menstrual flow samples arrived at the processing facility at temperatures ranging from about 6.6°C to about 16.9°C with an average temperature upon arrival for the group at about 10.5°C. The menstrual flow samples in this group arrived at the processing facility between about 4 hours to about 112 hours after sample collection with the average time of about 47.9 hours for the group. The menstrual flow sample volume comprising menstrual flow in collection media ranged from about 5 ml to about 30 ml with an average of 11.5 ml for the group. Cell viability for the group was assessed on post-processing samples and ranged from about 6% to about 100% viability with an average about 72% viability. Only three samples were assessed having less than 50% viability. Total nucleated cell count for the group was assessed on post-processing samples and ranged from about 0.2 million cells to about 9.24 million cells having an average of about 9.24 million cells. The post-processing menstrual cells were cryopreserved according to the cryopreservation methods of the present invention and then thawed in preparation for additional assessment of cell viability and total nucleated cell counts. In the thawed samples that were cultured, cell viability assessed by Trypan Blue indicated viability at a range of about 60% to about 100%. In the thawed samples, total nucleated cell counts ranged between about 0.1 million cells to about 13.0 million cells with an average of about 3.24 million cells.

The 34 menstrual flow samples that were collected using a collection device, placed in collection media without antibiotics, and transported at room temperature, were also assessed. The menstrual flow samples arrived at the processing facility at temperatures ranging from about 21 °C to about 25°C with an average temperature upon arrival for the group at about 22.8°C. The menstrual flow samples in this group arrived at the processing facility between about 7 hours to about 94 hours after sample collection with the average time of about 37 hours for the group. The menstrual flow sample volume comprising menstrual flow in collection media ranged from about 7.4 ml to about 17.5 ml with an average of 10.8 ml for the group. Cell viability for the group was assessed on post-processing samples and ranged from about 12% to about 100% viability with an average about 80% viability. Total nucleated cell count for the group was assessed on post-processing samples and ranged from about 0.3 million cells to about 50.0 million cells having an average of about 11.6 million cells. The post-processing menstrual cells were cryopreserved according to the cryopreservation methods of the present invention and then thawed in preparation for additional assessment of cell viability and total nucleated cell count. In the thawed samples, cell viability assessed by Trypan Blue indicated viability at a range of about 37% to about 100%. In the thawed samples, total nucleated cell counts ranged between about 0.1 million cells to about 5.9 million cells with an average of about 1.62 million cells.

The 81 menstrual flow samples that were collected using a collection device, placed in collection media without antibiotics, and transported on ice, were also assessed. The menstrual flow samples arrived at the processing facility at temperatures ranging from about 5.8°C to about 25.8°C with an average temperature upon arrival for the group at about 15.5°C. The menstrual flow samples in this group arrived at the processing facility between about 2.5 hours to about 164 hours after sample collection with the average time of about 54.6 hours for the group. The menstrual flow sample volume comprising menstrual flow in collection media ranged from about 5.1 ml to about 21.5 ml with an average of 10.8 ml for the group. Cell viability for the group was assessed on post-processing samples and ranged from about 10% to about 100% viability with an average about 73% viability. Total nucleated cell count for the group was assessed on post-processing samples and ranged from about 0.1 million cells to about 42.0 million cells having an average of about 5.84 million cells. The post-processing menstrual cells were cryopreserved according to the cryopreservation methods of the present invention and then thawed in preparation for additional assessment of cell viability and total nucleated cell counts. In the thawed samples, cell viability assessed by Trypan Blue indicated viability at a range of about 45% to about 100%. In the thawed samples, total nucleated cell counts ranged between about 0.1 million cells to about 48.3 million cells with an average of about 8.45 million cells.

**Menstrual Stem Cell Clones**

Menstrual flow samples were collected from four different donors according to the collection methods of the invention and MSC clones were developed from the menstrual flow samples.

**Menstrual Stem Cell Clones - M2-014-02**

Several different menstrual stem cell clones were obtained according to the practices of the invention. In particular, about 30 ml of a menstrual flow sample, designated and referred to herein as M2-014-02, was collected. The menstrual flow sample comprised menstrual flow and collection media was collected by a donor during a menstrual cycle. A sterilized collection device was cleaned with soap and water and placed within the donor's vagina for time enough to collection a sample of menstrual flow. The collection media comprised DPBS (Mediatech) without calcium, magnesium or phenol red, Penicillin at about 100 units per milliliter, Streptomycin at about 100 micrograms per milliliter, and preservation-free Heparin in a range of about 10 units to about 20 units per milliliter of the DPBS media. The menstrual flow sample and collection media were sealed in the collection tube, packed on ice in a collection container, and transported by shipment to a processing facility.

After about 17 hours from collection, about 30 ml of menstrual flow sample in collection media was subject to processing to isolate and collect menstrual stem cells. The menstrual flow sample arrived at the processing facility at about 10°C. The menstrual flow sample and collection media with antibiotics was disinfected upon arrival at the processing facility, transferred into a clean room, and placed on ice. The collection container was centrifuged at about 2000 rpm for about seven minutes at a temperature of about 18°C and was then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results were negative for aerobic and anaerobic microorganisms.

The remaining supernatant was aspirated and discarded. The pellet was suspended in about 2 ml of DPBS. About one ml of cellular suspension was collected as a post-processing sample and analyzed for cell count, flow analysis, and viability. The total cell count was performed using a Hemocytometer indicated that about 41 million cells were collected in the post-processing sample. The collected cells demonstrated about 75% viability prior to cryopreservation.

The cryopreserved menstrual stem cells were thawed, trypsinized, and cultured. Prior to culture, the post-thaw menstrual stem cells were analyzed. The total cell count on the post-thaw sample indicated that about 7 million menstrual cells were present with about 66% viability.

Prior to culture, the cells are diluted in a 2 step dilution with a 20% Chang's media to about 2.5 cells per ml, about 5.0 cells per ml, about 10.0 cells per ml, and about 15.0 cells per ml. About 0.2 ml of each dilution is added individually to 84 wells in the 96 well plates, the remaining 12 wells are filled with DPBS for humidifying the plate. The about 2.5 cells per ml dilution produces a plate with 0.5 cells per well. The about 5.0 cells per ml dilution produces a plate with 1.0 cells per well. The about 10.0 cells per ml produces a plate with 2 cells per well. The about 15.0 cells per ml dilution produces a plate with 3 cells per well. The wells are checked under an inverted microscope for a single cell. The media was changed out with fresh 20% Chang's media every 4 days to 6 days. The wells with a single cell are allowed to grow to 50% confluency, at this point the cells are trypsinized and cells are seeded in a T-25 flask with 20% Chang's media. Certain cells were removed from the wells for cell culture according to the methods of the invention.

Prior to cell culture, six menstrual stem cell clones were collected from the plates and each clone was analyzed by flow cytometry according to the methods disclosed herein for cell surface characteristics. A summary of the cell surface characteristics of each clone is shown in Table F.

**Table F: Menstrual Stem Cell Clones**

| | **M2-014-02 F9 P4** | **M2-014-02 2.0CW C2 P5** | **M2-014-02 1.0 G6 P4** | **M2.014-02 0.5 F9 P6** | **M2-014-02 3.0 B11 P4** | **M2.014-02 1.0 E2 P5** |
|---|---|---|---|---|---|---|
| | %POS | %POS | %POS | %POS | %POS | %POS |
| HLA-1 | 100 | 99 | 99 | 84.4 | 98.2 | 95.2 |
| CD133 | 0 | 0 | 0 | 0 | 0 | 0 |
| HLA-II | 0 | 20 | 0 | 3.9 | 3.2 | 5.1 |
| CD9 | 50 | 97 | 51 | 79 | 39.9 | 69.6 |
| CD54 | 20 | 20 | 0 | 14.3 | 1.3 | 22.4 |
| CD45 | 10 | 10 | 0 | 30.2 | 0.8 | 19.4 |
| CCD10 | 40 | 15 | 50 | 44.4 | 95.7 | 40.9 |
| CD59 | 98 | 100 | 99 | 80 | 98.9 | 97.4 |
| CD63 | 5 | 5 | 2 | 0 | 38.1 | 0 |
| CD34 | 12 | 12 | 14 | 23 | 10.3 | 808 |
| CD13 | 98 | 96 | 99 | 92.8 | 98.7 | 98.7 |
| CD49e | 94 | 93 | 95 | 71.1 | 51.7 | 55.6 |
| CD49f | 95 | 86 | 95 | 84.7 | 96.8 | 87.9 |
| CD81 | 99 | 95 | 98 | 86.2 | 96.2 | 88.3 |
| CD44 | 100 | 100 | 99 | 79.9 | 99.1 | 97.3 |
| CD117 | 0 | 0 | 0 | 0 | 1.1 | 2.1 |
| CD38 | 0 | 0 | 0 | 0 | 0 | 0 |
| CD29 | 100 | 99 | 100 | 79.9 | 98.9 | 97.8 |
| CD105 | 100 | 96 | 99 | 87.4 | 95.1 | 95.6 |
| CD90 | 17 | 80 | 1 | 0.4 | 0.6 | 15.3 |
| CD166 | 100 | 96 | 100 | 88.5 | 99.4 | 97.6 |
| NANOG | 0 | ND | ND | 0 | 0.2 | 0 |
| SSEA3 | 0 | ND | ND | 0 | 0 | 0 |
| SSEA4 | 8 | ND | ND | 2 | 22.8 | 0 |
| CD3 | 0 | 0 | 0 | 0 | 0 | 0 |
| CD19 | 0 | 0 | 0 | 0 | 0 | 0 |
| CD14 | 5 | 10 | 2 | 0 | | |
| CD56 | 87 | 30 | 94 | 43.5 | 13.2 | 88.8 |
| CD41 | 98 | 92 | 98 | 90.1 | 81.8 | 90.8 |

**Menstrual Stem Cell Clones M2-015-03**

Several different menstrual stem cell clones were obtained according to the practices of the invention. In particular, about 30 ml of a menstrual flow sample, designated and referred to herein as M2-015-03, was collected. The menstrual flow sample comprised menstrual flow and collection media was collected by a donor during a menstrual cycle. A sterilized collection device was cleaned with soap and water and placed within the donor's vagina for time enough to collection a sample of menstrual flow. The collection media comprised Dulbecco's Phosphate Buffer Saline (DPBS) (Mediatech) without calcium, magnesium or phenol red, Penicillin at about 100 units per milliliter, Streptomycin at about 100 micrograms per milliliter, and preservation-free Heparin in a range of about 10 units to about 20 units per milliliter of the DPBS media. The menstrual flow sample and collection media were sealed in the collection tube, packed on ice in a collection container, and transported by shipment to a processing facility.

After about 6 hours from collection, about 23 ml of menstrual flow sample in collection media was subject to processing to isolate and collect menstrual stem cells. The menstrual flow sample arrived at the processing facility at about 10°C. The menstrual flow sample and collection media with antibiotics was disinfected upon arrival at the processing facility, transferred into a clean room, and placed on ice. The collection container was centrifuged at about 2000 rpm for about seven minutes at a temperature of about 18°C and was then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results were negative for aerobic and anaerobic microorganisms.

The remaining supernatant was aspirated and discarded. The pellet was suspended in about 2 ml of DPBS. About one ml of cellular suspension was collected as a post-processing sample and analyzed for cell count, flow analysis, and viability. The total cell count performed using a Hemocytometer indicated that about 1 million cells were collected in the post-processing sample. The collected cells demonstrated about 18% viability prior to cryopreservation.

The cryopreserved menstrual stem cells were thawed, trypsinized, and cultured. Prior to culture, the post-thaw menstrual stem cells were analyzed. The total cell count on the post-thaw sample indicated that about 0.3 million menstrual cells were present with about 60% viability.

Prior to culture, the cells are diluted in a 2 step dilution processed with a 20% Chang's media to about 2.5 cells per ml, about 5.0 cells per ml, about 10.0 cells per ml, and about 15.0 cells per ml. About 0.2 ml of each dilution is added individually to 84 wells in the 96 well plates, the remaining 12 wells are filled with DPBS for humidifying the plate. The about 2.5 cells per ml dilution produces a plate with 0.5 cells per well. The about 5.0 cells per ml dilution produces a plate with 1.0 cells per well. The about 10.0 cells per ml produces a plate with 2 cells per well. The about 15.0 cells per ml dilution produces a plate with 3 cells per well. The wells are checked under an inverted microscope for a single cell. The media was changed out with fresh 20% Chang's media every 4-6 days. The wells with a single cell are allowed to grow to 50% confluency, at this point the cells are trypsinized and cells are seeded in a T-25 flask with 20% Chang's media. Certain cells were removed from the wells for cell culture according to the methods of the invention.

At passage 2, two menstrual stem cell clones were collected from the plates and cultured. Each clone was analyzed by flow cytometry according to the methods disclosed herein for cell surface characteristics. A summary of the cell surface characteristics of each clone is shown in Table G.

**Table G: Menstrual Stem Cell Clones**

| | **M2-015-03 ABi P2 B10 P6** | **M2-015-03 ABI P2 2.0 82** |
|---|---|---|
| | %POS | %POS |
| HLA-I | 95.2 | 100 |
| CD133 | 0 | 0 |
| **HLA-II** | 46.8 | 0 |
| CD9 | 56.4 | 70 |
| CD54 | 47.8 | 2 |
| CD45 | 22.4 | 2 |
| CCD10 | 10.6 | 20 |
| CD59 | 97.4 | 100 |
| CD63 | 0 | 1 |
| CD34 | 68.1 | 0 |
| CD13 | 96.4 | 100 |
| CD49e | 58.7 | 90 |
| CD49f | 46.6 | 98 |
| CD81 | 94.7 | 99 |
| CD44 | 97.4 | 100 |
| CD117 | 0 | 0 |
| CD38 | 0 | 0 |
| CD29 | 97.1 | 100 |
| CD105 | 96.7 | 100 |
| CD90 | 1 | 10 |
| CD166 | 97.9 | 100 |
| NANOG | 0 | 0 |
| SSEA3 | 0 | 0 |
| SSEA4 | 9.8 | 35 |
| CD3 | 0 | 0 |
| CD19 | 0 | 0 |
| CD14 | 0 | 0 |
| CD56 | 5.7 | 90 |
| CD41 | 95.2 | 98 |

**Menstrual Stem Cell Clone - M2-017-03**

Several different menstrual stem cell clones were obtained according to the practices of the invention. In particular, about 30 ml of a menstrual flow sample, designated and referred to herein as M2-015-03, was collected. The menstrual flow sample comprised menstrual flow and collection media was collected by a donor during a menstrual cycle. A sterilized collection device was cleaned with soap and water and placed within the donor's vagina for time enough to collection a sample of menstrual flow. The collection media comprised DPBS (Mediatech) without calcium, magnesium or phenol red, and preservation-free Heparin in a range of about 10 units to about 20 units per milliliter of the DPBS media. The menstrual flow sample and collection media were sealed in the collection tube and transported without ice by shipment to a processing facility.

After about 28 hours from collection, about 8 ml of menstrual flow sample in collection media was subject to processing to isolate and collect menstrual stem cells. The menstrual flow sample arrived at the processing facility at about 23°C. The menstrual flow sample and collection media with antibiotics was disinfected upon arrival at the processing facility, transferred into a clean room, and placed on ice. The collection container was centrifuged at about 2000 rpm for about seven minutes at a temperature of about 18°C and was then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results indicate contamination by *E. faecalis.*

The remaining supernatant was aspirated and discarded. The pellet was suspended in about 2 ml of DPBS. About 1 ml of cellular suspension was collected as a post-processing sample and analyzed for cell count, flow analysis, and viability. The total cell count performed using a Hemocytometer indicated that about 0.8 million cells were collected in the post-processing sample. The collected cells demonstrated about 87% viability before cryopreservation.

The cryopreserved menstrual stem cells were thawed, trypsinized, and cultured. Prior to culture, the post-thaw menstrual stem cells were analyzed. The total cell count on the post-thaw sample indicated that about 0.2 million menstrual cells were present with about 63% viability.

Prior to culture, the cells are diluted in a 2 step dilution with a 20% Chang's media to about 2.5 cells per ml, about 5.0 cells per ml, about 10.0 cells per ml, and about 15.0 cells per ml. About 0.2 ml of each dilution is added individually to 84 wells in the 96 well plates, the remaining 12 wells are filled with DPBS for humidifying the plate. The about 2.5 cells per ml dilution produces a plate with 0.5 cells per well. The about 5.0 cells per ml dilution produces a plate with 1.0 cells per well. The about 10.0 cells per ml produces a plate with 2 cells per well. The about 15.0 cells per ml dilution produces a plate with 3 cells per well. The wells are checked under an inverted microscope for a single cell. The media was changed out with fresh 20% Chang's media every 4 day to 6 days. The wells with a single cell are allowed to grow to 50% confluency, at this point the cells are trypsinized and cells are seeded in a T-25 flask with 20% Chang's media. Certain cells were removed from the wells for cell culture according to the methods of the invention.

During culture up to the first passage, Amphotericin B was included with the Penn/Strep with the culture media. At passage 3, each clone in culture was analyzed by flow cytometry according to the methods disclosed herein for cell surface characteristics. A summary of the cell surface characteristics of each clone is shown in Table H.

**Table H: Menstrual Stem Cell Clones**

| | **M2-017-03-G2 P3** | **M2-017-03 0.5 82** | **M2-017-03 P3 0.5 E1 P4** | **M2-017-03 P3 0.5 82 P5** |
|---|---|---|---|---|
| | %POS | %POS | %POS | %POS |
| HLA-1 | 99 | 93.8 | 99 | 99.1 |
| CD133 | 0 | 0 | 0 | 0 |
| HLA-II | 24 | 0.2 | 1.1 | 1 |
| CD9 | 98 | 73.3 | 61.7 | 64 |
| CD54 | 2 | 27.6 | 8.8 | 9.3 |
| CD45 | 16 | 8.2 | 5.9 | 1 |
| CCD10 | 98 | 11.3 | 10.7 | 9 |
| CD59 | 100 | 94.7 | 99.3 | 99.5 |
| CD63 | 0 | 1.5 | 0 | 1.4 |
| CD34 | 44 | 3 | 1.9 | 15.4 |
| CD13 | 100 | 99.1 | 99 | 99.2 |
| CD49e | 100 | 89.8 | 47.6 | 68.7 |
| CD49f | 100 | 94.5 | 97.8 | 98.8 |
| CD81 | 100 | 97.2 | 96 | 99.2 |
| CD44 | 99 | 94.1 | 99.4 | 99.6 |
| CD117 | 0 | 0 | 0 | 0 |
| CD38 | 0 | 0 | 0 | 0 |
| CD29 | 100 | 94.6 | 99.2 | 99.5 |
| CD105 | 97 | 98.3 | 99 | 99.4 |
| CD90 | 99 | 95 | 98.9 | 91.1 |
| CD166 | 99 | 99.3 | 99.3 | 99.6 |
| NANOG | ND | 0 | 0 | 0.3 |
| SSEA3 | 0 | 0 | 0 | 0 |
| SSEA4 | 20 | 38.6 | 10.4 | 42.4 |
| CD3 | 0 | 0 | 0 | 0 |
| CD19 | 0 | 0 | 0 | 0 |
| CD14 | 20 | 0 | 0 | |
| CD56 | 0 | 0 | 0 | 0 |
| CD41 | 99 | 97.1 | 98.4 | 97.6 |

**Menstrual Stem Cell Clones - M2-54-02**

Several different menstrual stem cell clones were obtained according to the practices of the invention. In particular, about 12 ml of a menstrual flow sample, designated and referred to herein as M2-54-02, was collected. The menstrual flow sample comprised menstrual flow and collection media was collected by a donor during a menstrual cycle. A sterilized collection device was cleaned with soap and water and placed within the donor's vagina for time enough to collection a sample of menstrual flow. The collection media comprised DPBS (Mediatech) without calcium, magnesium or phenol red, and preservation-free Heparin in a range of about 10 units to about 20 units per milliliter of the DPBS media. The menstrual flow sample and collection media were sealed in the collection tube, packed on ice in a collection container, and transported by shipment to a processing facility.

After about 73 hours from collection, about 12 ml of menstrual flow sample in collection media was subject to processing to isolate and collect menstrual stem cells. The menstrual flow sample arrived at the processing facility at about 20°C. The menstrual flow sample and collection media with antibiotics was disinfected upon arrival at the processing facility, transferred into a clean room, and placed on ice. The collection container was centrifuged at about 2000 rpm for about seven minutes at a temperature of about 18°C and was then disinfected. About a 5 ml sample of supernatant was aspirated and used to inoculate two BacT/ALERT blood culture bottles, about 4 ml into an aerobic culture bottle and about 1 ml into an anaerobic culture bottle. The culture bottles were incubated at about 37°C in a BacT/ALERT system for about fourteen days. The results indicated contamination by *S. epidermidis.*

The remaining supernatant was aspirated and discarded. The pellet was suspended in about 2 ml of DPBS. About one ml of cellular suspension was collected as a post-processing sample and analyzed for cell count, flow analysis, and viability. The total cell count performed using a Hemocytometer indicated that about 0.8 million cells were collected in the post-processing sample. The collected cells demonstrated about 72% viability prior to cryopreservation.

The cryopreserved menstrual stem cells were thawed and trypsinized and cultured. Prior to culture, the post-thaw menstrual stem cells were analyzed. The total cell count on the post-thaw sample indicated that about 21 million menstrual cells were present with about 92% viability.

Prior to culture, the cells are diluted in a 2 step dilution with a 20% Chang's media to about 2.5 cells per ml, about 5.0 cells per ml, about 10.0 cells per ml, and about 15.0 cells per ml. About 0.2 ml of each dilution is added individually to 84 wells in the 96 well plates, the remaining 12 wells are filled with DPBS for humidifying the plate. The about 2.5 cells per ml dilution produces a plate with 0.5 cells per well. The about 5.0 cells per ml dilution produces a plate with 1.0 cells per well. The about 10.0 cells per ml produces a plate with 2 cells per well. The about 15.0 cells per ml dilution produces a plate with 3 cells per well. The wells are checked under an inverted microscope for a single cell. The media was changed out with fresh 20% Chang's media every 4-6 days. The wells with a single cell are allowed to grow to 50% confluency, at this point the cells are trypsinized and cells are seeded in a T-25 flask with 20% Chang's media. Certain cells were removed from the wells for cell culture according to the methods of the invention.

Prior to culture, one menstrual stem cell clone was collected from culture and analyzed by flow cytometry according to the methods disclosed herein for cell surface characteristics. A summary of the cell surface characteristics of each clone is shown in Table I.

**Table I: Menstrual Stem Cell Clone**

| | **M2-054-02 H9 P5** |
|---|---|
| | %POS |
| HLA-I | 99 |
| CD133 | 0 |
| HLA-II | 8 |
| CD9 | 98 |
| CD54 | 10 |
| CD45 | 45 |
| CCD10 | 80 |
| CD59 | 99 |
| CD63 | 0 |
| CD34 | 41 |
| CD13 | 99 |
| CD49e | 95 |
| CD49f | 91 |
| CD81 | 98 |
| CD44 | 99 |
| CD117 | 0 |
| CD38 | 0 |
| CD29 | 99 |
| CD105 | 70 |
| CD90 | 7 |
| CD166 | 99 |
| NANOG | ND |
| SSEA3 | 0 |
| SSEA4 | 2 |
| CD3 | 0 |
| CD19 | 20 |
| CD14 | 40 |
| CD56 | 96 |
| CD41 | 94 |

**Therapeutic Uses**

The menstrual stem cells of the present invention may be prepared by combining the cells with a suitable cell media or pharmaceutically acceptable excipient. For example, the menstrual stem cells of the invention may be prepared for infusion or transplant according to GMP (Good Manufacturing Practices). Suitable media or diluents may include tissue culture media (including proper testing and results documented on a certificate of analysis), such as, HBSS, IMDM, RPMI, Alpha MEM, M199, and DMEM, which may require HEPES buffer to maintain an appropriate pH to sustain the menstrual stem cells. Protein may be added at minimum of about 0.2%, but may be added as high as about 30%. Protein used may also include Human Serum Albumin, Human Plasma Protein Fraction, Fetal Calf Serum (if using animal products they must include proper safety testing as required by the United States Food and Drug Administration), or Bovine Serum Albumin. The media may require an anticoagulant if the cells are at risk of coagulation, clumping, or clot. Anticoagulants may include Heparin (preservative free is preferred as preservative may potentially affect the stem cell growth and proliferative capability of the cells), Acid Citrate Dextrose, Citrate Phosphate Dextrose, or any suitable anticoagulant that is safe to combine with viable cells may be used. Cells may be shipped in the cell media they are expanded in as long as it is safe for infusion or transplant.

Cells may be shipped in a syringe for a short distance, for example, if the product is being prepared in the hospital and also administered in the hospital. Otherwise products may be transported in sterile bags, which may or may not include gas exchange. Products shipped short distance may also be transported in any other type of sterile container that the cells will tolerate and remain viable in for the short distance, which may include a sterile conical tube, or a sterile flat bottom container, which may be round or square. If the product is being shipped long distance more than 4 hours it may be sent in a bag that allows for gas exchange. The bag may also be shipped in a configuration that allows for air exchange. The product bag may be placed on a rack that will allow for proper gas exchange in the container it is being shipped in. The container including the cells may be optimized for a suitable temperature to maintain the menstrual stem cells. If the cells require a cool environment, ice packs or other cooling device may be used. If the cells require room temperature at about 20°C to about 24°C, then a gel pack may be required to maintain this temperature.

Once the cells arrive at the facility for infusion or transplant, the cells may be placed in a monitored temperature environment. Cells that will be stored for use at a later date may be shipped cryopreserved in a dry shipper to maintain temperatures less than or equal to -150°C. Cells shipped cryopreserved may be sent with thaw and infusion instructions and potentially the media required to maintain an optimally viable cell product for therapeutic use.

A suitable transport mechanism should be used to transport the product to the infusion site. Any number of different types of transport services may be used, such as, for example, a medical courier service, a courier service, or a company such as UPS, DHL, or FedEx. The menstrual stem cells in media have a greater chance of sate transportation when the courier understands the products needs such as the fact that a Dry Shipper must remain upright for the duration of travel. If the shipper is placed on its side it, may quickly loss the charge of liquid nitrogen and fail to hold the temperature it was validated for.

**Cosmeceutical Applications**

The term cosmeceutical is used in reference to a formulation or composition comprising (a) at least one biologically active ingredient that have an effect on the user of the product, and (b) at least one cosmeceutically-acceptable carrier. The biologically active ingredient may be, for example, MSCs collected in accordance with the practice of this invention, or growth factors or other compositions, chemicals, or proteins produced by MSCs, genetically-engineered MSCs, or differentiated MSCs of the present invention. By way of further example, MSCs, differentiated MSCs, or genetically-engineered MSCs may be grown in culture to produced a desired cellular product, such as, for example, a growth factor or other biologically active ingredient capable of use as a cosmeceutical. The growth factor or other biologically active ingredient may be collected from cell culture media or other media that the MSCs, differentiated MSCs, or genetically-engineered MSCs are grown. The growth factor or other biologically active ingredient may be collected from cell culture media or other media by way of centrifugation steps to collected the desired ingredient in a supernatant, which may be later processed to purify or concentrated the ingredient.

Cosmeceuticals may be viewed as cosmetics that, in certain applications and under appropriate conditions, may provide medicinal or drug-like benefits. In certain applications, for example, cosmeceuticals may affect the underlying structure of the skin, decrease wrinkle depth, or reverse or ameliorate the effect of photooxidation or aging on the skin. Cosmeceuticals may be particularly useful as skin care products, hair care products, tissue or organ modification or augmentation, and sun care products. In certain embodiments, cosmeceutical compositions comprise delivery systems including at least one of liposomes, cyclodextrins, polymer systems, or hyaluronic acid or related compounds. Cosmeceutical compositions comprise cosmeeeutically-acceptable carriers. A pharmaceutically-acceptable carrier or formulation that is suitable for topical applications will typically also be a cosmeceutically-acceptable carrier or formulation.

A topical cosmetic or cosmeceutical ointment, lotion, or gel composition typically contains a concentration of active ingredients comprising conditioned media or extracts thereof, from about 1 to 99%, about 5 to 95%, about 20 to 75%, or about 5 to 20%, in a cosmetically-acceptable carrier or a cosmeceutically-acceptable carrier, such as a pharmaceutical cream base, an oil-in-water emulsion, a water-in-oil emulsion, a gel, or the like. Various cosmetic and cosmeceutical compositions for topical use include drops, tinctures, lotions, creams, salves, serums, solutions, and ointments containing conditioned media or extracts, and an appropriate carrier. The optimal percentage of the conditioned media or extract in each composition varies according to the composition's formulation and the therapeutic effect desired.

The cosmoceuticals may comprise any of a number of cosmetically-, cosmeceutically, or pharmaceutically-acceptable formulations, depending on the type of product, the nature of the composition, the location of composition's use, the desired effect, and the like. While proprietary formulations are common in the formulation arts, formulators will be able to determine or readily select appropriate formulations for specific applications without undue experimentation.

Appropriate carriers of the inventive compositions typically will contain ingredients, such as those typically found in the cosmetic and cosmeceutical fields: oils, waxes or other standard fatty substances, or conventional gelling agents and/or thickeners; emulsifiers; moisturizing agents; emollients; sunscreens; hydrophilic or lipophilic active agents, such as ceramides; agents for combatting free radicals; bactericides; sequestering agents; preservatives; basifying or acidifying agents; fragrances; surfactants; fillers; natural products or extracts of natural product, such as aloe or green tea extract; vitamins; or coloring materials. The amounts of these various ingredients will vary depending on the use of the composition and the cosmetic or cosmeceutical effect desired.

Discussions of cosmetic- and cosmeceutically-acceptable ingredients and formulations may be found in, among other places, FDA Cosmetics Handbook, U.S. Food and Drug Administration; Handbook of Cosmetic and Personal Care Additives, Ash and Ash, compilers, 1994, Chemical Publishing, New York, N.Y.; Bennett's Cosmetic Formulary, 1993, Chemical Publishing Co.; Harry's Cosmeticology, 7.sup.th ed., Wilkinson & Moore, 1982 and 8.sup.th ed., Rieger, 2000, Chemical Publishing; Cosmetic Bench Reference-2001, Allerud Publishing Corp.; CTFA Compendium of Cosmetic Ingredient Composition, Nikitakis and McEwen, eds., 1990, Cosmetic, Toiletry, and Fragrance Association, Washington, D.C., Surfactant Encyclopedia, 2.sup.nd revised edition, Rieger, 1996, Allured Publishing; The Chemistry and Manufacture of Cosmetics, 2.sup.nd ed., De Navarre, Van Nostrand, Princeton, N.J.; Encyclopedia of Common Natural Ingredients Used in Food, Drugs, and Cosmetics, Leung, 1996, John Wiley; A Consumer's Dictionary of Cosmetic Ingredients, 5.sup.th ed., Winter, 1999, Three Rivers Press, New York, N.Y.; Cosmeceuticals: Active Skin Treatment, 1998, Allured Publishing; Handbook of Cosmetic Science and Technology, lCnowlton and Pearce, 1993, Elsevier Advanced Technology, Oxford, UK; Personal-Care Formulas, 1997, Allured Publishing; Beginning Cosmetic Chemistry, Scheuller and Romanowski, 1999, Allured Publishing; and Skin Permeation: Fundamentals and Application, Zatz, 1993, Allured Publishing. Discussions of pharmaceutically-acceptable ingredients and formulations may be found in, among other places, Remington's Pharmaceutical Sciences, 18.sup.th ed., Gennaro, ed., 1990, Mack Publishing.

**Definition**

As used herein, the following terms shall have the definitions set forth below, unless the context in which such term is used suggests otherwise.

DMEM means Dulbecco's Minimal Essential Medium.

DMSO means Dimethyl sulfoxide.

DNAse means Deoxyribonuclease used to break down DNA found after non-viable cells have lysed.

DPBS means Dulbecco's Phosphate Buffered Saline.

HBSS means Hank's balanced salt solution.

Heparin is a glycosaminoglycan having anticoagulant properties.

HSA means Human Serum Albumin which is an abundant plasma protein that can act as a transporter protein.

LSM means Lymphocyte Separation Media used to perform a density gradient cell separation.

µg means microgram.

µl, µL, ul and uL are used synonymously to mean microliter.

ml and mL are used synonymously to mean milliliter.

X means multiply, i.e., by concentration or dilution.

Other suitable replacement reagents, products, and manufacturers may be used in place of the specific reagents, products, and manufacturers referenced herein.

Modifications can be made to the embodiments described above without departing from the broad inventive concept thereof. Having described the preferred embodiments of the invention, additional embodiments, adaptations, variations, modifications, and comparable arrangements will be apparent to those skilled in the art. These and other embodiments will be understood to be within the scope of the appended claims.

## Claims

1. A population of viable multipotent cells prepared by isolating cells of the buffy coat component from the other components of menses obtained from a woman, and selecting for a population of cells expressing CD9, CD10, CD13, CD29, CD44, CD49e, CD49f, CD59, CD81, CD105, CD166, and HLA class I antigens.

2. The population of claim 1, wherein cells of the buffy coat component are isolated from the other components by a process that includes centrifugation, density-gradient centrifugation, filtration or sedimentation; and wherein the centrifugation, density-gradient centrifugation, filtration or sedimentation is optionally preformed using a medium sufficient to separate or isolate cells of the buffy coat component from the menses.

3. The population of claim 1, wherein the preparation includes combining cells of the buffy coat component with a decontamination solution comprising an antibiotic in an amount and for a period of time sufficient to substantially eliminate bacteriological contamination from the combination, wherein the preparation optionally further comprises subsequently substantially removing the antibiotic from the population.

4. The population of claim 1, wherein the cells of the buffy coat component are isolated from the other components by a process that includes starch sedimentation; and/or
wherein the preparation includes a cryopreservation step, wherein cells of the buffy coat component are combined with a cryopreservation medium and maintained under cryogenic storage condition, either before or after separating cells of the buffy coat component from the other components; and/or
wherein the preparation includes selecting for exclusion from the population cells which express on their surface a marker selected from the group consisting of CD3 and HLA class II antigens; and/or
wherein the preparation includes culturing cells of the buffy coat component in a cell culture medium for a period of time and under conditions sufficient to induce proliferation thereof.

5. The population of claim 1, wherein the preparation includes selecting for inclusion in the population cells which express CD117 on their surface, and wherein the preparation optionally further includes contacting cells of the buffy coat component with an antibody that specifically binds with CD117 and selecting for inclusion in the population cells having the antibody bound therewith.

6. The population of claim 5, wherein the antibody is linked with a fluorescent label and wherein the preparation includes a flow cytometry process for selecting fluorescently-labeled cells for inclusion in the population; and/or
wherein the antibody is linked with a magnetic label and wherein the preparation includes an magnetic separation process for selecting magnetically-labeled cells for inclusion in the population.

7. The population of claim 4, wherein the preparation includes passaging the cultured cells at least twice; and/or
wherein the preparation includes selecting cultured cells which express CD117 on their surface prior to further culturing of selected cells.

8. A population of viable multipotent cells prepared by:
(A)
(i) isolating cells of the buffy coat component from the other components of menses obtained from a woman by a process including centrifugation, density-gradient centrifugation, filtration or sedimentation using a medium sufficient to separate or isolate the buffy coat component from the menses;
(ii) combining cells of the buffy coat component with a decontamination solution comprising an antibiotic for a period of time sufficient to substantially eliminate bacteriological contamination from the combination; and
(iii) isolating cells which express CD9, CD10, and CD117 on their surface; and
thereafter
(B) culturing the cells of the buffy coat component which expresses CD9, CD10, and_CD117 in a cell culture medium for a period of time and under conditions sufficient to induce proliferation thereof to yield the population of viable multipotent cells.

9. The population of claim 8, combined with a cryopreservation medium.

10. The population of claim 9 maintained under cryogenic storage conditions; and/or
maintained at a temperature not greater than -85 degrees Celsius.

11. A process for collecting a population of multipotent cells from menses comprising:
collecting a sample of menses with a collection cup placed in a donor's vagina during menstruation at a collection site;
transferring the sample of menses into a collection media in a container;
shipping the sample of menses mixed with the collection media in the container from the collection site to a processing facility within about 5 hours to about 125 hours after collecting the menses and at a temperature between about 1° C to about 37° C;
isolating the multipotent cells from other menses components by at least one step of centrifugation, density gradient centrifugation, filtration, or starch sedimentation;
decontaminating isolated multipotent cells with an antibiotic in an amount and for a period of time sufficient to substantially eliminate bacteriological contamination from the sample of menses and subsequently substantially removing the antibiotic from the population; and
preparing the decontaminated multipotent cells for cell culture, cryopreservation, cell selection, therapeutic use, or cosmeceutical use.

12. The method of claim 11, wherein the preparing the decontaminated multipotent cells comprises contacting multipotent cells with antibodies for a CD117 cell surface marker and separating the cells bound with the antibody from under decontaminated cells, and wherein the method optionally further comprises culturing the cells that have the CD117 surface marker, and wherein the method optionally further comprises contacting cultured cells with antibodies for CD117 and separating the cells that have the CD117 antibody from the cell culture, and wherein the method optionally further comprises a further step of culturing the cells that have the CD117 surface marker.

13. The method of claim 12, wherein the method includes culturing the decontaminated multipotent cells in a culture media sufficient to expand the decontaminated multipotent cells with at least one population doubling within a time period from about 24 to about 48 hours; and/or
wherein the method includes creating a composition comprising the decontaminated multipotent cells and a cosmeceutical agent; and/or
wherein the method includes creating a composition the decontaminated multipotent cells and a cryopreservation agent, and wherein the composition is optionally cryopreserved at a temperature less than about -85°C; and/or
wherein the method includes creating a composition comprising the decontaminated multipotent cells and a pharmaceutically-acceptable excipients; and/or
wherein the decontaminated multipotent cells co-express the cell surface markers CD117 and SSEA-4.

14. A method for cryopreserving a population of multipotent cells comprising:
centrifuging a sample of menses to isolate the multipotent cells from other menses components, wherein the sample of menses was collected with a collection cup placed internally within a donor during menstruation at a location that is remote from a processing laboratory and was later shipped to the processing facility;
decontaminating isolated multipotent cells with an antibiotic in an amount and for a period of time sufficient to substantially eliminate bacteriological contamination from the sample of menses and subsequently substantially removing the antibiotic from the population;
concentrating the population of multipotent cells isolated from the sample of menses with centrifugation; and
cryopreserving the concentrated population of multipotent cells in composition with a cryopreservation agent.

15. The method of claim 31, wherein the centrifuging of the sample of menses to isolate the multipotent cells from other menses components comprises using density gradient centrifugation to separate the multipotent cells into a buffy coat component and separating the buffy coat component from the other menses components; and/or
wherein the step of concentrating the population of multipotent cells isolated from the menstrual flow comprises contacting multipotent cells with an antibody for CD117 and separating complexes of multipotent cells and anti-CD117 antibody from other cells in the population, and wherein the step of concentrating the population of multipotent cells isolated from menstrual flow optionally further comprises at least one step of culturing the multipotent cells selected for expression of CD117 cell surface marker; and/or
wherein the step of concentrating the population of multipotent cells isolated from menstrual flow comprises at least on step of culturing the decontaminated isolated multipotent cells and isolating viable multipotent cells for cryopreservation, and wherein the step of isolating viable multipotent cells optionally further comprises at least one step of selecting multipotent cells for having at least one cell surface marker comprising CD9, CD10, CD13, CD29, CD44, CD49e, CD49f, CD59, CD81, CD105, CD117, CD166, SSEA-4, and HLA class I; and/or
wherein the method comprises thawing the cryopreserved multipotent cells.

16. A method of obtaining proliferative multipotent cells that express SSEA-4 comprising:
using a collection cup placed within a donor during menstruation to collect a sample of menses at a location remote from a processing facility;
combining the collected sample of menses with a collection media at the remote location;
shipping the mixture of the sample of menses and the collection media to the processing facility within about 5 hours to about 125 hours after collecting the sample of menses;
isolating multipotent cells from the mixture of menses and collection media with centrifugation, density gradient centrifugation, starch sedimentation, or filtration;
decontaminating isolated multipotent cells with an antibiotic in an amount and for a period of time sufficient to substantially eliminate bacteriological contamination from the menses and subsequently substantially removing the antibiotic from the population; and
expending the decontaminated isolated multipotent cells in culture sufficient to enrich for multipotent cells that express SSEA-4.
